# EUROPEAN PATENT APPLICATION

(11) **EP 2 929 852 A1**
(43) Date of publication of application: **14.10.2015**
(21) Application number: 15164212.1
(22) Date of filing: 06.04.2011
(51) Int. Cl.: A61B 18/18, A61B 18/12, A61B 18/00, A61B 19/00

(54) **SYSTEM FOR PULMONARY TREATMENT**

(30) Priority: 06.04.2010 US 321346 P
(62) Divisional of application: 11714906.2
(71) Applicant: Holaira, Inc., Plymouth, MN 55447 (US)
(72) Inventor: Deem, Mark, Mountain View, CA California 94041 (US); Shenoy, Vivek, Redwood City, CA California 94062 (US); Mayse, Martin L., Wayzata, MN Minnesota 55391 (US)
(74) Representative: Grünecker Patent- und Rechtsanwälte PartG mbB

(57) **Abstract**

An apparatus and method for pulmonary treatment by denervation is provided. The apparatus includes an elongate member configured for insertion into the trachea to a positions adjacent a pulmonary plexus. The apparatus further includes at least one energy delivery element disposed on the elongate member. The energy delivery element is positionable to target at least one nerve in the tracheal wall when the elongate member is positioned in the trachea. Energy from the energy delivery element is delivered to the at least one nerve to treat pulmonary symptoms, conditions, and/or diseases, such as asthma, COPD, obstructive lung diseases, or other pulmonary diseases.

## Description

### CROSS-REFERENCE TO RELATED APPLICATION

This application claims the benefit under 35 U.S.C. § 119(e) of U.S. Provisional Patent Application No. 61/321,346 filed April 6, 2010. This provisional application is incorporated herein by reference in its entirety.

### BACKGROUND

### Technical Field

The present invention generally relates to the field of pulmonary treatments.

### Description of the Related Art

One treatment for asthma which was performed in the 1930's to 1950's, prior to the advent of effective asthma medications, was surgical sympathectomy of the posterior pulmonary nerve plexus. Although the surgery was very morbid, typically requiring severing large muscle groups and manipulating the ribs, pleura and lungs, it was in some cases effective. As an alternative for patients for whom medications and other conventional treatments are ineffective, it would be desirable to achieve the benefits of a pulmonary sympathectomy, but without the high morbidity rates typically associated with such a procedure in the past.

There exists, in addition to the posterior pulmonary nerve plexus, an anterior pulmonary nerve plexus. The anterior pulmonary nerve plexus was never approached surgically due to its proximity to the heart and the great vessels. It is possible that these nerves also are involved in airway constriction associated with asthma and other pulmonary diseases.

There are several complicating factors to performing a denervation of these nerves from within the body. The nerves of interest run along the outside of the anterior trachea and bronchi, and the posterior plexus runs along the posterior, along and within the junction between the trachea and the esophagus. As a result of such difficulties there has been minimal interest in such approaches to the treatment of asthma.

### BRIEF SUMMARY

At least some embodiments include a treatment system that can be used to perform pulmonary treatments to address a wide range of pulmonary symptoms, conditions, and/or diseases, including, without limitation, asthma, chronic obstructive pulmonary disease ("COPD"), obstructive lung diseases, or other diseases that lead to an unwanted (e.g., increased) resistance to airflow in the lungs.

In some embodiments, an apparatus for pulmonary treatment by select denervation includes an elongate member configured for insertion into the trachea to a position adjacent target nerve tissue, such as a pulmonary plexus. The apparatus further includes at least one energy delivery element disposed on the elongate member in a position corresponding to the anatomical location of at least one nerve in or adjacent the tracheal wall when the elongate member is positioned in the trachea. In certain embodiments, energy from a single energy delivery element ablates the at least one nerve. In other embodiments, a plurality of energy delivery elements cooperate to ablate or otherwise alter the nerve or other targeted tissue.

A pulmonary treatment method, in some embodiments, includes positioning at least one energy delivery element in a trachea or airway of the bronchial tree adjacent a nerve site to be treated. In some embodiments, energy from the element is delivered to a portion of the circumference of the trachea at the treatment site. Tissue adjacent the treatment site is cooled to prevent tissue damage outside the treatment site.

To cool the tissue, a cooling medium can be delivered through a device positioned along a lumen of the esophagus. The device can have one or more cooling balloons configured to contact the wall of the esophagus to absorb heat, thereby cooling non-targeted tissue. Additionally or alternatively, an apparatus in the trachea combined with or separate from the at least one energy delivery element can include one or more cooling devices (*e.g.,* cooling balloons).

Some embodiments include an apparatus and method for targeting one or more target sites positioned between the lumens of the trachea and the esophagus. In certain embodiments, one or more devices are placed on the lumens of the trachea and/or esophagus to deliver energy so as to damage or otherwise alter one or more target sites located between the lumens of the trachea and the esophagus. The target sites can include nerve tissue. Preferably, such target sites are damaged while tissue closer to the lumens of the trachea and/or esophagus are protected from damage.

In some embodiments, a system for pulmonary treatment includes a pulmonary treatment device and a protection device. The pulmonary treatment device has one or more energy delivery elements positionable through at least a portion of a trachea into in an airway. The one or more energy delivery elements are configured to deliver energy to a wall of the airway to alter nerve tissue located in or proximate to the wall of the airway. The protection device has a protection member positionable in an esophagus even when the pulmonary treatment device is positioned in the airway. The protection member is configured to absorb heat from a wall of the esophagus to inhibit damage to esophageal tissue. In some procedures, the system is used to ablate nerve tissue of nerve trunks travelling along the airway. Additionally or alternatively, nerve tissue within the airway wall can be ablated.

A cooling apparatus can be associated with the energy delivery element to limit tissue damage adjacent select denervation sites. The cooling apparatus can include one or more pumps, blowers, conduits, facemasks, valves, or the like. Media from the cooling apparatus can flow through the subject to cool internal tissue. In some embodiments, the cooling apparatus includes a pump that delivers chilled air through a conduit into a lumen of the esophagus. The chilled air circulates within the lumen to cool the esophageal tissue.

A method for pulmonary treatment includes positioning at least one energy delivery element through at least a portion of the trachea into an airway adjacent a treatment site to be treated. In certain procedures, the airway is part of the trachea. In other procedures, the at least one energy delivery element is delivered through and out of the trachea and into the bronchial tree.

The method can further include delivering energy from the element to a portion of the circumference of the airway. The temperature of tissues can be adjusted to prevent or limited damaged to non-target tissue. In some procedures, tissues of an esophagus are cooled to prevent damage of the esophageal tissues while the energy is delivered. The esophageal tissues can also be cooled before and/or after delivering the energy.

The energy delivery element can be repositioned any number of times. In certain embodiments, the energy delivery element can be positioned in close proximity to the previous position. Energy is delivered to an adjacent treatment site. The adjacent site can barely overlap with the previous site. Alternatively, a small gap can be between the two treatment sites. The apparatus can be moved (*e.g.,* rotated, translated, or both) to reposition the energy delivery element to provide a slight overlap or a slight gap circumferentially with respect to an already treated site.

In some embodiments, a pulmonary treatment apparatus includes an elongate member and a microwave antenna. The elongate member is insertable through at least a portion of a trachea into an airway. The microwave antenna is coupled to the elongate member and positionable in the airway at a treatment location proximate nerve tissue in a wall thereof. The microwave antenna is configured to deliver microwave energy so as to alter the nerve tissue in a manner which disrupts transmission of nerve signals therein while non-target tissue (*e.g.,* tissue disposed between the microwave antenna and the nerve tissue) is not permanently injured. An active electrode can be non-inflatably (*e.g.,* balloonlessly) expandable from a contracted configuration to an expanded configuration. Thus, the activate electrode can be moved without the use of a balloon or other type of expansion device.

A system for pulmonary treatment can include at least one pulmonary treatment device capable of damaging nerve tissue such that the destroyed nerve tissue impedes or stops the transmission of nervous system signals to nerves more distal along the bronchial tree. The nerve tissue can be temporarily or permanently damaged by delivering different types of energy to the nerve tissue. For example, the nerve tissue can be thermally damaged by increasing a temperature of the nerve tissue to a first temperature (*e.g.,* an ablation temperature) while the wall of the airway is at a second temperature that is less than the first temperature. In some embodiments, a portion of the airway wall positioned radially inward from the nerve tissue can be at the first temperature so as to prevent permanent damage to the portion of the airway wall. The first temperature can be sufficiently high to cause permanent destruction of the nerve tissue. In some embodiments, the nerve tissue is part of a nerve trunk located in connective tissue outside of the airway wall. The smooth muscle and nerve tissue in the airway wall can remain functional to maintain a desired level of smooth muscle tone. The airway can constrict/dilate in response to stimulation (*e.g.,* stimulation caused by inhaled irritants, the local nervous system, or systemic hormones). In other embodiments, the nerve tissue is part of a nerve branch or nerve fibers in the airway wall. In yet other embodiments, both nerve tissue of the nerve trunk and nerve tissue of nerve branches/fibers are simultaneously or sequentially damaged. Various types of activatable elements, such as ablation elements in the form of microwave antenna, RF electrodes, heating elements, or the like, can be utilized to output the energy.

At least some methods of pulmonary treatment include positioning an elongate member through at least a portion of the trachea. The elongate member has a treatment element and a sensor coupled thereto. A first tissue characteristic is sensed using the sensor with the treatment element at a first airway location. The first tissue characteristic is compared to a reference value to evaluate the location of the treatment element in the airway. The treatment element is activated to treat an airway.

In certain embodiments, an apparatus for pulmonary treatment includes an elongate member insertable through a trachea into an airway and an active electrode coupled to the elongate member. The active electrode is configured to deliver energy to target tissue in a wall of the airway. A return electrode is positionable in the airway or the esophagus and configured to receive the energy from the target tissue. A protection member is configured to cool non-target tissue proximate to the target tissue. The non-target tissue can be surrounded or can be spaced apart from the target tissue.

The active electrode is expandable from a contracted configuration to an expanded configuration without the use of a balloon. The device can be self-expanding. For example, the device can include a self-expanding basket, a cage, a wire mesh, or other type of component capable of assuming a helical, spiral, corkscrew, or similar configuration. As such the active electrode can be non-inflatably expanded or actuated.

A method of pulmonary treatment includes delivering energy at a first power level from an active portion of an energy delivery element to create a first lesion covering a first portion of a circumference of an airway. Energy is delivered at a second power level from the active portion of the energy delivery element to create a second lesion covering a second portion of the circumference of the airway displaced from the first portion. The first power level is substantially greater than the second power level. In certain embodiments, the second portion is circumferentially or axially displaced from the first portion relative to a lumen of the airway. For example, the second portion can be both circumferentially displaced and axially displaced from the first portion.

Another method of pulmonary treatment includes delivering a first amount of energy from an energy delivery device to a first portion of a wall of an airway and delivering a second amount of energy from the energy delivery device to a second portion of the airway wall. The first portion of the wall and the second portion of the wall are spaced apart from one another or can partially overlap one another. For example, most of the first and second portions by area or volume can overlap one another.

A method of pulmonary treatment includes positioning an energy delivery element in an airway of a subject. The energy delivery element is non-inflatably actuated. The energy delivery element can be moved into engagement with a wall of the airway without using a balloon or other type of inflation device. The energy delivery element can be self-expanding. For example, the energy delivery element can be a self-expandable cage. The non-inflatably expandable cage can move one or more electrodes proximate to or in contact with the airway wall.

Energy can be delivered from the energy delivery element to the wall of the airway to alter target nerve tissue therein or proximate thereto. A cooling medium is passed into the airway into direct contact with the wall to absorb heat from the wall while delivering the energy. Alternatively, a protection device can be used to cool the airway wall.

The energy delivery element can comprise a first electrode. The first electrode is positioned within a first space between a first pair of adjacent cartilage rings of the airway. A second electrode is placed in a second space between a second pair of adjacent cartilage rings of the airway. The electrode can be part of a helical or corkscrew shaped device.

A protection device can be positioned in the esophagus to absorb heat from esophageal tissue while delivering the energy. Energy can be received by the protection device with or delivering energy from a second electrode coupled to the protection device.

A surface layer of tissue of the wall (*e.g.,* a wall of the trachea, a wall of the esophagus, etc.) can be protected from permanent injury while a lesion of permanently injured tissue is created at a depth below the surface layer. The surface layer is at least about 2 mm in thickness. At least a portion of the lesion contains nerve tissue. In certain procedures, the nerve tissue is altered sufficiently to reduce airway constriction in the subject.

The cooling medium can include one or more gas or other type of media. The energy delivery element is coupled to an elongate member such that the cooling medium is introduced into the airway through a channel in the elongate member. The cooling medium flows through a channel in the energy delivery element to absorb heat therefrom.

### BRIEF DESCRIPTION OF THE SEVERAL VIEWS OF THE DRAWINGS

For the purpose of illustrating the invention, the drawings show aspects of one or more embodiments of the invention. However, it should be understood that the present invention is not limited to the precise arrangements and instrumentalities shown in the drawings, wherein:
Figure 1 shows a cross section of the trachea and esophagus, and approximate locations of the anterior and posterior plexus nerves.
Figure 2 shows the cartilaginous rings of the trachea. The connective tissue sheath is shown cut away.
Figure 3 shows the trachea in cross section, illustrating a target region in the pulmonary plexus for treatment in embodiments of the present invention.
Figure 4 is a lateral view illustrating the length of a potential target region corresponding to the cross section in Figure 3.
Figure 4A is an anatomical drawing showing details of the posterior pulmonary plexus.
Figure 5 is a lateral view of a treatment system positioned in the trachea and the esophagus.
Figure 6 is a detailed view of a treatment device in the trachea and an esophageal device in the esophagus.
Figure 7 is cutaway view of a trachea and a distal tip of the treatment device.
Figure 8A is a cross-sectional view of the trachea and isotherms in tissue of the trachea and the esophagus.
Figure 8B is a cross-sectional view of the trachea and isotherms in tissue of the trachea and the esophagus.
Figure 9 illustrates a tracheal treatment device and an esophageal treatment device.
Figure 10 is an isometric view of a treatment system.
Figure 11 is a cross-sectional view of a tracheal catheter taken along a line 11-11.
Figure 12 is a cross-sectional view of the tracheal catheter taken along a line 12-12.
Figure 13 is an isometric view of an electrode assembly.
Figure 14 is a cross-sectional view of the electrode assembly of Figure 13 taken along a line 14-14.
Figure 15 is a partial cross-sectional view of a treatment system with a catheter extending out of a delivery apparatus.
Figure 16 is a side elevational view of a deployed energy delivery assembly with fluid flowing through an energy emitter assembly.
Figure 17 is a cross-sectional view of the deployed energy delivery assembly with fluid flowing through an expandable member.
Figure 18 is a cross-sectional view of the energy delivery assembly with fluid flowing into the expandable member.
Figure 19 is an elevational view of the ablation assembly with fluid flowing through the energy emitter assembly.
Figure 20 is a side elevational view of an electrode adjacent a cartilaginous ring.
Figure 21 is a side elevational view of electrodes positioned between cartilaginous rings.
Figure 22 is an isometric view of an ablation assembly with a pair of electrodes.
Figure 23 is an isometric view of an ablation assembly with three electrodes.
Figure 24A is a schematic view of a treatment system employing monopolar electrodes for pulmonary treatment and an esophageal device in a subject.
Figure 24B is a schematic view of an embodiment of the present invention employing monopolar electrodes for treatment.
Figure 25A is a schematic view of a tracheal device and an esophageal device in a subject.
Figure 25B is a schematic view of an embodiment employing trachea-to-esophagus circumferential bipolar electrodes.
Figure 26 illustrates a circumferential bipolar energy distribution possible with the embodiment of Figures 25A and 25B.
Figure 27 is a schematic view of an embodiment employing trachea-to-esophagus bipolar, anterior esophageal return electrodes.
Figure 28 illustrates a bipolar energy density distribution possible with the embodiment of Figure 27.
Figure 29 is a schematic view of an embodiment of the present invention employing trachea-to-esophagus bipolar, posterior isolated electrodes.
Figure 30 illustrates a bipolar energy distribution possible with the embodiment of Figure 29.
Figures 31A and 32B are schematic views of an embodiment of the present invention employing trachea-to-esophagus bipolar electrodes with no balloon support.
Figure 32 is an elevational view of an exemplary basket embodiment according to the present invention.
Figures 33A and 33B are schematic views of an embodiment employing a bipolar wire cage with circumferential electrode bands.
Figures 34A and 34B are schematic views of an embodiment of the present invention employing bipolar balloons with circumferential electrode bands.
Figure 35 is a schematic view of an embodiment of the present invention employing tracheal bipolar electrodes with a single tracheal protection zone.
Figure 36A is a schematic view of an embodiment of the present invention in an airway and employing tracheal bipolar electrodes with a dual tracheal protection zone.
Figure 36B is a schematic view of the tracheal device of Figure 36A.
Figure 36C is a top plan view of the tracheal device of Figure 36A.
Figures 37A-37C are schematic views of an embodiment of the present invention employing inter-cartilage electrodes in a stacked ring configuration.
Figures 38A and 38B are schematic views of an embodiment of the present invention employing inter-cartilage electrodes in a coiled configuration.
Figures 39A and 39B are schematic views of an embodiment of the present invention employing inter-cartilage electrodes with a winding adjustment element.
Figures 40A and 40B are schematic views of an embodiment of the present invention employing inter-cartilage electrodes with adjustable D-shaped rings in a bipolar configuration.
Figures 41A and 41B are schematic views of an embodiment of the present invention employing inter-cartilage electrodes with adjustable D-shaped rings in a bipolar configuration with cooling means.
Figure 42 is a schematic view of an embodiment of the present invention employing an esophageal protection device.
Figure 43 is a schematic view of an embodiment of the present invention employing esophageal protection with conductive elements.
Figure 44 is a schematic view of an embodiment of the present invention employing a distal occlusion device with a gas protectant.
Figure 45 is a schematic view of an embodiment of the present invention employing a distal occlusion device with a gas protectant and conductive elements.
Figure 46 is a schematic view of an embodiment of the present invention employing a distal occlusion device with a gas protectant and conductive elements showing the protective gas flow.
Figure 47 is a schematic view of an embodiment of the present invention employing a multi-slot coaxial microwave antenna.
Figure 48A is a schematic side view of a tracheal device employing a single antenna microwave system.
Figure 48B is a schematic view of the tracheal device of Figure 48A.
Figure 49 is a side view of a tracheal device.
Figure 50A is a schematic side view of a tracheal device with a dual antenna microwave system.
Figure 50B is a schematic front view of the tracheal device of Figure 53A.
Figure 51A is a schematic side view of a tracheal device with a dual antenna microwave system and an esophageal reflector/protector.
Figure 51B is a schematic front view of the tracheal device and esophageal reflector/protector device of Figure 51A.
Figure 52A is a schematic side view of a tracheal device with a microwave device with a cooling or coupling jacket.
Figure 52B is a schematic front view of the tracheal device of Figure 55A.
Figure 53 is a cross-sectional view of a tracheal device positioned within the trachea.
Figure 54A is a schematic view of an alternative embodiment of the present invention employing a microwave device with a cooling/coupling element.
Figure 54B illustrates a specific absorption rate profile generated by the treatment system of Figure 54A.
Figure 54C is a graph of an axial profile along a specific absorption rate observation line.

### DETAILED DESCRIPTION

Throughout this disclosure, the words disrupt, ablate, modulate, denervate will be used. It should be understood that these globally refer to any manipulation of the nerve that changes the action of that nerve. This can be a total cessation of signals, as in ablation or severing, or it can be a modulation, as is done by partial or temporary disruption, pacing, etc.

Similarly, trachea is often used to describe a segment wherein the devices and methods will be used. It should be understood that this is shorthand and can be meant to encompass the trachea itself, as well as the right and left main bronchi and other portions of the pulmonary tree as necessary.

It should be noted that the pulmonary nerves referred to in the disclosure not only include nerves that innervate the pulmonary system but also any neural structures that can influence pulmonary behavior. For example, elements of the cardiac plexus, or the nerves that innervate the esophagus, also interact with the airways and may contribute to asthmatic conditions. The nerves can include nerve trunks along the outer walls of hollow vessels, nerve fibers within the walls of hollow vessels (*e.g.,* the wall of the trachea and/or esophagus), nerves within a bridge between the trachea and esophagus, or at other locations. The left and right vagus nerves originate in the brainstem, pass through the neck, and descend through the chest on either side of the trachea. These nerves can be targeted. The vagus nerves spread out into nerve trunks that include the anterior and posterior pulmonary plexuses that wrap around the trachea, the left main bronchus, and the right main bronchus. The nerve trunks also extend along and outside of the branching airways of the bronchial tree. Nerve trunks are the main stem of a nerve comprising a bundle of nerve fibers bound together by a tough sheath of connective tissue. The vagus nerves, including their nerve trunks, along the trachea or other nerve tissue along, proximate to, or in the bronchial tree can be targeted. A treatment device in the form of a tracheal device can be positioned at different locations within an airway (*e.g.,* the trachea, one of the main stem bronchi, or other structures of the bronchial tree).

The pulmonary branches of the vagus nerve along the left and right main stem bronchus intermedius are particularly preferred targets. The nerve trunks of the pulmonary branches extend along and outside of the left and right main stem bronchus and distal airways of the bronchial tree. Nerve trunks of the main stem nerve comprise a bundle of nerve fibers bound together by a tough sheath of connective tissue. Any number of procedures can be performed on one or more nerve trunks to affect the portion of the lung associated with those nerve trunks. Because some of the nerve tissue in the network of nerve trunks coalesce into other nerves (*e.g.,* nerves connected to the esophagus, nerves though the chest and into the abdomen, and the like), specific sites can be targeted to minimize, limit, or substantially eliminate unwanted damage of those other nerves.

Some fibers of anterior and posterior pulmonary plexuses coalesce into small nerve trunks which extend along the outer surfaces of the trachea and the branching bronchi and bronchioles as they travel outward into the lungs. Along the branching bronchi, these small nerve trunks continually ramify with each other and send fibers into the walls of the airways. Any of those nerve trunks or nerve tissue in walls can be targeted. Various procedures that may be performed with at least some of the devices and methods of embodiments of the present invention are described in copending application Serial No. 12/463,304 filed on May 8, 2009, which is incorporated herein by reference in its entirety.

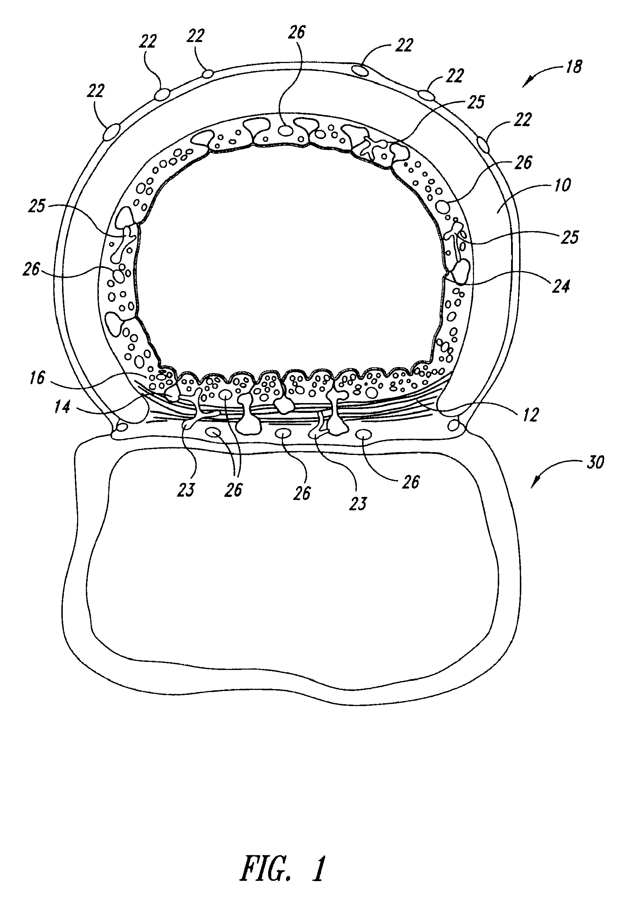

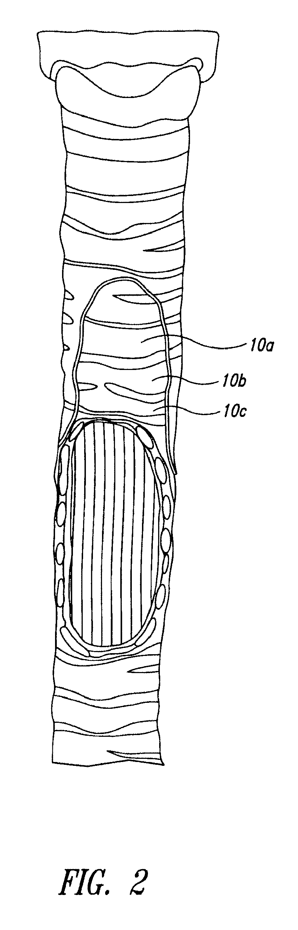

As illustrated in Figure 1, the C-shaped structure 10 that separates the inner elements of the airway-the smooth muscle 12, goblet cells 16, mucosa, anterior plexus nerves 22, posterior plexus nerves 23, epithelium 24, nerves 25, arteries 26, etc.,-from the nerves are thick bands of cartilage 10. These bands 10 cover the majority of the circumference of the trachea and larger bronchi, with a discontinuity only along the posterior segment where the trachea and esophagus are coincident. As further shown in Figure 2, these bands 10a, 10b, 10c (collectively "10") are discrete elements, arranged longitudinally along the length of the trachea 18 and large bronchi, with thinner areas of connective tissue between them. The anterior plexus runs outside of these bands. So it can be seen that any modality designed to sever or disrupt these nerves will be heavily guarded against by these rings.

A different complication exists along the posterior border where the discontinuity in the cartilage bands exists. Here, the trachea and esophagus are coincident, connected to one another by an area of connective tissue. Here the problem is the opposite of that on the posterior side. The esophagus can be easily damaged by devices operating from within the lung to disrupt or modulate the nerves running between the two lumens. A rare but fatal complication of cardiac ablation for the treatment of atrial fibrillation occurs when ablations performed within the heart create a weakness along the esophagus (the posterior left atrium is also adjacent the esophagus). In some cases, this weakness turns into a fistula, causing atrial rupture, massive hemorrhage and death. So it is critical to protect these ancillary structures or to direct the means for disruption or modulation away from them.

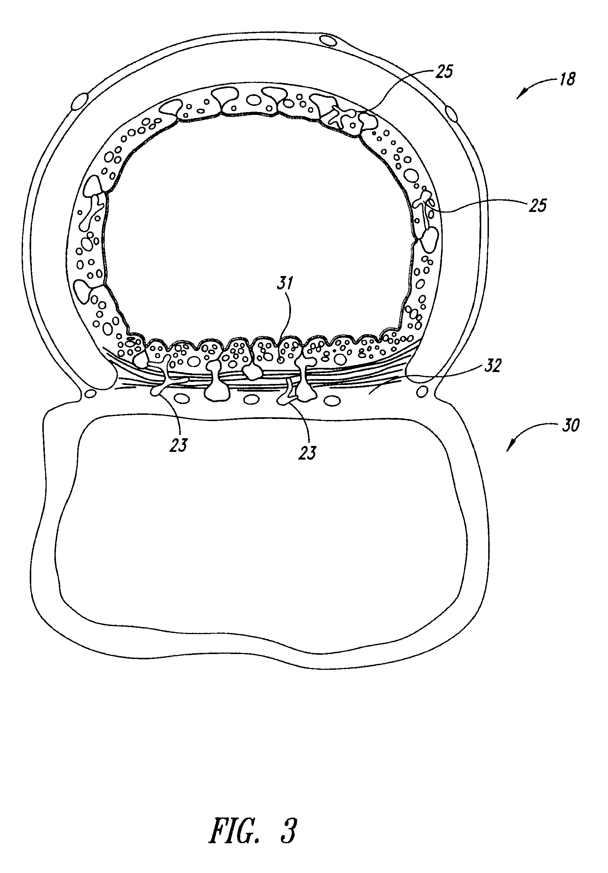

It can be seen from these descriptions of the anatomy of the trachea 18 and esophagus 30 that (as shown in Figure 3) energy or treatment means directed at or through the posterior wall 31 of the trachea 18, or the anterior wall 32 of the esophagus 30, would have direct access to the posterior pulmonary plexus 23.

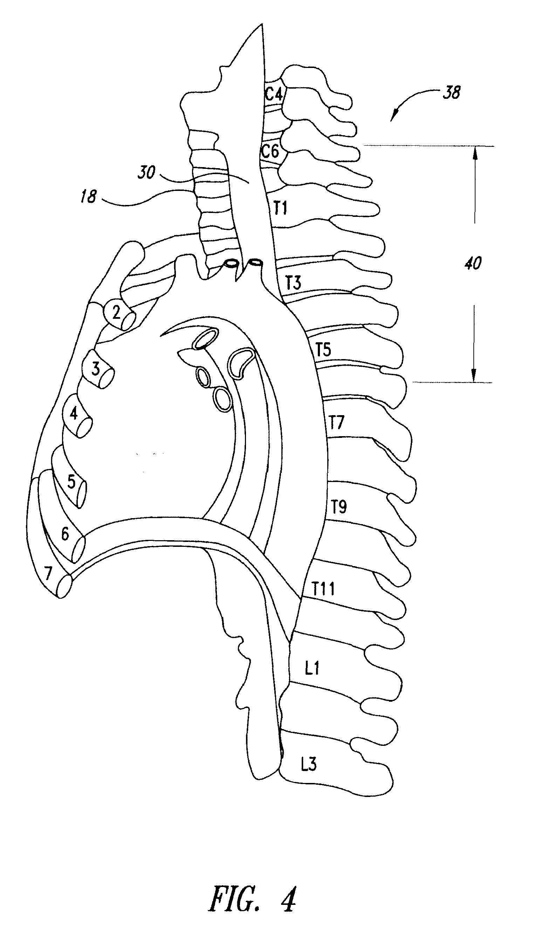

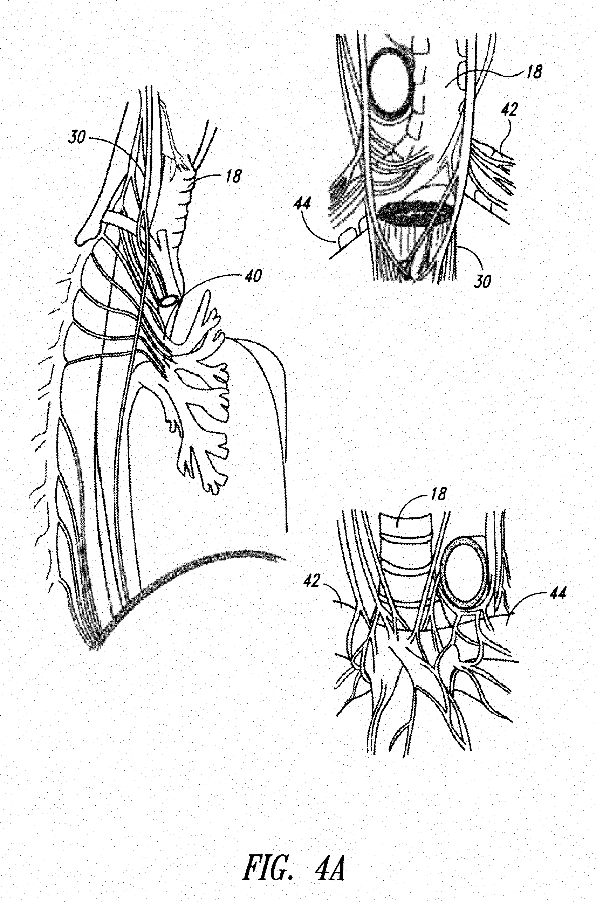

A potential region of interest for pulmonary nerve therapy is further described with reference to Figure 4A. Nerves which supply the pulmonary plexus arise from multiple levels of the thoracic spine 38 as well as multiple levels of the vagus nerve. Treatment and/or therapy delivery may occur anywhere within this potential target region 40, as a single treatment or as a plurality of treatments, administered in a single treatment session or staged over multiple sessions.

To modulate or disable the pulmonary nerves, it can be seen from the above anatomical descriptions that protection and or therapy can be delivered via the trachea 18, main stem bronchii or other airways further distally in the bronchial tree, the esophagus 30, or combinations of these. Following are brief descriptions of a number of different embodiments wherein energy is delivered to the targeted nerves through combinations of devices, or in some embodiments, through a single device. The targeted nerves can run along the trachea 18 and the esophagus 30, between the trachea 18 and the esophagus 30, or other suitable locations. For example, nerve tissue within walls of the trachea 18 and/or the esophagus 30 can be destroyed or otherwise altered. Alternatively or additionally, nerve trunks running along the outer wall of the trachea 18 and/or the esophagus 30 can be altered or destroyed.

In addition to the potential access to the pulmonary plexus 23 from the area of the trachea 18 and the correlated area in the esophagus 30, it can be seen from Figure 4A that a good number of branches from the thoracic ganglia 40 converge in the area of the carina, and the areas of the upper right bronchi 42 and upper left bronchi 44. Thus, the esophagus 30 may still need to be protected if tissue modification is to be done in the area of the carina, but as the target area moves more distally down the right and left bronchi, the need for esophageal protection diminishes.

Another reason that it may be beneficial to focus the treatment area more towards the individual right and left bronchi 42, 44 is that the recurrent laryngeal nerve may in some cases be collocated with nerves supplying the pulmonary plexus as they travel down the tracheal/esophageal interface to the lower areas of the plexus. Damage to the laryngeal nerve was shown in the surgical literature for pulmonary sympathectomy to be associated with complications of speech and swallowing, so preserving its function is critical.

Of note, as the treatment zone is located farther down the bronchial tree, past the carina and away from the trachea, the cartilaginous rings become completely circumferential-the area of non-coverage which was available for exploitation by a treatment device is no longer present. With this in mind, devices targeting regions of full cartilaginous coverage may have the requirement that they need to traverse and deliver therapy around, between or through these rings in order to reach the target nerves.

According to certain embodiments of the invention, devices may be configured for the delivery of radio frequency energy to modulate or disable the pulmonary plexus. While embodiments shown are configured for delivery of RF energy, many of the configurations can also be adapted to accommodate a catheter based microwave antenna, high energy pulse electroporation, or similar energy modalities.

The RF energy can be delivered in a traditional conductive mode RF, where the energy is directly applied to the tissue through a direct contact electrode, or it can be delivered through the use of capacitive coupling to the tissue. In capacitive coupling, a slightly higher frequency signal is typically used compared to traditional RF, and the energy is delivered to the tissue across a dielectric, which is often a cooling element. In one example of capacitive coupling, energy may be delivered across a cooling plate that keeps the surface of tissue contacted from being harmed as energy is delivered deeper into the target tissue.

The RF energy can be delivered to different target regions, which can include, without limitation, nerve tissue (*e.g.,* tissue of the vagus nerves, nerve trunks, etc.), fibrous tissue, diseased or abnormal tissues (*e.g.,* cancerous tissue, inflamed tissue, and the like), cardiac tissue, muscle tissue, blood, blood vessels, anatomical features (*e.g.,* membranes, glands, cilia, and the like), or other sites of interest. In RF ablation, heat is generated due to the tissue resistance as RF electrical current travels through the tissue. The tissue resistance results in power dissipation that is equal to the current flow squared times the tissue resistance. To ablate deep tissues, tissue between an RF electrode and the deep tissue can become heated if active cooling is not employed using a cooling device, such as a cooling plate or cooling balloon. The cooling device can be used to keep tissue near the electrode below a temperature that results in cell death or damage, thereby protecting tissue. For example, cooling can prevent or limit overheating at the electrode-tissue interface. Overheating (*e.g.,* tissue at temperatures above 95°C to about 110°C) can lead to the formation of coagulum, tissue desiccation, tissue charring, and explosive outgassing of steam. These effects can result in increased tissue resistance and reduced RF energy transfer into the tissue, thereby limiting the effective RF ablation lesion depth. Active cooling can be used to produce significantly deeper tissue lesions. The temperature of coolant for active cooling can be about 0°C to about 24°C. In some embodiments, the coolant and electrode produce a lesion at a therapeutic depth of at least about 3 mm while protecting tissue at shallower depths from lethal injury. In some embodiments, the lesions can be formed at a depth of about 3 mm to about 5 mm to damage nerve tissue. Other temperatures and depths can be achieved.

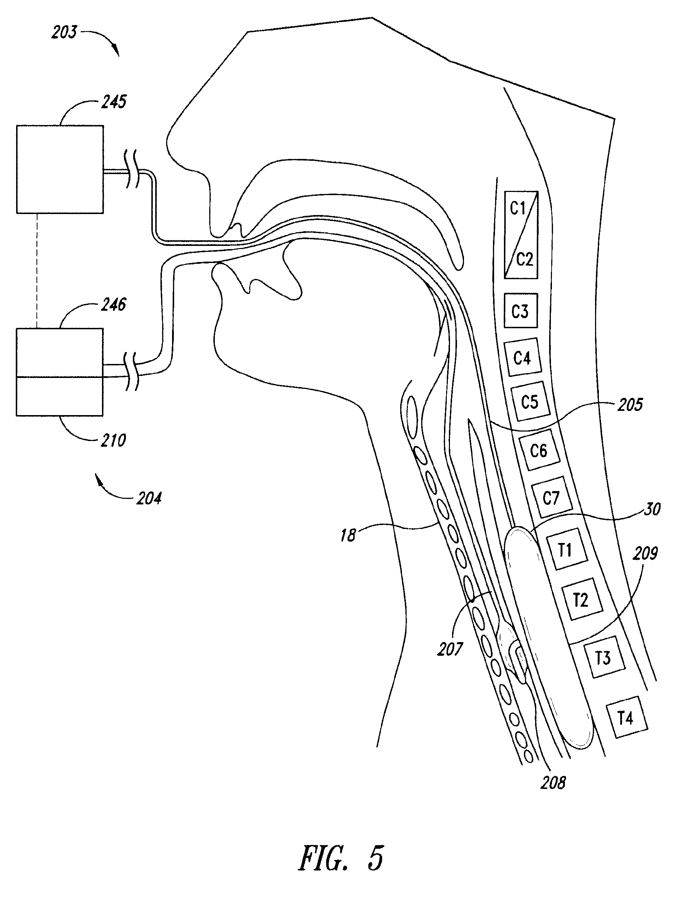

Figure 5 shows a system 204 including a pulmonary treatment device in the form of a tracheal catheter 207 positioned in the trachea 18 and a protection device 205, or temperature control device, positioned in the esophagus 30. An energy delivery assembly 208 is positioned to deliver energy to ablate targeted tissue between the trachea 18 and esophagus 30 while protecting non-targeted tissue. The temperature control device 205 includes a protection member 212 that absorbs heat to cool and protect tissue of the esophagus 30, thereby inhibiting damage to esophageal tissue. The tracheal catheter 207 can deliver a sufficient amount of energy to the trachea wall to heat and damage target tissue while the temperature control device 205 absorbs a sufficient amount of heat from the esophagus wall to inhibit damage to esophageal tissue while the target tissue is damaged. The tracheal device 204 and the temperature control device 205 can cooperate to ablate or otherwise alter targeted tissue, such as the pulmonary plexus 32.

It will be understood that, with regard to any of the embodiments described herein, while described here for use in the trachea, the devices and methods of the invention may be used for treatment in more distal airways including the mainstem bronchii, broncus intermedius, and more distal branches of the bronchial tree. Thus the terms "tracheal device" and the like are not intended to be limited to devices used in the trachea and may be interpreted to mean devices for use in any location in the trachea or bronchial tree where nerve tissue may be targeted to treat asthma and other pulmonary diseases using the techniques described herein.

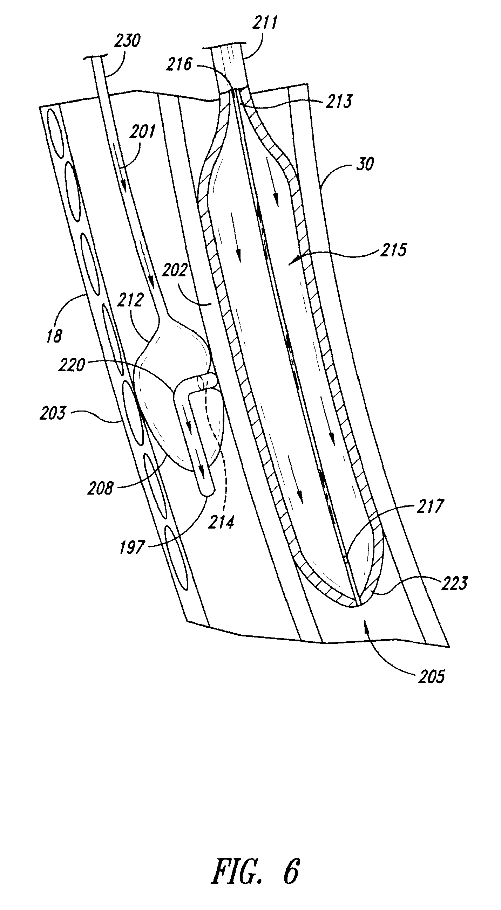

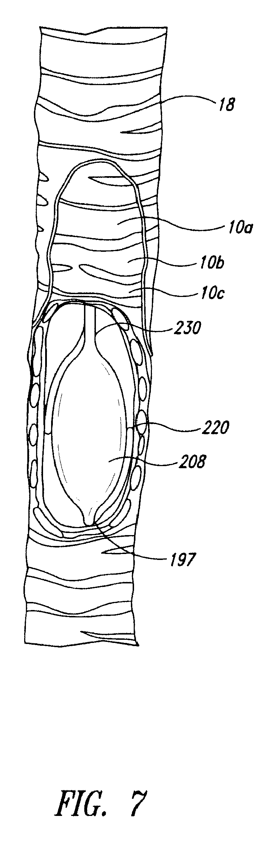

Referring to Figures 6 and 7, if the energy delivery assembly 208 includes an energy delivery element in the form of an RF electrode 214, the electrode 214 can be brought into contact with or proximate to an inner surface of the trachea 18. The RF electrode 214 can output RF energy which travels through the tissue and is converted into heat. The heat causes formation of a lesion. The RF energy can be directed radially outward towards the targeted tissue without causing appreciable damage to non-targeted tissue (*e.g.,* tissue of the esophagus 30, inner tissue of the trachea 18, anterior tissue of the trachea 18) using coolant (represented by arrows 201). A wide range of different procedures, such as, for example, denervation of a portion of the trachea 18, an entire circumference of the trachea 18, target nerve trunks travelling to one lung or both lungs, or the like. Nerve tissue is damaged to relax the muscle tissue in the bronchial tree to dilate the airway to reduce air flow resistance in one or both lungs, thereby allowing more air to reach the alveolar sacs for the gas exchange process. Decreases in airway resistance may indicate that passageways of airways are opening, for example in response to attenuation of nervous system input to those airways. The balloon 212 can absorb heat to cool the anterior region 203 (shown removed in Figure 7) of the trachea 18. Emitter assembly 220 wraps around the balloon 212 to contact the posterior region 202 of the trachea 18, as shown in Figure 6. The emitter assembly 220 extends along the balloon 212 to a distal tip 197.

A physician can select and ablate or otherwise alter appropriate nerve tissue to achieve a desired decrease in airway resistance, which can be measured at a subject's mouth, a bronchial branch that is proximate to the treatment site, a trachea, or any other suitable location. The airway resistance can be measured before performing the therapy, during the therapy, and/or after the therapy. In some embodiments, airway resistance is measured at a location within the bronchial tree by, for example, using a vented treatment system that allows for respiration from areas that are more distal to the treatment site. Any number of procedures can be used to treat asthma, COPD, and other diseases, conditions, or symptoms.

The temperature control device 205 of Figure 6 includes an elongate member 211 connected to the inflatable member 223. Media, such as chilled saline, flows through an input lumen 213 and circulates through a chamber 215. The media absorbs heat and exits the chamber 215 through an outlet 217. The media flows proximally through an output tube 216. The longitudinal length of the inflatable member 223 can be longer than a longitudinal length of the energy delivery assembly 208 to ensure that a longitudinal section of tissue extending distally and proximally of the targeted tissue is cooled to avoid unwanted tissue alteration, for example, tissue damage.

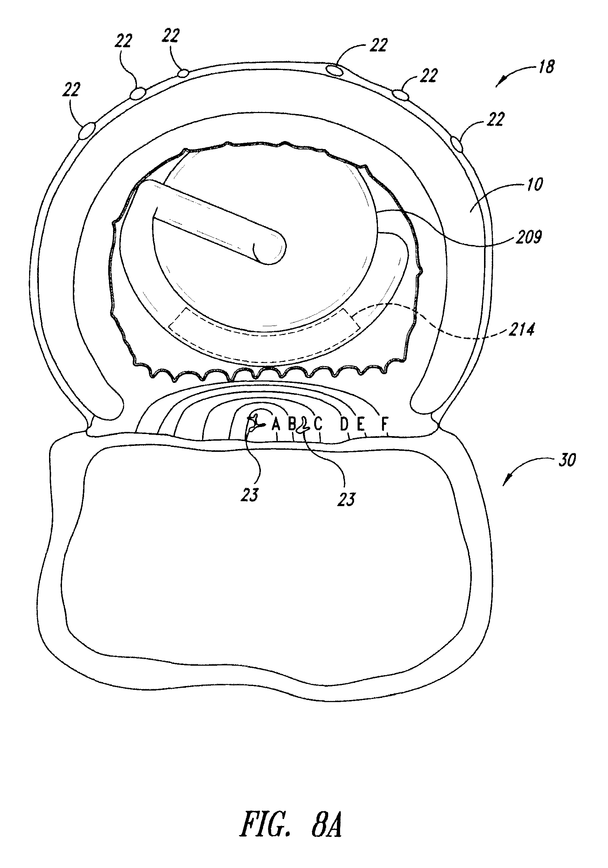

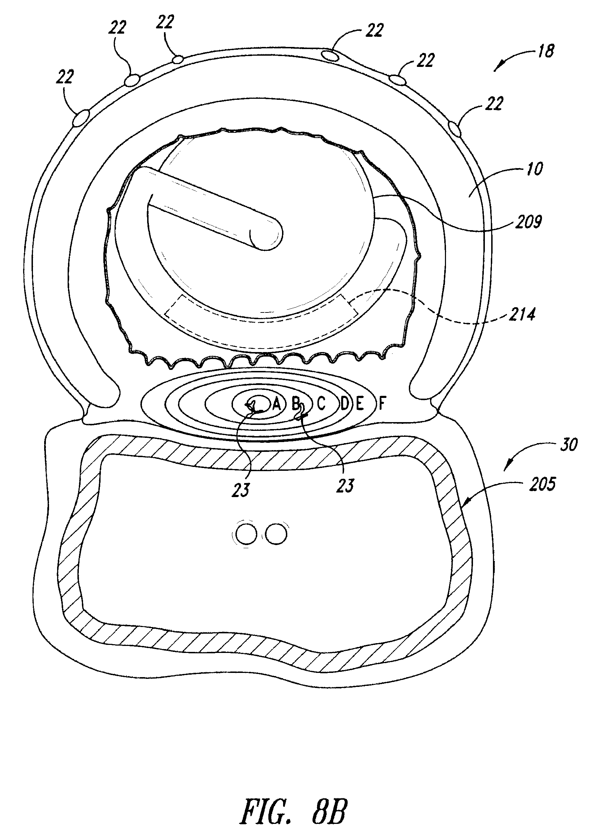

Figures 8A and 8B show isotherms. By adjusting the rate of power delivery to an electrode 214, the rate at which media is passed into the energy delivery assembly 208, the rate at which media is passed into the inflatable member 212, the temperatures of the media, the sizes and configuration of energy delivery assembly 208/inflatable member 212, and the exact contour and temperature of the individual isotherms can be modified. An energy distribution can be produced which results in isotherm A being warmest and, moving radially outward from isotherm A, each successive isotherm becomes cooler, with isotherm F being coolest. At minimum, the temperature at isotherm A will be high enough to produce cell death in the target tissue. In at least some preferred embodiments, isotherm A will be in a range of about 50°C to about 90°C, more preferably about 60°C to about 85°C, and most preferably about 70°C to about 80°C. Isotherm F will be at or around body temperature, and the intervening isotherms will be at intervals between body temperature and the temperature at isotherm A. For example, by selecting the proper temperature and flow rate of saline and the rate of power delivery to the electrode, it is possible to achieve temperatures in which isotherm A = 70°C, B = 55°C, C = 50°C, D = 45°C, E = 40°C, and F = 37°C. In some tissues, a lethal temperature may be greater than or equal to about 70°C. For example, the A isotherm can be about 75°C to about 80°C to form lesions in nerve tissue. Different isotherms and temperature profiles can be generated for different types of tissue because different types of tissue can be affected at different temperatures. Further adjustments make it possible to achieve temperatures where isotherm A = 50°C, B = 47.5°C, C = 45°C, D = 42.5°C, E = 40°C, and F = 37°C. Alternative adjustments make it possible to achieve temperatures where isotherm A is equal to or greater than 90°C, B = 80°C, C = 70°C, D = 60°C, E = 50°C, and F = 40°C. Only those areas contained within the A and B isotherms will be heated enough to induce cell death for certain types of tissue. Other temperature ranges are also possible depending on the lethal temperature of the target tissue. In some procedures, tissue at a depth of about 2 mm to about 8 mm in the airway wall can be ablated while other non-targeted tissues at a depth of less than 2 mm in the airway wall are kept at a temperature below a temperature that would cause cell death. The isotherms of Figure 8A can be generated without cooling using the temperature control device 205. By cooling tissue using the temperature control device 205, the isotherms generate bands, as illustrated in Figure 8B. Advantageously, the interior tissues of the trachea 18 and the esophagus 30 can be undamaged while deep tissue, including nerve tissue 23, is damaged.

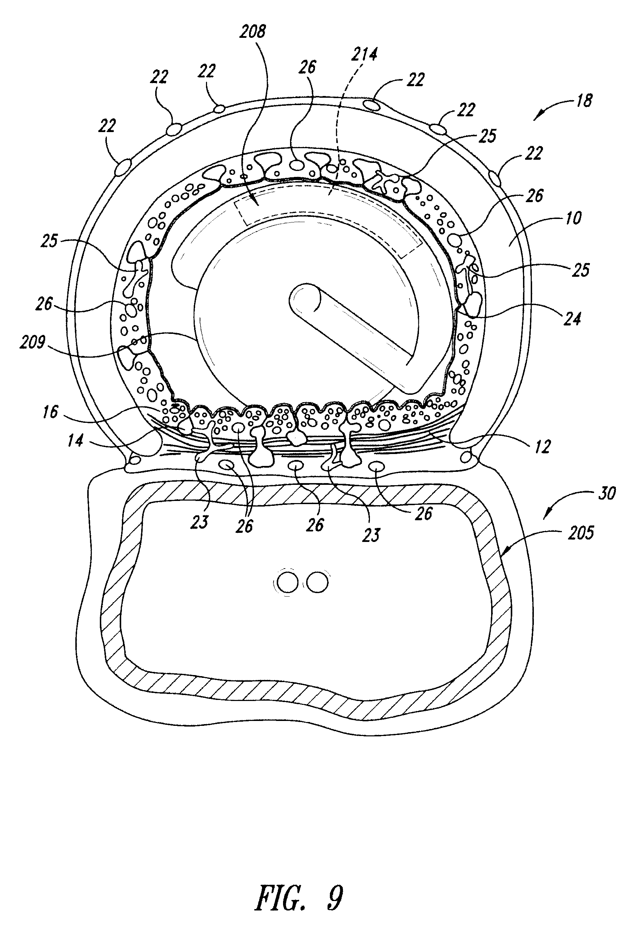

The RF electrode 214 can be positioned at other locations. Figure 9 shows the RF electrode 214 positioned to target the right anterior plexus 22. After each application of energy, the energy delivery assembly 208 can be angularly rotated to treat a different section of the trachea wall. In some procedures, an entire circumference of the trachea wall 18 can be treated. In other embodiments, circumferential segments of the trachea wall 18 are treated to target specific tissue while minimizing tissue damage of adjacent sections of the trachea wall. Throughout the procedure, the temperature control device 205 can cool the esophageal tissue.

Different amounts of energy can be delivered to different sections of the trachea 18. Energy delivered at a first power level from the electrode 214 can create a first lesion covering a first portion of a circumference of the airway. Energy delivered at a second power level from the electrode 214 can create a second lesion covering a second portion of the circumference of the airway displaced from the first portion. The first power level is substantially different (*e.g.,* greater) than the second power level. For example, the second power level can be about 40% to about 90% of the first power level, more preferably about 50%-80% of the first power level. The second power level can be selected to avoid permanent injury to non-target tissue proximate to the treatment site. The second portion can be circumferentially or axially displaced from the first portion relative to lumen of the airway. The first portion of the circumference can be on an anterior aspect of the airway, and the second portion can be on a posterior aspect of the airway.

Because the anterior region of the trachea 18 is spaced well apart from the esophagus 30, a higher amount of energy can be used to ablate the pulmonary plexus 22. As the electrode 214 is rotated towards the esophagus 30, the amount of emitted energy can be reduced. This can help minimize, limit, or substantially eliminate tissue damage to the esophageal tissue. Different amounts of energy can be delivered to different regions (*e.g.,* circumferential locations) of the trachea 18. A relatively high amount of energy can be delivered to the anterior region of the trachea 18 as compared to the amount of energy delivered to the posterior region of trachea 18. A lower amount of energy can be delivered to the posterior tissue of the trachea 18 to avoid damage to esophagus tissue. In some protocols, about 20 watts of energy is delivered to electrode 214 to ablate tissue located at the anterior region of the trachea 18. The electrode 214 can emit no more than about 15 watts of energy when it is positioned to contact the posterior region of the trachea 18. In various procedures, the amount of energy delivered to the electrode 214 can be at least about 40% but less than 90% of the energy delivered to the electrode 214 at a different region of the trachea 18. In certain embodiments, the amount of energy emitted by the electrode 214 positioned along the posterior portion of the trachea 18 is in a range of about 50% to about 80% of the energy delivered to the electrode 214 positioned at the anterior portion of the trachea 18. In other embodiments, the amount of energy emitted by the electrode 214 positioned along the posterior portion of the trachea 18 is in a range of about 60% to about 90% of the energy delivered to the electrode 214 positioned at the anterior portion of the trachea 18. Other relative percentages are also possible.

As the mainstem bronchi pass from the lung root at the main carina out towards the lungs, a variety of external structures lie in close proximity to their outer surfaces. Anteriorly, these external structures are the pulmonary arteries and veins, aorta and superior vena cava; medially they are the soft tissues of the mediastinum and the heart; laterally the external structure is the lung parenchyma; posteriorly on the right it is again lung parenchyma; proximally on the left it is the esophagus; and distally it is the lung. Additionally, the continuation of the left main vagus nerve as it passes inferiorly to innervate the abdomen and pelvis is interposed between the esophagus and the left main bronchi.

Due to the high rate of blood flow through the blood vessels and the heart, these structures are effective heat sinks and much of the heat generated during treatment is removed from their walls during treatment. Thus, the walls of the blood vessels and of the heart are relatively unaffected by the treatment. The mediastinal soft tissues and the lung lack the heat sinking effect seen in the blood vessels and heart, but they may tolerate thermal injury without untoward clinical consequences. However, the esophagus and interposed vagus nerve lack significant blood flow and may be susceptible to thermal injury during treatment in the left mainstem bronchus.

In one procedure, the treatment site to which RF energy is applied is the most distal centimeter of the left mainstem bronchus. Because the esophagus 30 runs along the posterior aspect of the proximal potion of the left mainstem bronchus, at this most distal aspect of the bronchus, the posterior wall is in contact with lung parenchyma only. Thus, the RF energy can be delivered to the most distal centimeter of the left mainstem bronchus to avoid injury to the esophagus 30. Other types of energy can also be delivered to this location.

In another procedure, the posterior wall of the left mainstem bronchus is either not treated or is treated with a lower dose of energy, while the remainder of the airway's circumference is treated with a higher dose of energy. When the balloon 212 of Figures 5 and 6 has a longitudinal length of about 8 mm to about 12 mm, the electrode 214 can be cooled with either room temperature water or iced water coolant passing through the electrode 214 and balloon 212. In certain procedures, the rate of flow of the water or coolant through the balloon 212 and the electrode 214 can be maintained at about 100 ml per minute for a treatment duration of about 120 seconds, while power levels are maintained at less than 15 W applied on the posterior wall of the mainstem bronchus to cause substantially no injury to the esophagus 30 or the interposed vagus nerve. Other combinations of electrode size, coolant, coolant temperature, coolant flow, treatment duration and power could be used to achieve the same results.

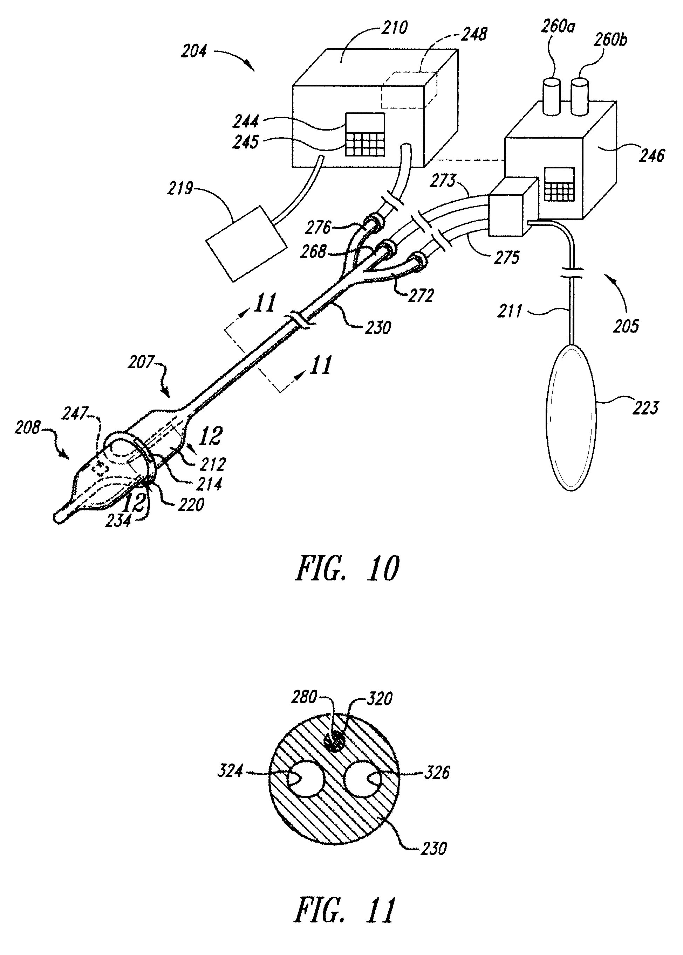

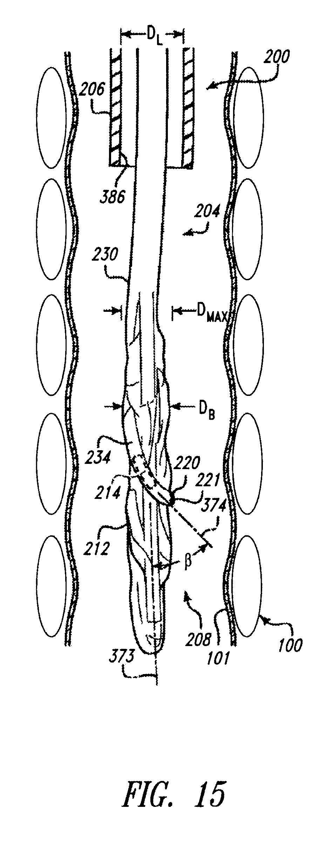

Referring to Figure 10, the treatment system 204 includes a media delivery system 246 and a control module 210 coupled to an elongate member in the form of a shaft 230 of the catheter 207. The temperature control device 205 is coupled to the media delivery system 246. An electrode pad 219 for placement against the patient is connected to the control module 210. Energy delivery assembly 208 comprises an emitter assembly 220 extending from the elongate shaft 230 and wrapping around a balloon 212. The balloon 212 can be inflated from a collapsed state (see Figure 15) to the expanded state shown in Figure 10. As the balloon 212 inflates, the electrode 214 can be moved towards the airway wall. The fully inflated balloon 212 can hold the electrode 214 near (*e.g.,* proximate or in contact with) tissue through which energy is delivered. The coolant can absorb thermal energy to cool the balloon 212 or the energy emitter assembly 220, or both. This in turn cools the outer surface of the airway wall.

The control module 210 can include, without limitation, one or more computers, processors, microprocessors, digital signal processors (DSPs), field programmable gate arrays (FPGA), computing devices, and/or application-specific integrated circuits (ASICs), memory devices, buses, power sources, and the like. For example, the control module 210 can include a processor in communication with one or more memory devices. Buses can link an internal or external power supply to the processor. The memories may take a variety of forms, including, for example, one or more buffers, registers, random access memories (RAMs), and/or read-only memories (ROMs). Programs, databases, values, or other information can be stored in memory. For example, in some embodiments, the control module 210 includes information associated with tissue characteristics. A comparison can be performed between sensed tissue characteristics and stored tissue characteristics. Operation of the catheter 207 can be adjusted based, at least in part, on the comparison. Different types of reference values (*e.g.,* reference values for non-treated tissue, reference values for treated tissues, impedance values, etc.) corresponding to tissue characteristics can be utilized in such a protocol. The control module 210 may also include a display 244, such as a screen, and an input device 245. The input device 245 can include one or more dials, knobs, touchpads, or a keyboard and can be operated by a user to control the catheter 207. Optionally, the input device 245 can also be used to control operation of the temperature control device 205.

The control module 210 can store different programs. A user can select a program that accounts for the characteristics of the tissue and desired target region. For example, an air-filled lung can have relatively high impedance, lymph nodes have medium impedance, and blood vessels have relatively low impedance. The control module 210 can determine an appropriate program based on the impedance. A differential cooling program can be executed to deliver different temperature coolants through the balloon 212 and the emitter assembly 220. The temperature difference can be at least 10°C. Performance can be optimized based on feedback from sensors that detect temperatures, tissue impedance, or the like. For example, operation of the energy delivery assembly 208 can be controlled based on a surface temperature of the tissue to which energy is delivered. If the surface temperature becomes excessively high, cooling can be increased and/or electrode power decreased in order to produce deep lesions while protecting surface tissues.

The control module 210 can function as an energy generator, such as a radio frequency (RF) electrical generator. RF energy can be outputted at a desired frequency. Example frequencies include, without limitation, frequencies in a range of about 50 KHZ to about 1,000 MHZ. When the RF energy is directed into tissue, the energy is converted within the tissue into heat causing the temperature of the tissue to be in the range of about 40°C to about 99°C. The RF energy can be applied for about 1 second to about 120 seconds. In some embodiments, the RF generator has a single channel and delivers approximately 1 to 25 watts of RF energy and possesses continuous flow capability. Other ranges of frequencies, time intervals, and power outputs can also be used. An internal power supply 248 can be an energy storage device, such as one or more batteries. Electrical energy can be delivered to the energy emitter assembly 220, which converts the electrical energy to RF energy or another suitable form of energy. Other forms of energy that may be delivered include, without limitation, microwave, ultrasound, direct current, or laser energy. Alternatively, cryogenic ablation may be utilized wherein a fluid at cryogenic temperatures is delivered through the shaft 230 to cool a cryogenic heat exchanger on the assembly 208.

Referring again to Figures 5 and 10, the control module 210 can have one or more communication devices to wirelessly, optically, or otherwise communicate with the media delivery system 246. Pumps of the media delivery system 246 can be operated based on the signals. In other embodiments, the control module 210 can include the media delivery system 246. A single unit can therefore control operation of the catheter 207 and the temperature control device 205.

The media delivery system 246 can pump cooling media through the pulmonary treatment device 207 and the temperature control device 205 and includes a media container 260a coupled to a supply line 268 and a media container 260b coupled to a return line 272. Luer connectors or other types of connectors can couple the lines 268, 272 to lines 273, 275. The media container 260a can include a container (*e.g.,* a bottle, a canister, a tank, a bag, or other type of vessel for holding fluid or other media). In pressurizable embodiments, the media container 260a includes one or more pressurization devices *(e.g.,* one or more pumps, compressors, or the like) that pressurize coolant. Temperature control devices (*e.g.,* Peltier devices, heat exchangers, or the like) can cool or recondition the fluid. The media can be a coolant including saline, deionized water, refrigerant, cryogenic fluid, gas, mixtures thereof, or the like.

In other embodiments, the media container 260a can be an insulated container that holds and delivers a chilled coolant to the supply line 268. In embodiments, the media container 260a is bag, such as an IV type bag, configured to be held on a pole.

The balloon 212 optionally has a sensor 247 (illustrated in dashed line in Figure 10) that is communicatively coupled to the control module 210. The control module 210 can command the catheter 207 based on signals from the sensor 247 (*e.g.,* a pressure sensor, a temperature sensor, a thermocouple, a pressure sensor, a contact sensor, an impedance sensor, or the like). Sensors can also be positioned on energy emitter assembly 220, along the elongate shaft 230, or at any other location. In a closed loop system, the electrical energy is delivered to the electrode 214 based upon feedback signals from one or more sensors configured to transmit (or send) one or more signals indicative of one or more tissue characteristics, energy distribution, tissue temperatures, or any other measurable parameters of interest. Based on those readings, the control module 210 adjusts operation of the electrode 214. Alternatively, in an open loop system, the operation of the electrode 214 is set by user input. For example, the user can observe tissue temperature or impedance readings and manually adjust the power level delivered to the electrode 214. Alternatively, the power can be set to a fixed power mode. In yet other embodiments, a user can repeatedly switch between a closed loop system and an open loop system.

In certain procedures, the sensor 247 can sense one or more tissue characteristics. The control module 210 can analyze the sensed tissue characteristics. For example, the control module 210 compares at least one sensed tissue characteristic to at least one stored reference value to, for example, evaluate the location of the electrode 214 relative to the airway. The evaluation can include, without limitation, determining the position of the electrode 214 relative to a reference location. The control unit 210 can estimate the location of at least one non-target structure or tissue based on impedance and/or other measurable characteristic. After estimating the location of the non-target structure or tissue, the electrode 214 can be repositioned before delivering energy so as to avoid injury to the non-target structures or tissue. Previously treated tissue can be detected based on impedance and/or other measurable characteristics. The electrode 214 can be activated to treat the airway when it is determined that the electrode 214 is located in the desired position.

Media flowing through the conduit 234 cools the electrode 214. Alternatively, flow diverters within the balloon 212 can direct some or all of the coolant in the balloon 212 towards the electrode 214 or a balloon sidewall and may provide a separate cooling channel for the electrode 214. In some embodiments, one or more cooling channels extend through the electrode 214 (*e.g.,* electrode 214 may be tubular so that coolant can flow through it). In other embodiments, the coolant flows around or adjacent the electrode 214. For example, an outer member, illustrated as the conduit 234 in Figure 10, can surround the electrode 214 such that fluid can flow between the electrode 214 and the conduit 234. Additionally or alternatively, the energy delivery assembly 208 can be actively cooled or heated using one or more thermal devices (*e.g.,* Peltier devices), cooling/heating channels, or the like.

Referring to Figures 10 and 11, the elongate shaft 230 extends from the control module 210 to the energy delivery assembly 208 and includes a power line lumen 320, a delivery lumen 324, and a return lumen 326. A power line 280 extends through the power line lumen 320 and couples the control module 210 to the electrode 214. The delivery lumen 324 provides fluid communication between the media container 260a and the energy emitter assembly 220 and balloon 212. The return lumen 326 provides fluid communication between the balloon 212 and/or electrode 214 and the fluid receptacle 260b. The elongate shaft 230 can be made, in whole or in part, of one or more metals, alloys (*e.g.,* steel alloys such as stainless steel), plastics, polymers, and combinations thereof, as well as other biocompatible materials, and can be flexible to pass conveniently along highly branched airways. Sensors can be embedded in the elongate shaft 230 to detect the temperature of the fluids flowing therethrough.

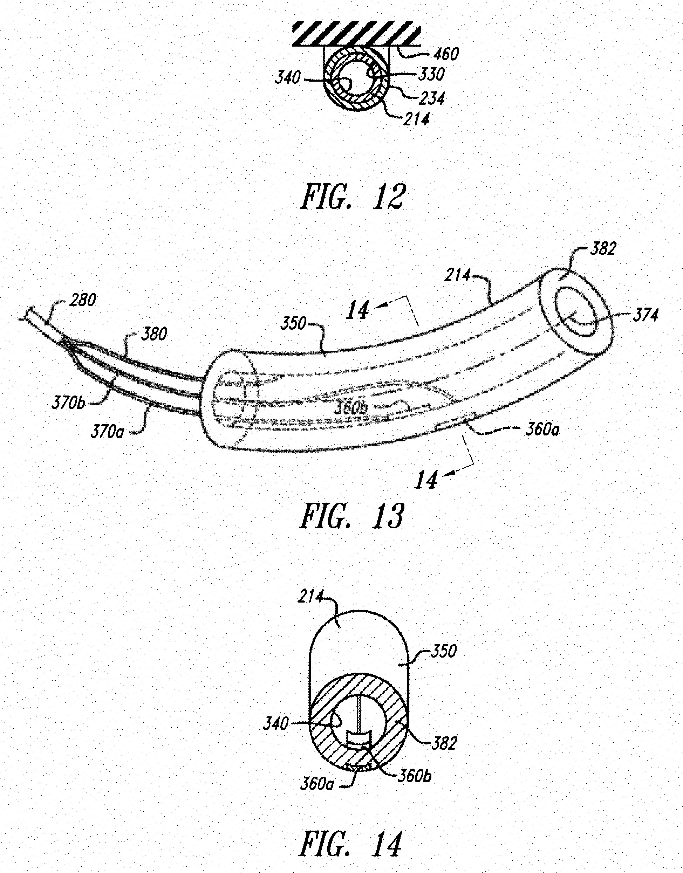

Figure 12 shows the electrode 214 positioned in a channel 330 of the conduit 234 and includes a coolant channel 340. The electrode main body 350 can be a rigid tube made, in whole or in part, of metal (*e.g.,* titanium, stainless steel, or the like). In some embodiments, conduit 234 does not extend over the entire electrode 214, leaving a central portion of the tubular electrode exposed for direct contact with the airway wall. In other embodiments, the electrode main body 350 is made, in whole or in part, of a shape memory material. Shape memory materials include, for example, shape memory metals or alloys (*e.g.,* Nitinol), shape memory polymers, ferromagnetic materials, combinations thereof, and the like. These materials can assume predefined shapes when released from a constrained condition or different configurations when activated with heat. In some embodiments, the shape memory material can be transformed from a first preset configuration to a second preset configuration when activated (*e.g.,* thermally activated).

As shown in Figures 13 and 14, sensors 360a, 360b (collectively "360") are coupled to the electrode main body 350. A pair of lines 370a, 370b (collectively "370") pass through the channel 340 and are coupled to the sensors 360a, 360b, respectively. In some embodiments, the sensor 360a is a contact sensor, and the sensor 360b is a temperature sensor, impedance sensor, and/or a pressure sensor. The number, positions, and types of sensors can be selected based on the treatment to be performed.

In multilayer embodiments, the electrode main body 350 can include at least one tube (*e.g.,* a non-metal tube, a plastic tube, etc.) with one or more films or coatings. The films or coatings can be made of metal, conductive polymers, or other suitable materials formed by a deposition process *(e.g.,* a metal deposition process), coating process, etc., and can comprise, in whole or in part, silver ink, silver epoxy, combinations thereof, or the like.

Radio-opaque markers or other types of visualization features can be used to position the main body 350. To increase visibility of the electrode 214 itself, the electrode 214 may be made, in whole or in part, of radiographically opaque material.

Figures 15-17 show one exemplary method of using a treatment system 200. A physician can visually inspect the airway 100 using a delivery apparatus 206 to locate and evaluate the treatment site(s) and non-targeted tissues before, during, and/or after performing a therapy. The airway 100 can be part of the trachea, main stem bronchi, or any other airway of the bronchial tree. A delivery apparatus 206 can be a bronchoscope, a guide tube, a delivery sheath, or an endoscope and can include one or more viewing devices, such as optical viewing devices (*e.g.,* cameras), optical trains (*e.g.,* a set of lenses), and the like. For example, the delivery apparatus 206 can be a bronchoscope having one or more lights for illumination and optical fibers for transmitting images. The catheter 207 may be adapted to be delivered over a guidewire (not shown) that passes between the balloon 212 and the energy emitter assembly 220. This provides for rapid exchange capabilities.

When the delivery apparatus 206 of Figure 15 is moved along a body lumen 101 (*e.g.,* an airway), the collapsed energy delivery assembly 208 is held within a working channel 386 of the delivery apparatus 206. The conduit 234 can form a loop 221 such that the electrode 214 is almost parallel to a long axis 373 when the catheter 207 is in a substantially straight configuration. In the illustrated embodiment of Figure 15, an angle β is defined between the direction of the long axis 373 of the catheter 207 and a long axis 374 of the electrode 214. The angle β can be in a range of about 0 degrees to about 30 degrees. In some embodiments, the angle β is in a range of about 0 degrees to about 20 degrees. The electrode 214, being curved, can also nest with and partially encircle the elongate shaft 230. In certain embodiments, at least a portion of the elongate shaft 230 is disposed within an arc of the electrode 214 for a further reduced profile. As such, the shaft 230 can be positioned between the ends of the electrode 214. Electrode 214 may have various lengths, depending on the desired length of the lesion to be created in each electrode position. In preferred embodiments, electrode 214 has a length of at least about 1 mm to about 4 mm. In certain embodiments, the length of the electrode 214 is about 2 mm up to about 3 mm. The electrode can have a width (or diameter if cylindrical) no larger than the width of the spaces between the cartilage rings, in some embodiments being about 0.1 mm to about 3 mm.

With continued reference to Figure 15, the diameter D_{L} of the working channel 386 can be less than about 8 mm. The diameter D_{B} of the deflated balloon 212 can be relatively small. For example, a minimum diameter D_{B min} can be in a range of about 2 mm to about 3 mm, and a maximum diameter D_{B max} in a range of about 5 mm to about 6 mm when the balloon 212 is fully collapsed. If the electrode 214 is collapsible, the diameter Dₘₐₓ of the assembly 208 can be less than about 3 mm. In ultra low-profile configurations, the maximum diameter Dₘₐₓ can be less than about 2.8 mm.

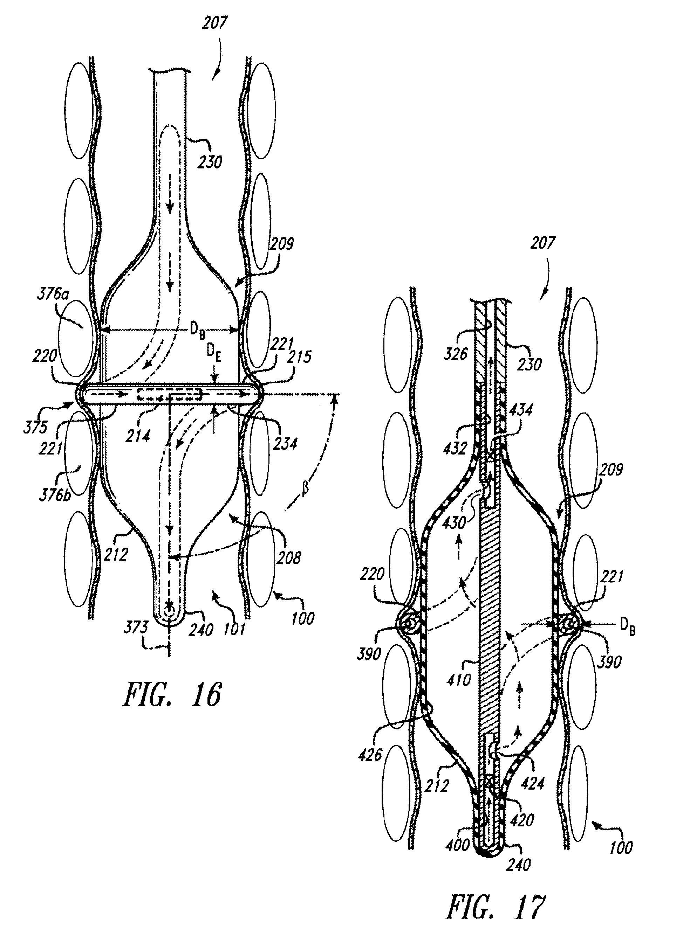

The balloon 212 can be inflated to move the energy emitter assembly 220 near (*e.g.,* proximate to or in contact with) the airway 100. The angle P can be increased between 70 degrees and about 110 degrees when the balloon 212 is fully inflated. Figure 16 shows the energy delivery assembly 208 deployed, wherein the electrode 214 can be about perpendicular to the long axis 373. There can be play between the energy emitter assembly 220 and the balloon 212 such that the angle β is in a range of about 60 degrees to about 120 degrees in order to accommodate variations of anatomical structures, misalignment (*e.g.,* misalignment of the catheter shaft 230), or the like. In some embodiments, the electrode 214 moves towards a circumferentially extending orientation as it moves from a delivery orientation to the deployed orientation. The electrode 214 in the deployed orientation extends substantially circumferentially along the wall of the airway 100. In certain embodiments, the electrode 214 will be configured to be positioned entirely within the spaces 375 between cartilage rings 376 along the airway wall when the energy delivery assembly 208 is in the fully deployed configuration.

Figures 16 and 17 show the energy emitter assembly 220 fluidically coupled to both the elongate shaft 230 and the balloon 212. Generally, coolant cools the tissue-contacting portion of the energy emitter assembly 220. The cooling section 209 of the energy delivery assembly 208 contacts the airway wall 100 so as to cool tissue adjacent to the tissue-contacting portion while energy is outputted by the electrode 214. The cooling section 209 can be formed by the portions of the energy emitting assembly 220 and the balloon 212 that contact the airway wall 100. If the electrode 214 faces an anterior region of the trachea 18, the assembly 208 can seat between cartilage rings 376 to avoid or limit movement of the electrode 214 along the length of the airway 100. If the energy delivery assembly 208 is positioned in the bronchial tree, especially in the main stem bronchi, the electrode 214 can be seated between spaced apart cartilage rings 376.

As the balloon 212 inflates, the electrode 214 moves (*e.g.,* pivots, rotates, displaces, etc.) from a first orientation of Figure 15 in which the electrode 214 extends axially along the airway 100 and a second orientation of Figure 16 in which the entire electrode 214 is disposed in a space 375 between adjacent cartilage rings 376a, 376b. The balloon 212 can both cool the airway 100 and cause the electrode 214 to seat in the space 375.

Figure 16 shows the energy emitter assembly 220 positioned to locate the electrode 214 in the space 375. In certain embodiments, the electrode 214, in the first orientation, extends a distance with respect to a longitudinal axis 373 (see Figure 15) that can be greater than the distance the electrode 214, in the second orientation, extends with respect to the longitudinal axis 373.

To deploy the energy emitting assembly 208, coolant from the elongate shaft 230 flows through the energy emitter assembly 220 and into the balloon 212. The electrode 214 can output a sufficient amount of energy to ablate a target region. The electrode 214 can be at a position corresponding to the anatomical location of at least one nerve in or proximate to the airway wall 100. The electrode 214 outputs energy to ablate the targeted nerve tissue. The coolant absorbs thermal energy from electrode 214 and the airway wall 100.

To treat tissue along the trachea, the diameter D_{E} of the electrode 214 and conduit 234 can be in a range of about 1.5 cm to about 2 cm when pressurized with coolant. In some embodiments, the diameter D_{E} of the electrode 214 and conduit 234 can be in a range of about 2 cm to about 2.5 cm to treat an average sized adult human. To treat tissue along one of the main stem bronchi, the diameter D_{E} can be in a range of about 1.5 mm to about 2.5 mm. Such embodiments are well suited to treat tissue outside the lung along the main bronchi. In certain embodiments, the diameter D_{E} is about 2 mm. In yet other embodiments, the diameter D_{E} can be in a range of about 0.1 mm to about 3 mm. The diameter D_{E} of the deflated conduit 234 and electrode 214 can be about 0.1 mm to about 1 mm. For example, to treat a bronchial tree of a human, the diameter of the inflated balloon 212 can be in a range of about 12 mm to about 18 mm. For enhanced treatment flexibility of the bronchial tree, the inflated balloon diameter may be in a range of about 7 mm to about 25 mm. Of course, the balloon 212 can be other sizes to treat other organs or tissue of other animals.

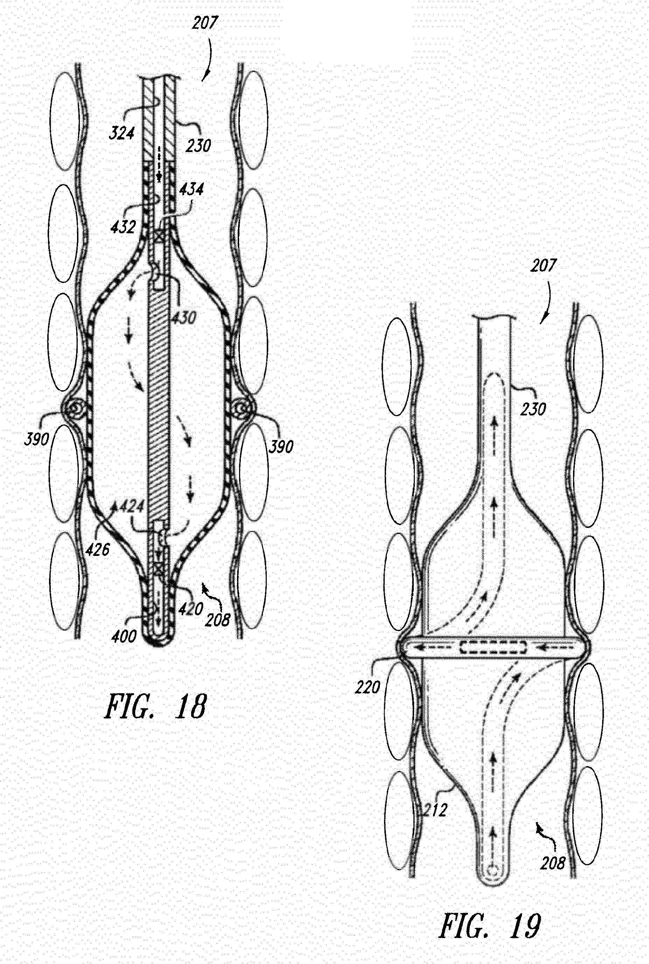

The energy delivery assembly 208 provides differential cooling because the coolant in the energy emitter assembly 220 is at a lower temperature and a higher velocity than the coolant in the balloon 212. Coolant, represented by arrows, flows out of the elongate shaft 230 and into the energy emitter assembly 220. The coolant proceeds through the energy emitter assembly 220 and the coolant channel 340 (Figure 14) of the electrode 214. The coolant absorbs thermal energy from the electrode 214. The heated coolant flows into the tip 240 and proceeds proximally through a lumen 400, as shown in Figure 18. The coolant flows through a valve 420 (*e.g.,* a throttle) and passes through a port 424. The valve 420 is disposed along a fluid path connecting the energy emitting assembly 220 and the portion of the balloon 212 defining the cooling section 209. The coolant circulates in a chamber 426 and absorbs heat from the tissue. This helps keep shallow tissue below a temperature that would cause cell death or tissue damage.

The coolant flows through a port 430, a lumen 432, and a throttle 434. The throttles 420, 434 can cooperate to maintain a desired pressure. The throttle 420 is configured to maintain a first flow rate of the coolant through the energy emitting assembly 220 and a second flow rate of the coolant through the cooling section 209. The first flow rate can be significantly different from the second flow rate.

The conduit 324 can assume a preset shape when pressurized. The valves 420, 434 can cooperate to maintain the desired pressure within the balloon 212 within a range of about 5 psig to about 15 psig. Such pressures are well suited to help push the electrode 214 between cartilaginous rings. Other pressures can be selected based on the treatment to be performed. The valves 420, 434 can be throttle valves, butterfly valves, check valves, duck bill valves, one-way valves, or other suitable valves.

When RF energy is transmitted to the electrode 214, the electrode 214 outputs RF energy that travels through tissue. The RF energy can heat tissue (*e.g.,* superficial and deep tissue) of the airway wall while the coolant cools the tissue (*e.g.,* superficial tissues). The net effect of this superficial and deep heating by RF energy and superficial cooling by the circulating coolant is the concentration of heat in the outer layers of the airway wall 100. Tissue structures can vary between different types of airways. In the bronchial tree, the temperature of the connective tissue can be higher than the temperatures of the epithelium, stroma, and/or smooth muscle. By example, the temperature of the connective tissue can be sufficiently high to cause damage to the nerve trunk tissue or other deep tissue while other non-targeted tissues of the airway are kept at a lower temperature to prevent or limit damage to the non-targeted tissues.

Heat can be concentrated in one or more of the internal layers (*e.g.,* the stroma) of the airway wall or in the inner lining (*e.g.,* the epithelium) of the airway wall. Furthermore, one or more of the vessels (*e.g.,* vessels of the bronchial artery) may be within the lesion. The heat generated using the electrode 214 can be controlled such that blood flowing through the bronchial artery branches protects those branches from thermal injury while nerve trunk tissue is damaged, even if the nerve tissue is next to the artery branches. The catheter 207 can produce relatively small regions of cell death. For example, a 2 mm to 3 mm section of tissue in the middle of the airway wall 100, along the outer surface of the airway wall 100, or between the airway wall 100 and other body tissue (*e.g.,* tissue of the esophagus) can be destroyed. By the appropriate application of power and the appropriate cooling, lesions can be created at any desired depth.

A circumferential lesion can be formed around all or most of the circumference of the airway wall 100 by ablating tissue while slowly rotating the energy delivery assembly 208 or by positioning the energy delivery assembly 208 in a series of rotational positions at each of which energy is delivered for a desired time period. Some procedures form adjacent lesions that become contiguous and form a circumferential band all the way around the airway wall 100. In some embodiments, the entire loop 221 (Figure 16) can be an electrode. The loop 221 can be coated with a conductive material and can carry the electrode. A single procedure can produce a circumferential lesion. After forming the lesion, coolant flowing into the balloon 212 can be stopped. The balloon 212 is deflated causing the energy emitter assembly 220 to recoil away from the airway wall 100. The catheter 207 may be repositioned to treat other locations or removed from the subject entirely.

If the user wants the coolant in the balloon 212 to be at a lower temperature than the coolant in the energy emitter assembly 220, chilled coolant can be delivered into the balloon 212 and then into the energy emitter assembly 220. Figures 18 and 19 show such a coolant flow. Low temperature coolant flowing through the elongate body 230 passes through the valve 434 and the port 430. The coolant circulates in the chamber 426 and absorbs heat. The heated coolant flows through the valve 420 and proceeds through the energy emitter assembly 220 to cool the electrode 214.

Airway cartilage rings or cartilage layers typically have a significantly larger electrical resistance than airway soft tissue (*e.g.,* smooth muscle or connective tissue). Airway cartilage impedes energy flow (*e.g.,* electrical radio frequency current flow) and makes the formation of therapeutic lesions with radio frequency electrical energy to affect airway nerve trunk(s) challenging when the electrode is next to cartilage.

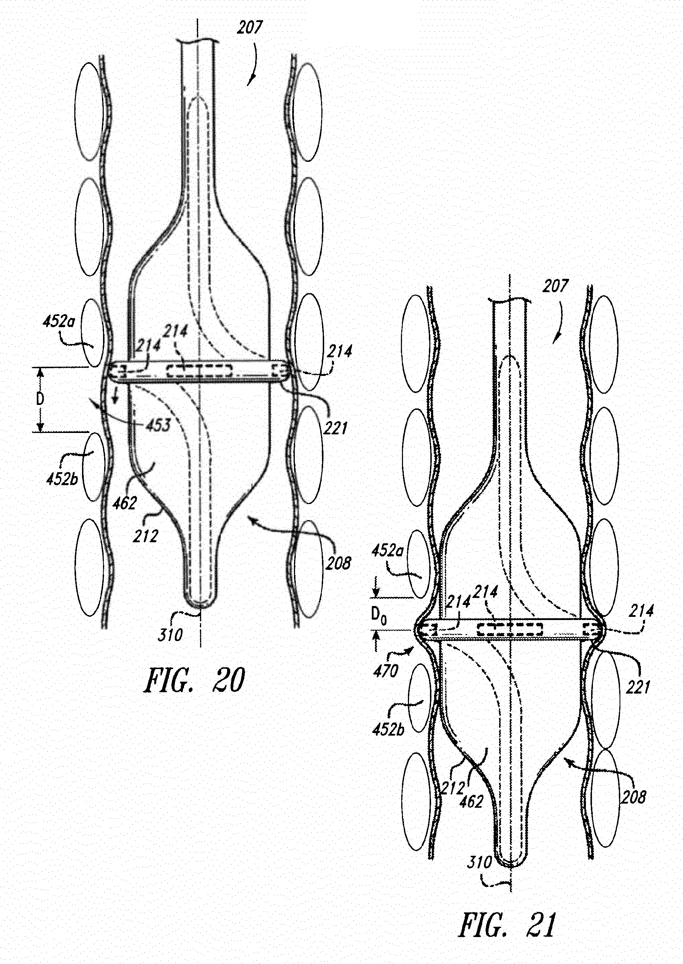

Positioners can facilitate positioning of the electrodes. Such positioners include, without limitation, bumps, bulges, protrusions, ribs or other features that help preferentially seat the electrode 214 at a desired location, thus making it easy to perform the treatment or to verify correct positioning. Figures 20 and 21 show the energy emitter assembly capable of serving as an intercartilaginous positioner. When the balloon 212 presses against the airway 100, the loop 221 moves along the balloon 212 to preferentially position the electrodes 214 between cartilage rings 452a, 452b. The loop 221 protrudes outwardly from the balloon 212 a sufficient distance to ensure that the energy delivery assembly 208 applies sufficient pressure to the airway wall to cause self-seating. The catheter 207 can be moved back and forth to help position the electrodes 214 next to soft compliant tissue 453 in the space 453. The energy emitter assembly 220 can be configured to displace a distance Dₒ (*e.g.,* measured along a long axis 310), which is at least half of the distance D between the cartilage rings 452a, 452b. This ensures that the electrodes 214 can be positioned generally midway between the cartilage rings 452a, 452b.

The plurality of electrodes 214 can reduce both treatment time and procedure complexity as compared to a catheter with a single electrode. This is because the multi-electrode catheter may have to be positioned a smaller number of times within a bronchial tree (or other hollow organ) as compared to single electrode catheters to produce a number of lesions of a desired therapeutic size. Multi-electrode catheters can thus precisely and accurately treat a user's respiratory system.

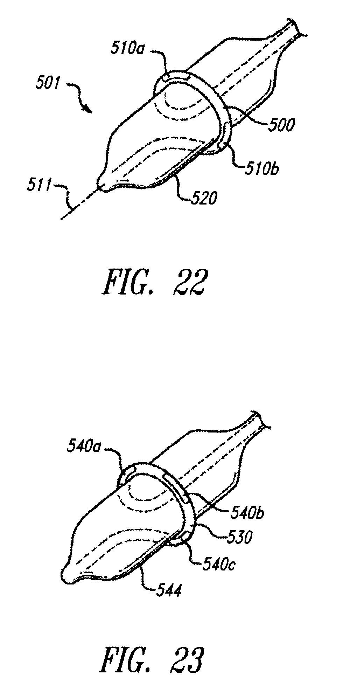

Figure 22 shows an energy emitter assembly 500 that includes two energy delivery elements including electrodes 510a, 510b spaced apart from one another about a circumference of a balloon 520. The electrodes 510a, 510b can be about 45 degrees to 210 degrees from another with respect to a long axis 511 of an ablation assembly 501. Other electrode positions are possible. Figure 23 shows an energy emitter assembly 530 with three energy delivery elements 540a, 540b, 540c positioned about 60 degrees from one another. In these embodiments, each electrode may be coupled to separate power lines to allow for independent control of each, or all electrodes may be coupled to the same power line so as to be operated together. Further, a pair of electrodes may be operated in a bipolar manner, wherein one electrode is positive and the other negative, with RF power being transmitted from one to the other through the tissue.

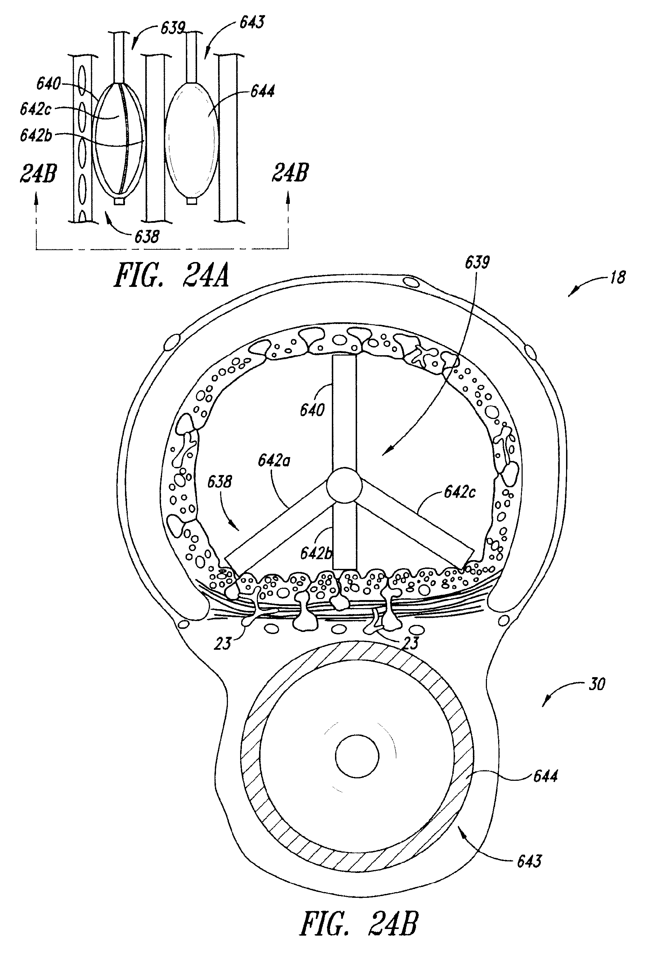

Figures 24A and 24B illustrate a portion of a treatment apparatus in the form of a tracheal device 639 in a delivered configuration for treating the trachea 18 in a monopolar fashion. The tracheal device 639 includes a basket 638 with a positioning member 640 and electrode members 642a, 642b, 642c (collectively "642"). The electrode members 642 can cooperate to treat the posterior plexus nerves 23. In this instance, an active device is placed in the trachea, with a ground pad placed on the patient's skin, typically in the thigh area. In order to prevent damage to the esophagus 30, a cooling or protection device is inserted into the esophagus 30. This device can be inserted through the mouth, or preferably, trans-nasally. The trans-nasal placement keeps the device separated from the manipulations of the device, to be placed in the trachea.

The basket 638 can be a cage or other type of self-expanding device. Advantageously, the basket 638 can be moved from a low profile (or collapsed configuration) to deployed state (or an expanded configuration) without the use of a balloon. Such non-inflatably expandable embodiments can be made of one or more shape memory materials (*e.g.,* Nitinol) capable of assuming different configurations. Additionally or alternatively, the basket 638 can be actuated using one or more pull wires or similar components.

A protection device in the form of a catheter 643 has a cooling balloon 644. In order for such an embodiment to efficiently circulate cooling media, the protection catheter 643 can include an inlet and an outlet to allow circulation of media (e.g., cooling media) through the balloon 644. The protective or cooling media is introduced through one lumen, allowed to inflate and circulate within the balloon 644, and exit through a second lumen. Additionally, the cooling media can be either gas or liquid, and can be chosen from a number of different varieties of either. Example gasses include room temperature or cooled air, nitrogen, cryogenic media, or the like. Example liquids include room temperature or cooled water, saline, ringer's solution, glucose solutions or the like.

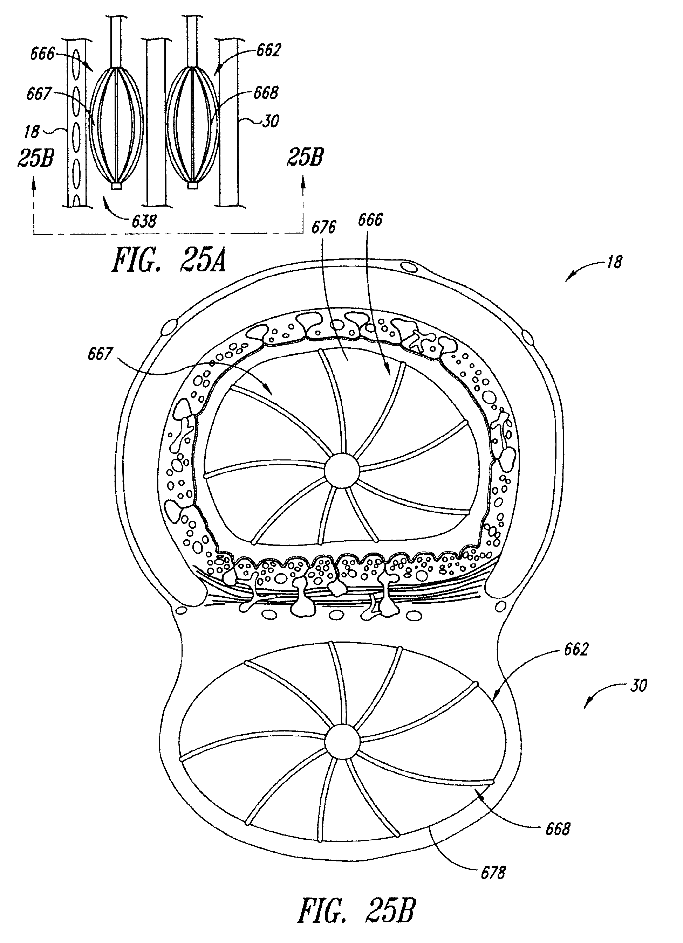

Whereas Figures 24A, 24B referred to above describe a monopolar device with esophageal protection, Figures 25A and 25B illustrate one of a group of embodiments which will be called the trachea-to-esophagus, or T:E devices. In these embodiments, devices 666, 662 are inserted into the trachea 18 and esophagus 30, respectively. The devices 666, 662 cooperate to form a therapy and protection system encompassing the use of both devices to send and receive energy to the targeted tissue, and to protect the non-target tissue as well, as desired and required.

The protection or cooling media in the two different devices 666, 662 can be set up to maintain the same level of protection in both devices and both structures, or they may be set to provide differential cooling to one structure over another. For example, it may be desirable to cool the esophagus 30 more than the trachea 18, in order to provide greater protection to the esophagus 30, and in order to locate the lesion within the tissue bridge between the structures biased toward the trachea side of the bridge. This might better target the neural plexus specifically, while providing greater safety to the esophagus 30.

In Figures 25A and 25B, two devices 666, 662, which may be essentially the same in design, are inserted into each of the lumens (trachea and esophagus). The devices 666, 662 have an optional central lumen for guide wire guidance, a balloon with inflation lumens, and optionally, a second lumen for circulation of protective cooling media, and outer electrodes 667, 668. In the embodiments of Figures 25A and 25B, the outer electrodes 667, 668 are comprised of a cage of wires surrounding balloons 676, 678. Each cage can be deployed by the respective balloon 676, 678 directly, or they can be made of a suitable shape memory alloy to allow them to expand to contact the tissue independent of balloon action. The electrodes 667, 668 can be comprised of any suitable conductive material, including stainless steel, chromium cobalt, nickel titanium, metal-loaded conductive polymers, or the like. One of the devices can be attached to the energy delivery aspect of a delivery control box, and one acts as the return electrode. Depending on the specific energy density desired, the active device can placed in either the trachea 18 or the esophagus 30, and the return in the other. A cooled fluid may be circulated through balloons 676, 678 to absorb heat from energy delivery elements including electrodes 667, 668 and from the tissue of the esophageal and tracheal wall. During treatment, the balloons 676, 678 can be inflated to physically contact the inner surfaces of the trachea 18 and esophagus 30, respectively. The balloons 676, 678 have a generally circular shape as viewed along the lumen of the trachea 18, similar to the embodiments shown in Figure 24B. The balloons 676, 678 can have transverse cross-sections that are substantially circular, elliptical, polygonal, or combinations thereof and can have a smoother exterior surface, roughened exterior surface, undulating or wavy exterior surface, or the like. The electrodes 667, 668 deliver energy directly to the tissue. In other treatments, the balloons 676, 678 can be smaller than the lumens of the trachea 18 and the esophagus 30.

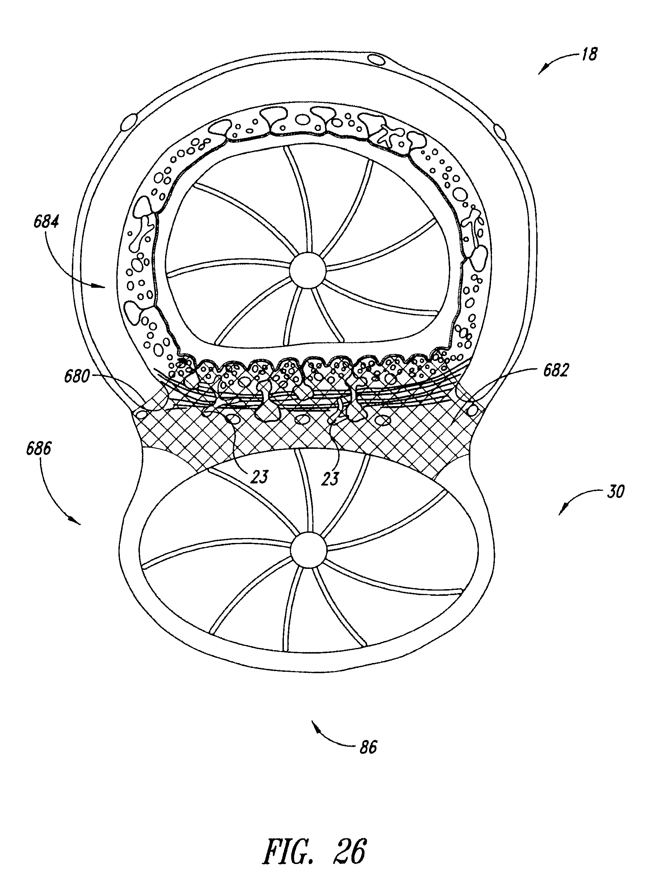

Figure 26 shows the energy distribution around the esophagus 30 and trachea 18 as may be produced by a system as described in Figures 25A and 25B. An area of high energy density 680 (shown hatched) exists in the tissue bridge 682 between the two structures, with relatively lower energy density 684, 686 (shown non-hatched) in other tissues around the perimeter of each of the individual structures. Without cooling, the tissue of the high energy density region 680 is ablated or otherwise altered (*e.g.,* damaged, destroyed, etc.) and preferably includes the posterior plexus nerves 23. In certain treatments, all of the posterior plexus nerves 23 between lumens of the trachea 18 and the esophagus 30 are damaged. In other treatments, targeted posterior plexus nerves 23 are damaged. If cooling media is circulated through one or both balloons, 676, 678, the tissue near the inner surface of the tracheal wall, as well as the tissue of the esophagus, can be protected from injury, while ablating target nerve tissues. Energy delivery and cooling may be adjusted to produce the isotherms of Figures 8A and 8B which are well suited for targeting damage to the interior tissue, such as the posterior plexus nerves 23, without damaging other tissue of the trachea 18, esophagus 30, and bridge 682.

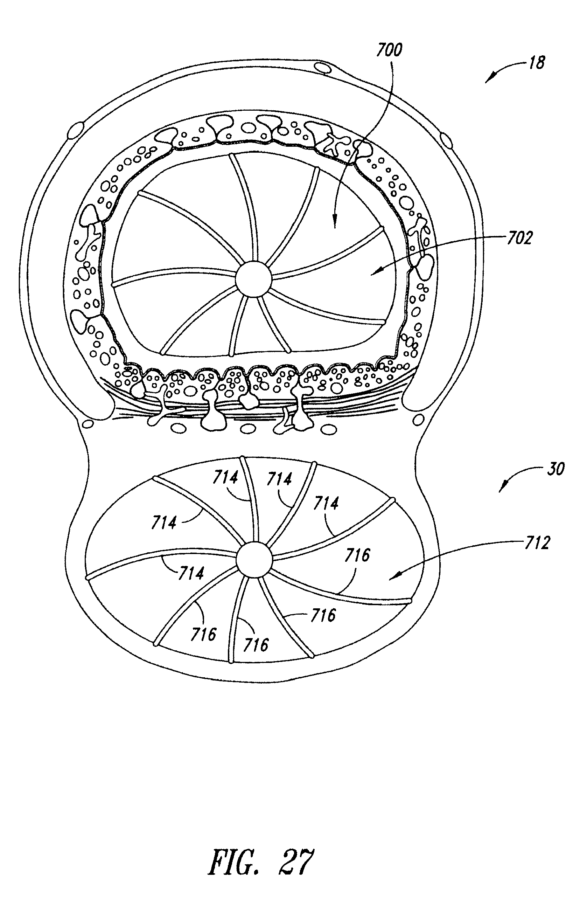

An embodiment designed to optimize energy density around the trachea 18 is shown in Figure 27. In this embodiment, the active electrodes 700 of a device 702 are arranged around the entire circumference in the trachea 18, and the return electrodes 714 are disposed only on the anterior aspect of the esophageal device 712. In this case, the anteriorly oriented support electrodes 714 are conductive, while the posterior and optionally the posterior-lateral elements 716 are non-conductive. To render them non-conductive, they could simply be insulated from the return leads at the points of connection at the distal and proximal ends of the balloon, insulated over the length of the members via insulating shrink tubing, polymer coextrusion or coating, or made of completely non-conductive materials, such as an extruded polymer.

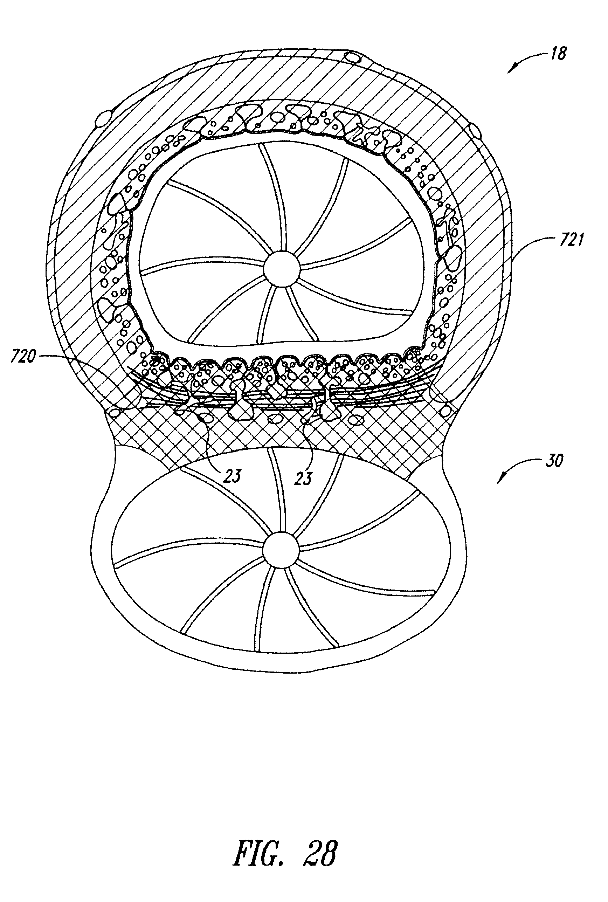

Figure 28 illustrates a resultant energy density distribution that may be created by the system of Figure 27. A relatively high energy density 720 (shown hatched) develops between the trachea 18 and esophagus 30, in the area of the posterior plexus 23, with a slightly lower density 721 developing around the lateral and anterior aspects of the trachea 18 (still sufficient to ablate the anterior plexus), and almost no field develops around the majority of the circumference of the esophagus 30. By circulating cooling media through the balloon of the esophageal device, the tissue of the esophagus may be protected from injury. Further, by circulating cooling fluid through the balloon of the tracheal device, the surface tissue on the inner wall of the trachea may be protected.

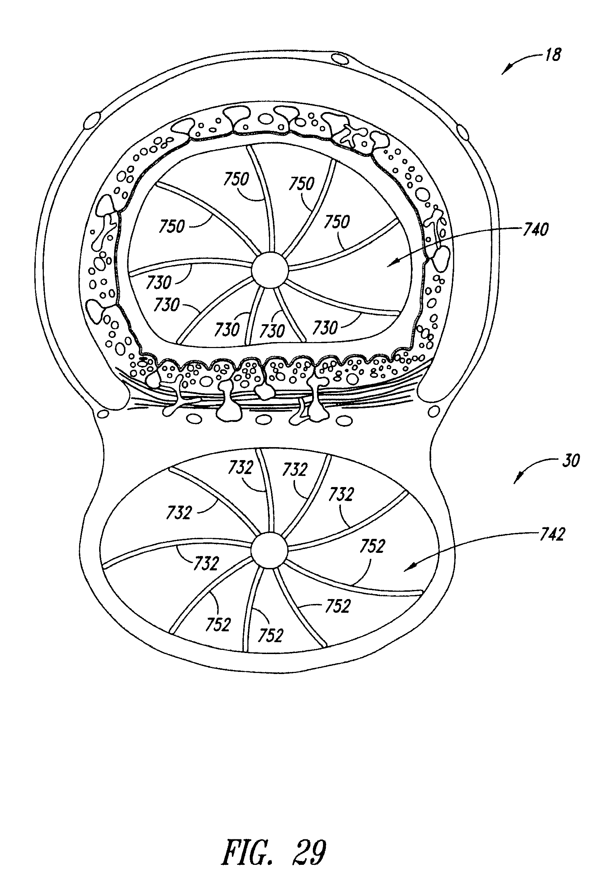

A further localization of the energy field may be achieved through alternative embodiments, for example, as shown in Figure 29. In this embodiment, the active electrodes 730, 732 are confined to the posterior aspect of the tracheal device 740 and the anterior aspect of the esophageal device 742. The opposing arms 750, 752 of the devices 740, 742 can be passive (*e.g.,* ground electrodes). All of the aforementioned alternatives for achieving this electrode localization apply, as well as those describing the potential differential cooling/protection options.

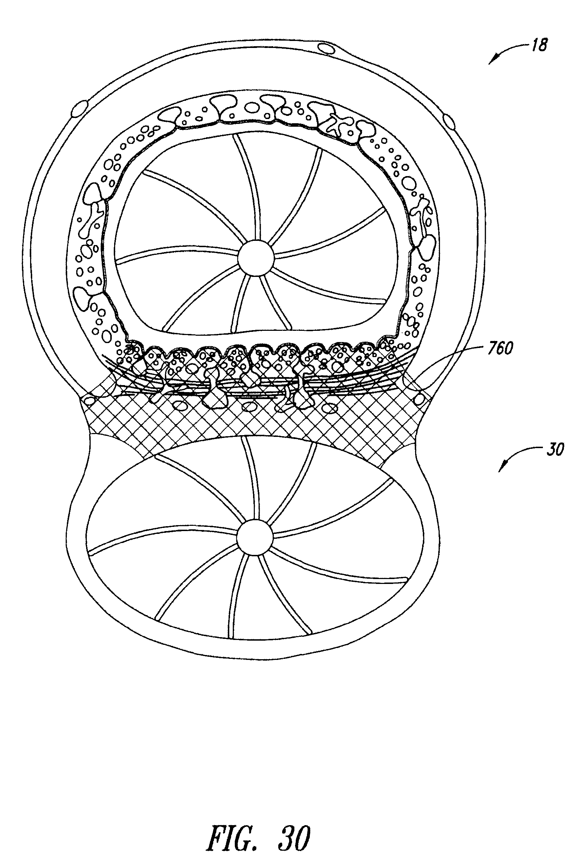

Figure 30 illustrates an energy density localization as may be achieved by the embodiment of Figure 29. Such embodiments localize the energy density in the region 760 between the trachea 18 and the esophagus 30, and target more specifically the posterior plexus. Again, esophageal cooling may be applied to minimize damage to esophageal tissue.

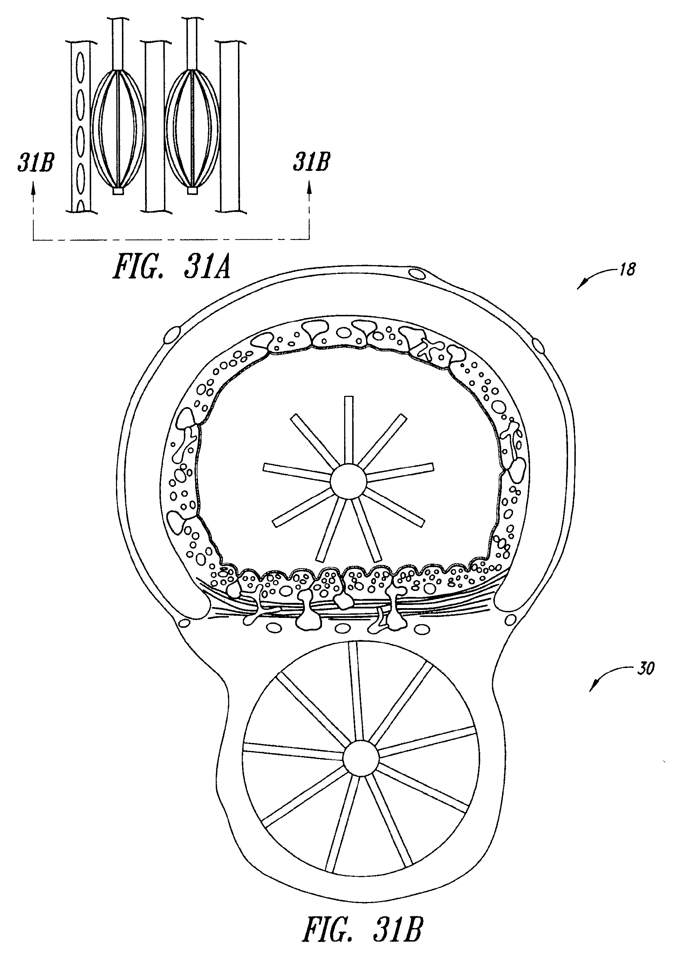

It should also be appreciated that any of the above balloon supported embodiments (Figures 25A through 30) can be made with the electrode and support elements only without the use of balloons, and can be made to create the same ablation patterns seen in all of the above balloon supported embodiments. For example, Figures 31A and 31B illustrate an alternative embodiment similar to the embodiment described in connection with Figures 29 and 30, but in a non-balloon-supported embodiment. An energy density distribution pattern such as shown in Figure 30 also may be produced by the embodiment of Figures 31A and 31B.

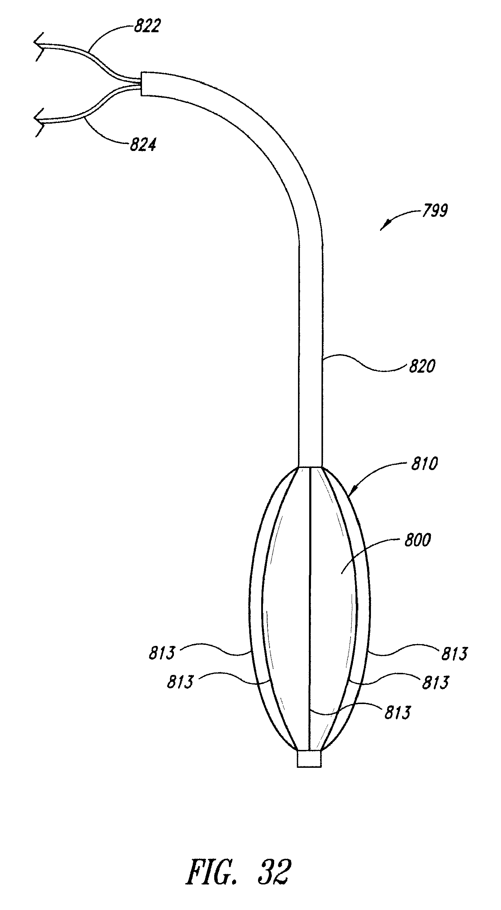

Figure 32 illustrates an embodiment of the present invention in side elevation that may correspond to the types of device described in the previous embodiments. Note that in Figure 32, the device 799 includes a balloon 800 shown in conjunction with the basket electrode array 810. In some embodiments, as described, the balloon 800 is eliminated and the basket array 810 is carried directly on a central shaft 820. The basket array 810 includes a plurality of flexible, resilient, elongated electrode struts 813 oriented in a longitudinal direction and arranged around the circumference of shaft 820. Electrode struts 813 bow outwardly into an expanded, arcuate shape either under the expansion force of balloon 800, or by pulling on the distal ends thereof in a proximal direction, whereby electrode struts 813 bow outwardly under compression. The device 799 includes in inflow conduit 822 and an outflow conduit 824 used to circulate media through the balloon 800.

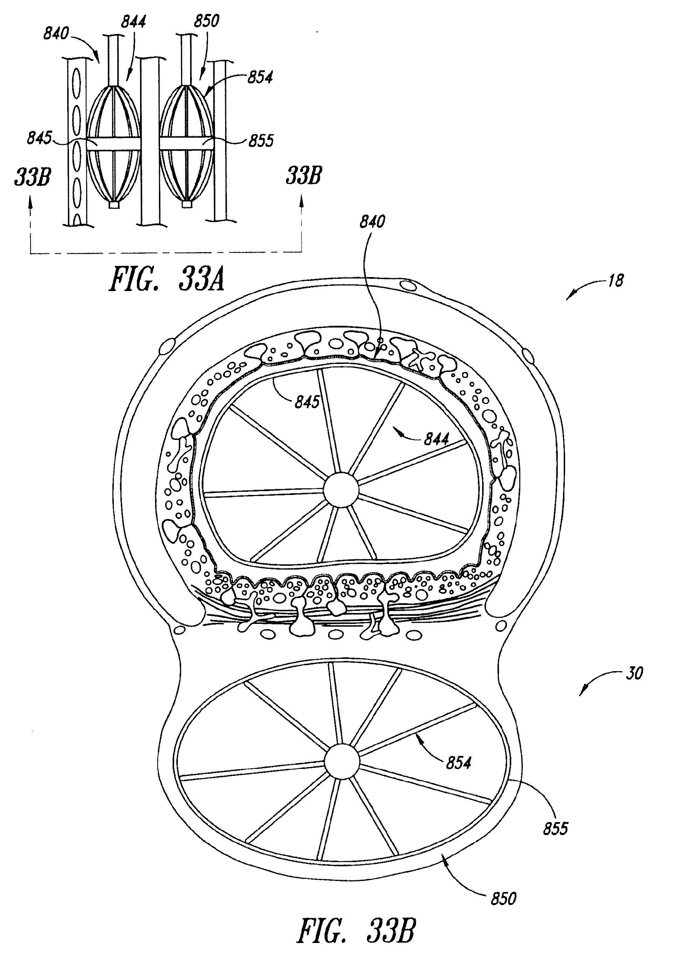

Other variations of the embodiments described so far are shown in Figures 33A and 33B. In Figures 33A and 33B, a tracheal device 840 includes a support cage 844 which carries on its periphery a circumferential band 845 that can be selectively insulated and energized to create any of a variety of energy density patterns, including those shown in Figures 26, 28 or 30. The band 845 can be a conductive flexible member that is in the form of a conductive strip, tubular band, or the like. The band may have one or more discontinuities or a sinusoidal or other shape to allow it to expand circumferentially. The band 845 can be movable from a contracted configuration to an expanded configuration. Spaced apart struts of the support cage 844 extend radially outward to the circumferential band 845. Any number of bands of different sizes and configurations can be carried by the cage 844.

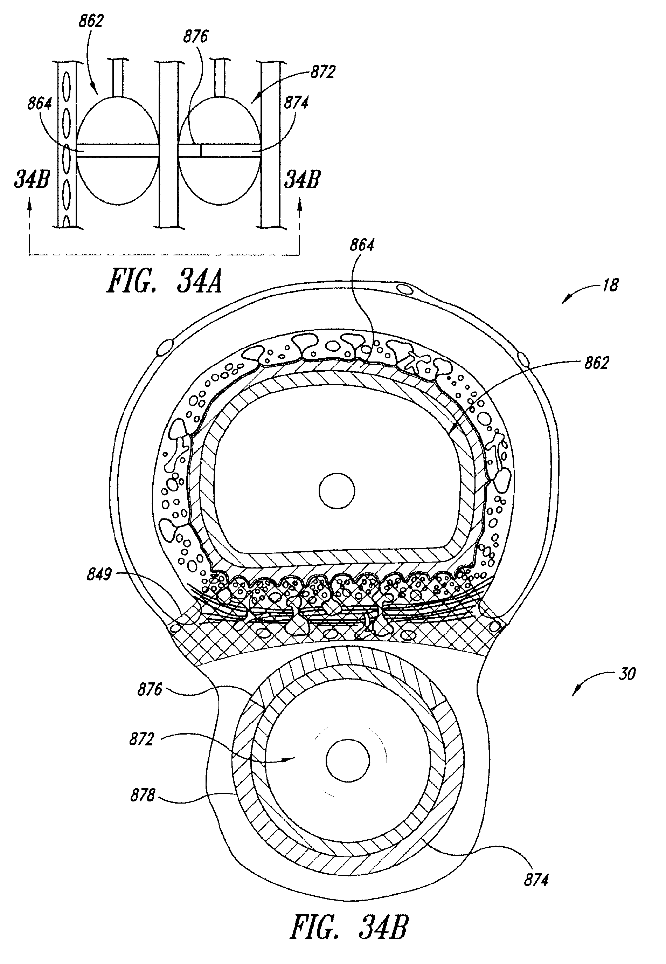

The esophageal device 850 includes a support cage 854 that may also carry on its periphery a circumferential band 855 that can be selectively insulated and energized to create any of the energy density patterns shown in Figures 26, 28 or 30 or a variety of other patterns. Similarly, the support structures 844, 854 for the circumferential band of Figures 33A and 33B could be replaced by a balloon 846, as shown in Figure 34A and 34B. Figure 34B also show one possible energy density pattern, including high energy density region 849 (shown hatched), achieved by the embodiments in either Figures 33A-33B or Figures 34A-34B.

A tracheal device 862 of Figures 34A and 34B can include a band 864 with an active electrode. In some embodiments, the entire band 864 is an electrode. In other embodiments, one or more portions of the band 864 can be electrodes while other portions are insulated. A device 872 includes a band 874 with an active portion 876 and a passive portion 878. The active portion 876 can be an electrode that cooperates with the band 864 to target the posterior pulmonary plexus or other target region. The bands 864, 874 can be portions of a balloon or other type of inflatable or expandable member. In some embodiments, the walls of the balloons include electrodes mounted or adhered thereto. The balloon (wire basket or cage) can be an actuable device movable between a delivery configuration and a deployed configuration to move the band 874.

Eliminating the balloon in the longitudinal support structure embodiments described above may require different means for providing cooling or protection. Further description of such alternative embodiments are provided later in the present disclosure.

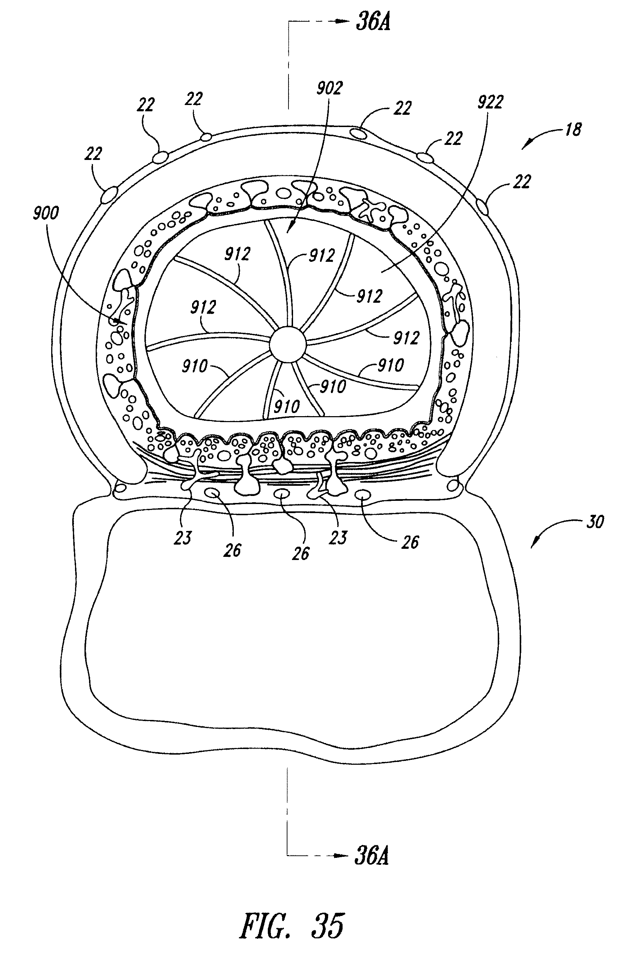

Embodiments described to this point have either shown monopolar devices within the trachea, or bipolar devices which energize from trachea to esophagus, or vice versa. Figure 35 illustrates a further embodiment whereby bipolar energy can be delivered from within the trachea 18 alone, in order to concentrate the energy density around the circumference of the trachea 18 and target both the anterior plexus 22 and posterior plexus 23, with potentially higher energy density than would be achievable by monopolar energy alone.

In the embodiment of Figure 35, a device 900 includes an electrode array 902 that is divided into two distinct sections, wherein one section serves as the active electrodes 910 and the other section serves as return electrodes 912 (*e.g.,* ground electrodes). In this way energy may be delivered from active electrodes 910 to return electrodes 912 via the tissue in the tracheal wall to produce the desired energy density pattern. Other aspects of electrode design and material selection previously described apply to this embodiment as well.

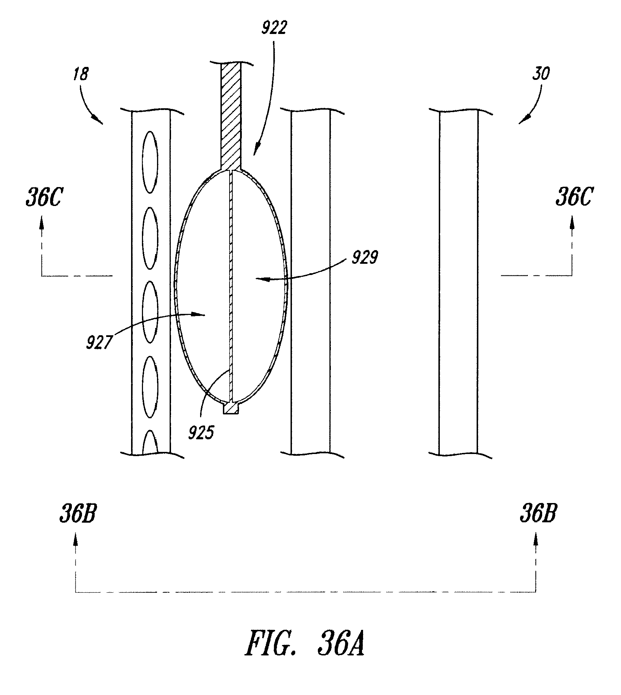

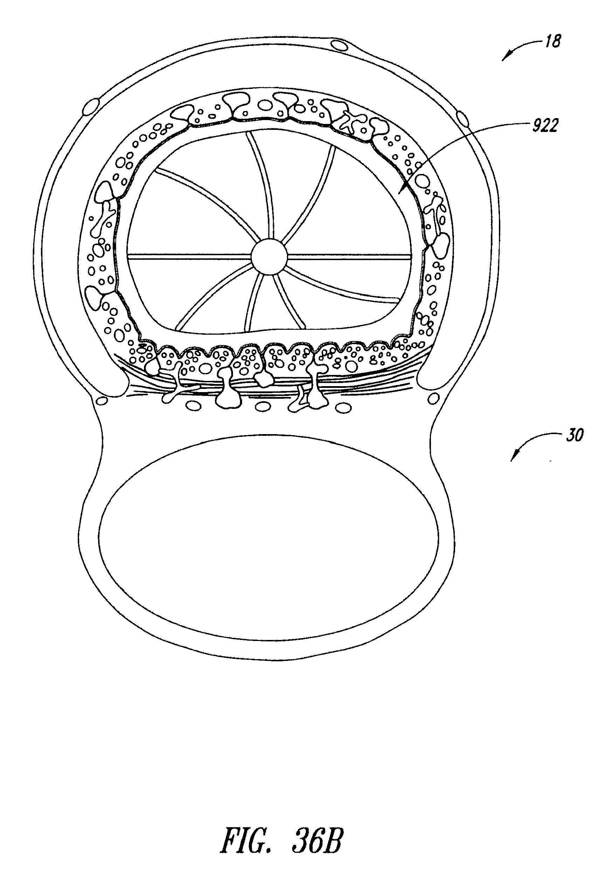

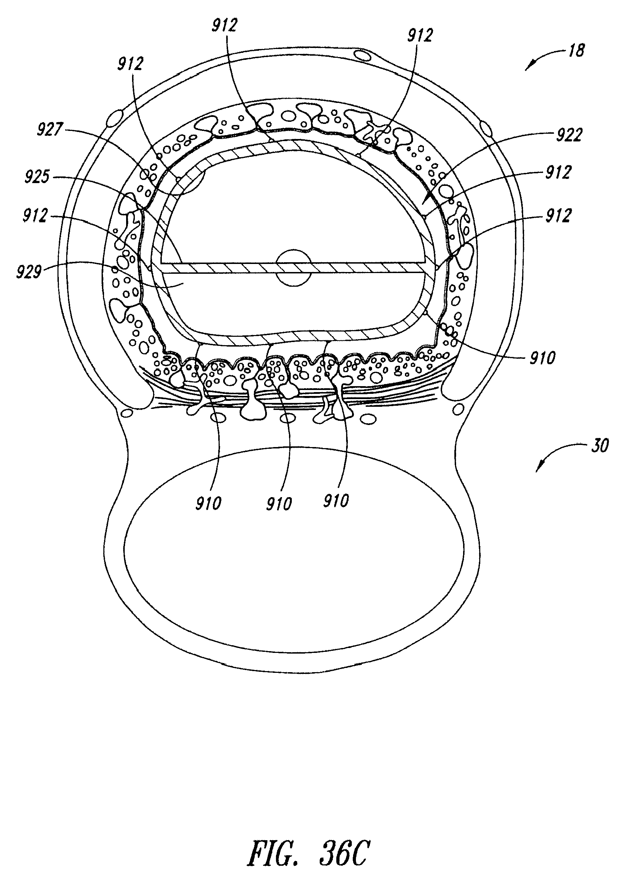

Figures 36A-36C show a variation of the bipolar system in Figure 35. The system includes a basket-type electrode array as described in previous embodiments having a plurality of electrode bands. The electrode array is disposed around a balloon 922. The balloon 922 is divided into different sections by a septum 925 within the balloon 922. The septum 925 divides chambers 927, 929. Fluid at different temperatures can be delivered to the chambers 927, 929 to provide differential cooling between opposing surfaces of the balloon 922. In a further alternative, there would be a dual balloon system having one balloon facing the anterior and one balloon facing the posterior portion of the trachea 18. Different temperatures or different flow rates of media can be introduced into the different cooling/protection zones in order to provide greater protection for one area than the other. This differential in temperature profiles can also be used to direct the area of ablation more deeply into the wall of the trachea 18, directing it more towards the nerves. For example, if the nerves 23 on the posterior side are more deeply embedded in the bridge tissue between the trachea 18 and esophagus 30, more cooling might be desired here than on the anterior side. Another scenario is one in which the user only wants to protect the superficial mucosa on the anterior side, and so a comparatively low level of protection is required. On the posterior side, on the other hand, more protection may be required to preserve the integrity and function of the esophagus 30, and to prevent fistulas from occurring. A wide range of different types of split or multi-chambered inflatable members can be used.

It can also be appreciated that embodiments disclosed herein, such as the embodiment of Figures 36A-36C, which occlude the lung during treatment, can be deployed and retracted in order to allow for ventilation. Alternatively (not shown), any of these occlusive devices can be designed with a lumen or lumens which provide flow through the devices, allowing for ventilation of the lung distal to the occlusion site. Room air, oxygen or the like can be supplied to the distal lung.

The following family of designs shares a common attribute in that they take advantage of the cartilaginous rings which surround the upper airways to actually locate the delivery portions between the insulating rings, directing the energy directly into the only weakness in the wall of the airway from which the energy can reach the nerves on the anterior side.

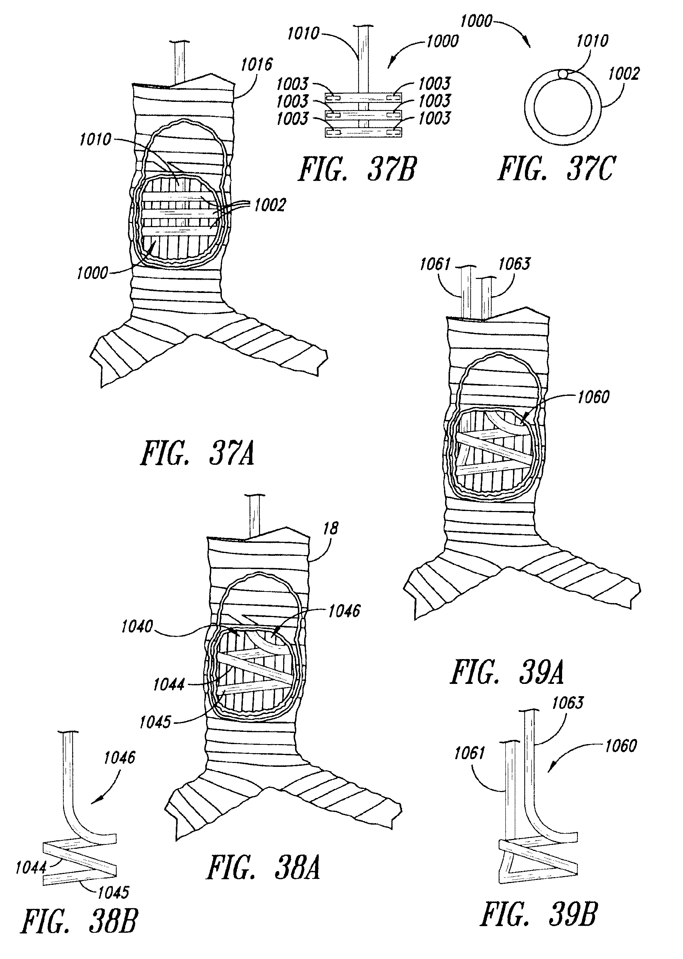

Figures 37A-37C illustrate an embodiment with a device 1000 that includes a stack of a plurality of ring electrodes 1002 attached to a central or offset shaft 1010 which lends support and provides electrical connection to the control box of the system. The illustrated ring electrodes 1002 extend circumferentially about the inner wall of the trachea. The shaft 1010 extends vertically from the rings along a lumen of the trachea. The diameter and width of the ring material is chosen such that it fits entirely or substantially within the gap between two adjacent cartilaginous rings.

The diameter of the rings 1002 can be set to slightly oversize or to roughly match the diameter of the airway 1016, as shown in Figure 37A. The rings 1002 themselves may be resilient and expandable similar to a self-expanding vascular stent such that, regardless of airway diameter, they expand to fill the airway circumference. Various designs and methods to vary the diameter of the rings 1002 can be employed in these designs. For example, one end of a given ring may be fixed to the longitudinal spine of the device, and the other formed to engage another longitudinal element which winds the ring down into a smaller diameter for more distal placement (not shown).

The impedance sensors 1003 (shown in dashed line) of Figure 37B detect the impedance of the tissue of the airway wall and any external structures that may be in contact with the airway wall, such as the pulmonary artery or esophagus. Each of the various tissues and fluids in and surrounding the airway, such as smooth muscle, cartilage, nerves, blood vessels, mucous, air, and blood, has a different impedance. Moreover, previously treated (ablated) tissue will have different impedance than untreated tissue. Thus, the longitudinal and rotational position of the sensor (and hence the electrode) may be detected by measuring the impedance at the location and comparing it to a reference value or to the impedance of tissue at other locations. In this way, the power level or degree of cooling or both may selected based upon the location of the electrode to ensure target nerve structures are ablated without damaging other critical structures such as the esophagus. In addition, the presence of previously created lesions may be detected so that overlapping such lesions and over-treating tissue can be avoided.

Impedance sensors 1003 may be adapted to be manually activated by the user at any particular electrode location. Alternatively, the system may be configured to run the sensors continuously or automatically trigger them prior to or simultaneous with energy delivery through the electrode at each treatment location. Prior to energy delivery, the system may provide an indication of the impedance to the user so that power or coolant delivery may be adjusted, or the system may automatically adjust the power delivered through the electrode based on the measured impedance.

Impedance may also be detected using the electrodes themselves without a separate sensor. The RF generator may be equipped with an impedance detection system which calculates the impedance seen by the electrode when power is delivered. In this way prior to lesion creation at any particular location a very low power signal may be delivered from the electrode and impedance then calculated to ensure proper positioning and power settings.

In use, the rings 1002 are deployed within the desired treatment area. They can be delivered within a sheath or tubular cannula in a compressed state and released when in position to expand into contact with the airway wall. Once deployed, the system is withdrawn proximally, or pushed distally by a small amount. Tactile feedback lets the physician know when the rings have slipped into place. In some embodiments, an active electrode is configured to fit between a first pair of adjacent cartilage rings of the airway in the expanded configuration. Return electrodes arc configured to fit between a second pair of adjacent cartilage rings of the airway while the active electrode is positioned between the first pair of adjacent cartilage rings. Alternatively, tissue impedance can be measured, with lower impedance signaling the electrodes are between rings, and in position to access the nerves.

As an alternative to the stacked ring design, a coil could be formed to provide the same inter-cartilaginous locking functionality as the stacked ring design. Figure 38A shows a device 1040 that includes a coiled or corkscrew-shaped ring 1044. The pitch of the coils 1044 is set such that adjacent turns of the coil lock into separate neighboring inter-cartilaginous regions. In one version of the coiled ring design, a length of resilient coil is provided straightened out inside of a delivery catheter or capture sheath. When the distal tip of the capture sheath is in place at the distal end of the treatment region, a distal tip 1045 and the coils 1044 are delivered to the distal end of the treatment region. The capture sheath is withdrawn until the entire treatment area of interest is covered by the coiled elements. Again, tactile feedback confirms that the rings are locked into place, or impedance is measured. A shaft 1046 extends from the coiled ring 1044 along the lumen of the trachea.

Figures 39A and 39B show another embodiment of the coiled ring system 1060 wherein the distal and proximal ends of the coils are both attached to longitudinal members. Coil diameter can be varied by twisting the two elements relative to one another in order to tighten or loosen the diameter of the coils. The coils can seat between the cartilage rings. The system 1060 includes a winding arm 1061 and a proximal electrode 1063.

The locking ring electrode concept can be incorporated into a number of the previously described tracheal-esophageal embodiments in order to recreate the energy density distributions shown in Figures 26, 28, and 30. A ring-type device in the lung could be used in combination with any of the previously described esophageal devices to provide esophageal cooling, or to provide esophageal electrodes for a bipolar delivery system.

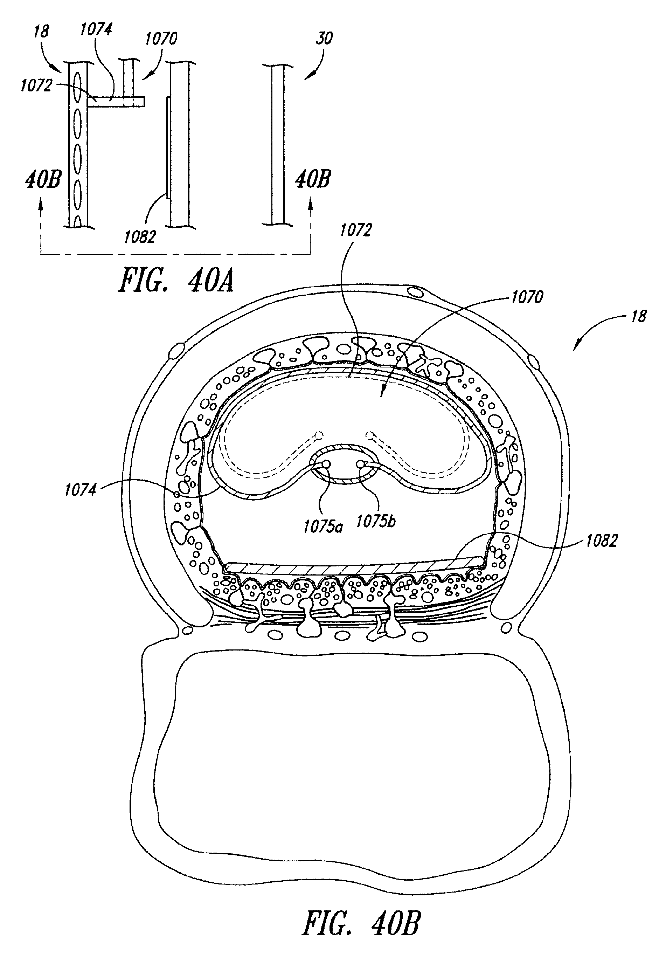

Another variation of the locking ring embodiment is shown in Figures 40A and 40B. In this case, a device 1070 includes an anterior portion 1072 defined by a resilient member 1074 formed into a roughly "D" or kidney-shaped or rabbit ear-shaped member or ring. The ends of member 1074 may be wrapped around two independently rotatable longitudinal members 1075a, 1075b, so that the size and shape of the "D" can be modified by rotating the longitudinal members 1075a, 1075b to wrap or unwrap the resilient member. For example, rotating the left longitudinal member 1075a counterclockwise and the right one 1075b clockwise in Figure 40B would result in the D ring reducing in size (as shown by the dashed lines).

A plurality of these D-rings can be attached above or below one another in a configuration similar to the one shown in Figure 37A, and if desired can all be made expandable and contractible as described above. If a bipolar energy pattern is desired, a second set of D-rings can be positioned to contact the posterior wall of the trachea as well (not shown). The anterior and posterior rings can be alternated, or interleaved, such that each subsequent ring faces the opposite direction, or a series of rings can face one direction, and then a separate series of rings faces the opposite direction. The latter configuration provides longitudinal separation of the active and return electrode as well as the anterior/posterior separation provided by the interleaved design.

Alternatively, as shown in Figures 40A and 40B, a non-ring electrode 1082 can be used along the posterior aspect of the trachea 18. Since there are no cartilaginous rings on the posterior aspect, an electrode 1082 can be in the form of a mesh electrode, arrays of longitudinal spine electrodes, or any other suitable electrode design can be used in conjunction with the ring or D-ring electrodes described above to allow bipolar energy delivery.

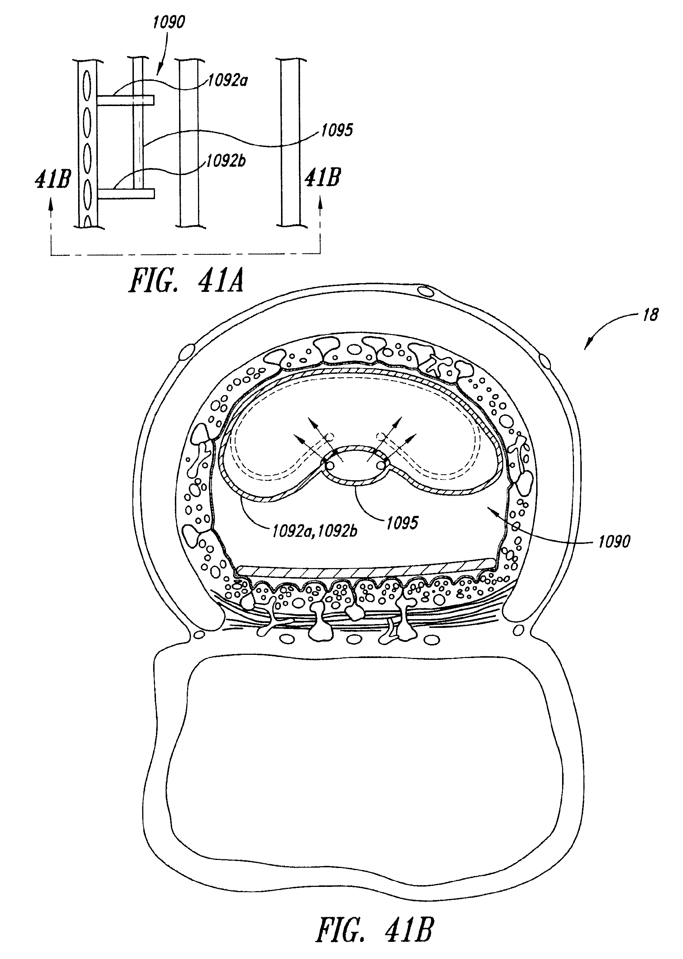

Figures 41A and 41B illustrate a further alternative device 1090 that includes holes or vents for introduction of cooling media, and a plurality of spaced apart ring electrodes 1092a, 1092b. Cooling vents may be disposed in the shaft 1095 to which the electrodes 1092a, 1092b are attached. Through these vents cooling or protectant media (represented by arrows) can be directly applied to the electrodes and/or to the tissue adjacent to the electrodes. The media can be any of the aforementioned media. Alternatively, any of the vented designs described in this disclosure can use a liquefied gas wherein the gas flows into the system liquefied and cools via an endothermic phase transition.

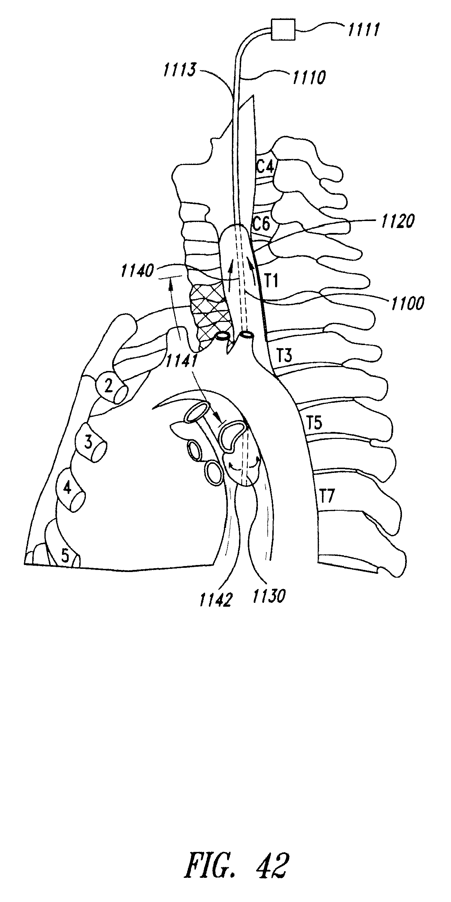

In another exemplary embodiment, shown in Figure 42, the esophagus is protected by an esophageal device 1100 in the situation where the tracheal device (not shown) alone is involved in the modification or ablation of the nerves. The tracheal device can be monopolar RF, bipolar RF with both leads in the trachea, or microwave.

The embodiment of Figure 42 is shown to cover a substantial portion of the entire zone of the esophagus 1141 which could potentially suffer tissue damage from a delivery device positioned within the trachea. This affords protection of the entire exposed esophageal territory with a single device placement. Alternatively, the esophageal device could be made shorter, and moved either in concert with, or at appropriate intervals to the movement of the tracheal device. Such an embodiment may include features such as an elongate shaft to insert the balloon and circulate cooling fluid through a balloon 1142, multiple lumens to effectively circulate protectant, and/or an optional guide wire lumen to aid in placement of the device.

Although there is an area of the trachea shown in crosshatch Figure 42 as the treatment area of the trachea, it should be noted in this and all figures that show exemplary treatment areas that this area is not the only potential treatment area. It is shown merely to point out that in some embodiments the esophageal device covers substantially the entire potential intended treatment zone.

A catheter shaft 1113 of Figure 42 is connected to a generator/pump unit and can be a multi-lumen shaft to allow bidirectional fluid flow. In certain embodiments, the catheter shaft 1113 has two lumens coupled to side holes. Fluid can be delivered into a proximal balloon end 1142 through one lumen. Media can be circulated within the balloon 1142 to cool the tissue surrounding the esophagus. The media can flow out of the balloon 1142 using the other lumen.

The catheter shaft 1113 can have a sealed tip 1130. A fluid can be delivered through the chamber of the balloon 1142 and returned via the body 1110. One or more conductive elements 1140 can be positioned to be adjacent to or to contact the potential ablative zone. During ablation, the conductive element can help conduct heat between the tissue and the cooling media circulating within the expandable balloon 1142 covering the potential ablative zone 1141.

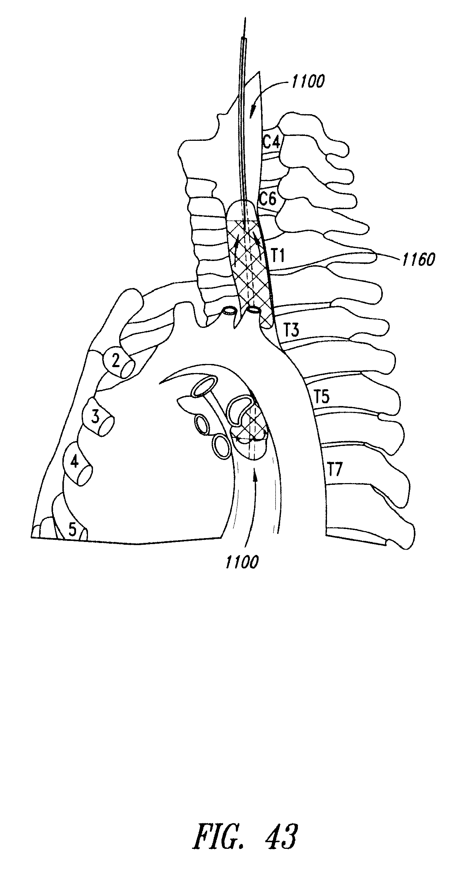

The exemplary embodiment illustrated in Figure 43 is a variation of the embodiment of Figure 42, in which conductive means are added to the basic protection system to allow for bipolar trachea-to-esophagus treatment options. All of the previously mentioned features and benefits apply the embodiment of Figure 43 as well. While Figure 43 shows a circumferential conductive zone, such as a wire mesh 1160 on the device, it should be appreciated that any of the conductive elements described herein (wire cages, ring electrodes, etc.) could be configured onto the protective device 1100. In the case where the protective device is long enough to cover substantially all of the potential treatment area, the conductive elements of the protective device will also cover substantially the entire potential treatment zone.

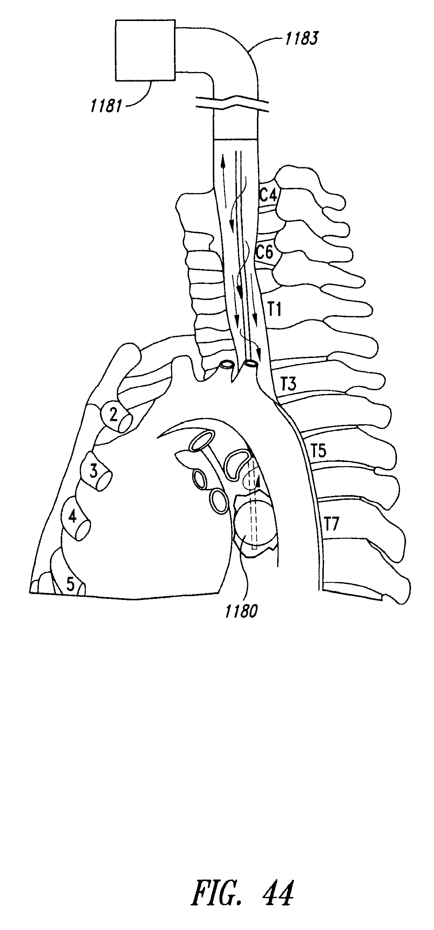

Figure 44 illustrates another alternative embodiment including means for protecting the esophagus during nerve modification. In this case, a relatively short occlusion device 1180 is delivered to the esophagus distal to the most likely termination of the potential treatment zone. Behind this occlusion device 1180, protectant media is circulated freely in the esophagus. In this embodiment, cooled gasses are most likely to be used. Room air, nitrogen, oxygen, etc., may be used. Forced media (*e.g.,* forced cool air) can be circulated above the occlusion device 1180 illustrated as a balloon. A wide range of different types of sources 1181 with one or more pumps (*e.g.,* piston pumps, positive displacement pumps, roller pumps, etc.) or blowers can pass media through a conduit 1183. The illustrated conduit 1183 is positioned in the lumen of the esophagus 30 to circulate the media in the lumen of the esophagus 30. The media can flow at a relatively high flow rate to protect the trachea and/or esophagus. The occlusion device 1180 prevents media from distending the stomach and/or the gastrointestinal tract.

As shown in the exemplary embodiment of Figure 44, the occlusion device 1180 is a balloon, but other devices which provide substantial blockage to the passage of gas can be used. Additionally, Figure 44 shows the protectant being introduced via the nose or the mouth directly. Custom nose plugs or facemasks can be designed to effect this delivery. For example, a pump or blower can deliver chilled media to the airway or esophagus of the patient via a facemask. Alternatively (not shown), side holes in the shaft of the occlusion device can be used for introduction of protectant. In this case, liquefied gas that is allowed to warm in the catheter shaft and exit the catheter as a gas can be used. The degree of protection, as with all of the protective devices, can be varied through temperature of the protective media, or through the flow rate of the protective media.

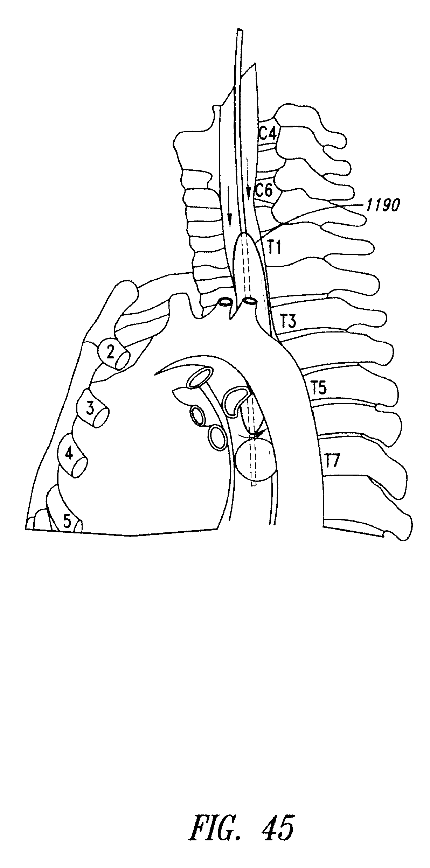

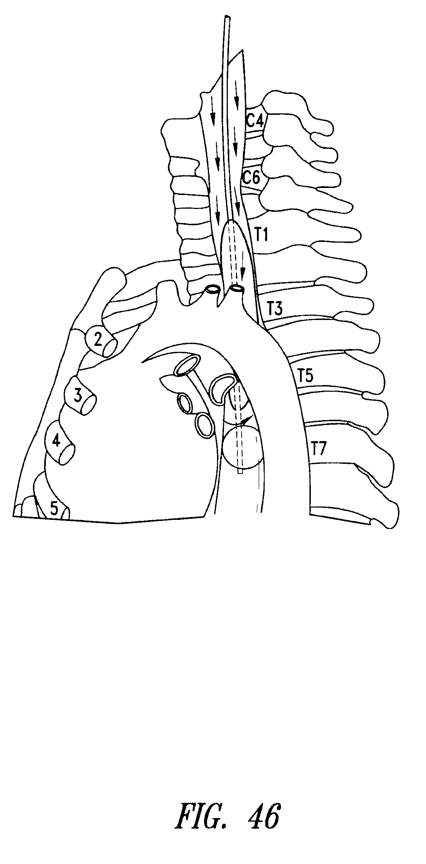

Figures 45 and 46 show further alternative embodiments of a distal occlusion protective device wherein a conductive element is incorporated into the system. This enables bipolar trachea-to-esophagus treatment. The conductive element may be attached to the same shaft as the occlusion device, such that the entire system is introduced at once. Alternatively, the conductive elements could be a separate device which is placed alongside of or over top of the occlusion device, and which is insertable and operable separately from the occlusion device. The conductive element may be constructed similarly to any of the esophageal devices described herein, such as a basket electrode array 1190 having a plurality of electrode bands.

Figure 46 shows the embodiment of Figure 45 with protectant circulating around and through the elements of the conductive system. As with prior embodiments, the protectant can be introduced through the nose or mouth, through the central shafts of the devices, or through the conductive elements themselves. Introduction through the conductive elements themselves provides the added bonus of cooling those elements and preventing tissue charring during thermal ablation. Charring on the electrodes greatly increases the impedance of the system and decreases or eliminates the effectiveness of the ablation.

Microwave energy has found increasing uses over the past few years and may be used in embodiments of the present invention as an alternative energy system. Principally, microwave energy is delivered through an antenna. There are a number of different types of microwave antennae. With suitable modifications based on the teachings of the instant disclosure, some the basic microwave antenna forms may be incorporated into devices designed for modulating or modifying pulmonary nerves as described herein. Of particular use for the application of catheter based microwave energy within the trachea-to-esophagus region is the family of antenna based upon coaxial wire leads. There are a number of different designs using the coaxial leads. These types of antennae come in many different configurations-monopole, dipole, slot, capped, choked, cap-choke, sleeved, etc. Each antenna variation is intended to either shift the field orientation, to improve the efficiency of energy delivery, or both. Wave guide antennae are another known antennae for microwave applications. Wave guide antennae are typically a metal jacketed dielectric, which is fed with a coaxial cable inserted into a side hole in the device.

Examples of basic configurations for microwave antennae that may be modified and configured for use with embodiments of the present invention by persons of ordinary skill in the art may be found in the following publications: Microwave Catheter Design; Robert D. Nevels, G. Dickey Arndt, George W. Raffoul, James R. Carl, and Antonio Pacifico. IEEE TRANSACTIONS ON BIOMEDICAL ENGINEERING, VOL. 45, NO. 7, JULY 1998*,* and A Review of Coaxial-Based Interstitial Antennas for Hepatic Microwave Ablation, John M. Bertram, Deshan Yang, Mark C. Converse, John G. Webster, & David M. Mahvi; Critical Reviews™ in Biomedical Engineering, 34(3):187-213 (2006). Both of these publications are incorporated by reference in their entirety. Among the reasons that such antennae designs cannot be directly incorporated into embodiments of the present invention is their unsuitability for pulmonary devices without modification. Among the parameters that must be reconfigured for deployment in the pulmonary tree according to embodiments of the present invention are the size, stiffness and general deliverability.

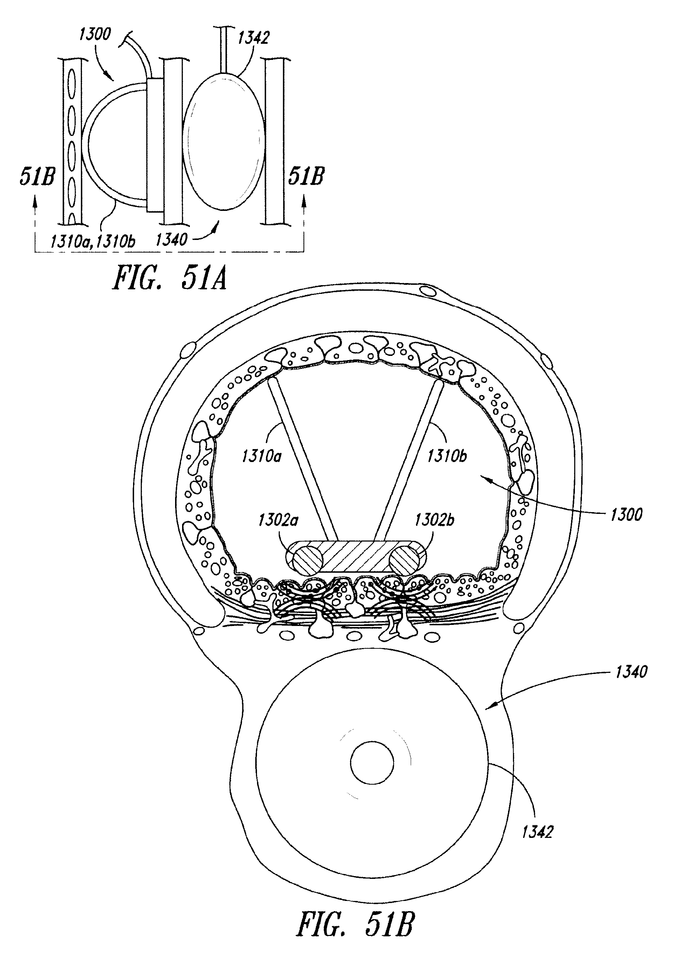

In pulmonary applications, the devices need to be introduced through or in conjunction with bronchoscopes, and manipulated down tortuous paths into the area of the lung to be treated. This necessitates the translation of conventional microwave antenna designs into application specific embodiments, such as the exemplary embodiments shown in Figures 51A-54C. One generally common aspect for these pulmonary devices is flexibility, although in some cases a flexible body member is coupled to more rigid segments in the area of the slots, caps, and chokes. Other aspects that must be specially considered for pulmonary applications are features to provide tissue coupling, maintain positioning relative to the target tissue, cool non-target tissue, etc.

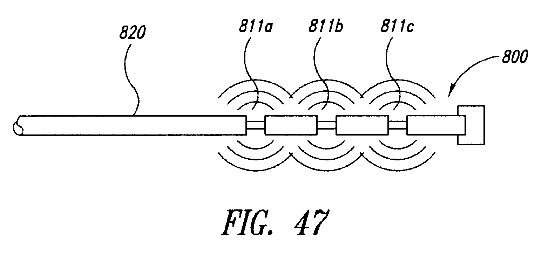

In one exemplary embodiment, an antenna that may be particularly effective in pulmonary applications for microwave energy delivery is a multi-slot coaxial design such as shown Figure 47. In this embodiment, in addition to a slot near the tip, a plurality of additional slots are positioned at appropriate distances down the shaft of the device, with the distances being determined by wavelengths of operation, desired specific absorption rate (SAR) pattern, etc. Specific absorption rate, or SAR, is a proxy for energy delivery to the tissue, or heating profiles of the tissue, and are the standard way in which antenna designs are evaluated and optimized.

In many microwave antenna applications in medicine, the desire is to provide the largest effective area of energy delivery to tissue, with the area of treatment extending from the edge of the antenna or applicator to the periphery of the largest area possible. However, in the case of pulmonary nerve modulation, protection of the structures immediately adjacent the applicator is preferred. Ideally, the energy would pass through a cooling or protective layer, heat tissue within a few millimeters of a zone, and then drop off in intensity in order not to harm critical non-target tissues such as the esophagus and alveoli. This is not possible in any of the antenna designs shown from the prior art. Embodiments to achieve these ends are shown and described in detail below in Figures 58A-53.

In microwave terms, the more "lossy" a material is, the higher the propensity of that material to heat up. Lossy materials in the body are typically those with higher water content. This is due to the fact that microwaves heat dipole molecules by causing rotation of the dipole molecule under the oscillations of the wave. Water is a strong dipole molecule, and heats extremely well under microwaves.

The tables below show various electrical properties of different tissues at two different commonly used medical microwave frequencies, 915 MHz and 2.45 GHz. One aspect that is apparent from these data is that as microwave frequency increases, depth of penetration decreases-so lesions are made more shallowly. For this reason, it is likely that the preferred frequency for pulmonary nerve modulation will be 2.45 GHz or higher. At least one microwave system designed by Microsulis Inc. operates at frequencies in the 9 GHz region. The frequency can be selected so that the microwave energy penetrates the tissue to a depth of the target tissue with an intensity sufficient to alter the target tissue while having insufficient intensity in non-target tissue, such as non-target tissue beyond the nerve tissue.

Frequency alone does not determine depth and character of penetration and tissue modification. It is known that standing waves can develop in microwave fields, and specific systems must be modeled with FEA systems to determine the most likely resultant SAR patterns within a given tissue system.

For example, the permittivities of most of the tissue types listed below are roughly in a similar range, indicating that they will heat similarly. However, there are a couple of exceptions-the esophagus may heat more easily than other tissues, and so may require the protection that has been discussed throughout this disclosure. Also, it is of particular interest that the permittivity of the lung differs significantly as between the inflated and deflated states.

| **Tissue name** | **Frequency [Hz]** | **Conductivity [S/m]** | **Relative permittivity** | **Loss tangent** | **Wavelength [m]** | **Penetration depth [m]** |
|---|---|---|---|---|---|---|
| Cartilage | 915000000 | 0.7892 | 42.6 | 0.36394 | 0.049412 | 0.044603 |
| Cartilage | 2450000000 | 1.7559 | 38.77 | 0.33228 | 0.019393 | 0.019077 |
| | | | | | | |

| **Tissue name** | **Frequency [Hz]** | **Conductivity [S/m]** | **Relative permittivity** | **Loss tangent** | **Wavelength [m]** | **Penetration depth [m]** |
|---|---|---|---|---|---|---|
| LungInflated | 915000000 | 0.45926 | 21.972 | 0.41063 | 0.068523 | 0.05527 |
| LungInflated | 2450000000 | 0.80416 | 20.477 | 0.28813 | 0.02677 | 0.030175 |
| | | | | | | |

| **Tissue name** | **Frequency [Hz]** | **Conductivity [S/m]** | **Relative permittivity** | **Loss tangent** | **Wavelength [m]** | **Penetration depth [m]** |
|---|---|---|---|---|---|---|
| Mucous Membrane | 915000000 | 0.85015 | 46.021 | 0.36291 | 0.047545 | 0.043032 |
| Mucous Membrane | 2450000000 | 1.5919 | 42.853 | 0.27255 | 0.018524 | 0.022029 |
| | | | | | | |

| **Tissue name** | **Frequency [Hz]** | **Conductivity [S/m]** | **Relative permittivity** | **Loss tangent** | **Wavelength [m]** | **Penetration depth [m]** |
|---|---|---|---|---|---|---|
| Nerve | 915000000 | 0.57759 | 32.486 | 0.34929 | 0.056652 | 0.053157 |
| Nerve | 2450000000 | 1.0886 | 30.145 | 0.26494 | 0.022097 | 0.027006 |
| | | | | | | |

| **Tissue name** | **Frequency [Hz]** | **Conductivity [S/m]** | **Relative permittivity** | **Loss tangent** | **Wavelength [m]** | **Penetration depth [m]** |
|---|---|---|---|---|---|---|
| Oesophagus | 915000000 | 1.1932 | 65.02 | 0.36053 | 0.040007 | 0.036435 |
| Oesophagus | 2450000000 | 2.2105 | 62.158 | 0.26092 | 0.015392 | 0.019092 |
| | | | | | | |

| **Tissue name** | **Frequency [Hz]** | **Conductivity [S/m]** | **Relative permittivity** | **Loss tangent** | **Wavelength [m]** | **Penetration depth [m]** |
|---|---|---|---|---|---|---|
| Trachea | 915000000 | 0.7757 | 41.971 | 0.36308 | 0.049785 | 0.04504 |
| Trachea | 2450000000 | 1.4488 | 39.733 | 0.26753 | 0.019244 | 0.023299 |

The significance of the change in permittivity of the lung upon inspiration may be of particular interest in a case where the nerve modulation is to be conducted at or below the area of the carina. Once into the right and left bronchi, tissue surrounding the bronchi is increasingly alveolar tissues-highly compliant, and highly air-filled. It is this air that is likely responsible for the decrease in permittivity of filled lungs. The permittivity of air is 1-it does not heat in any significant way in the presence of microwaves.

One significance of this fact for the subject applications is that it may be beneficial to tie the application of microwave energy to the inspiration cycle of respiration, when the lung is filled with air. Alternatively, the method of treatment could include a breath-hold or a ventilatory hold induced by a ventilator machine in order to ensure air-filled tissue surrounding the bronchi supporting the nerves to be treated.

Microwaves encountering materials of different permittivities can also act in unusual ways. Reflections can be created at tissue interfaces or air/tissue interfaces which can be exploited to focus ablative or modulatory energy more specifically on the tissues to be treated.

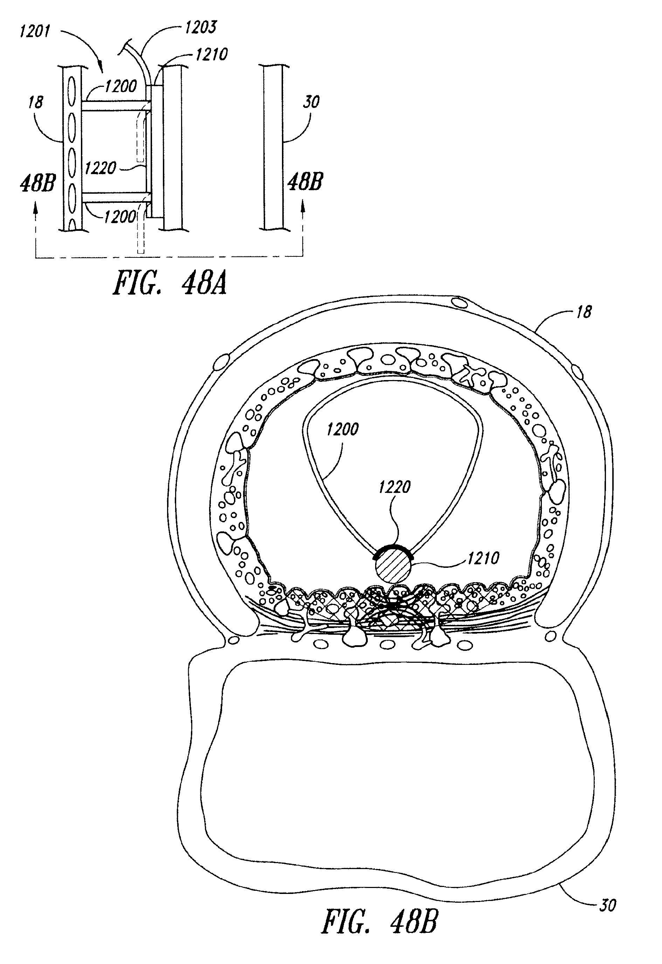

Figures 48A and 48B show embodiments of microwave systems. The pulmonary treatment apparatus 1201 includes an elongate member 1203 and a microwave antenna 1210 coupled the elongate member 1203. The microwave antenna 1210 is positioned at treatment location proximate a target site in or proximate to the airway. The microwave antenna 1210 delivers microwave energy so as to alter nerve tissue in a manner which disrupts transmission of nerve signals while non-target tissue disposed between the microwave antenna 1210 and the nerve tissue is not permanently injured.

Expandable or deployable supporting elements 1200 are provided which ensure solid coupling of the antenna 1210 to the tissue. The supporting elements 1200 are movable from a contracted position (shown in dashed line in Figure 48A) to the illustrated expanded position. These elements can be wires, balloons, fingers, or the like. The supporting elements 1200 of Figures 48A and 48B are illustrated as a pair of elongate members 1210 configured to bow outwardly to engage the anterior wall of the airway. Optionally, shielding 1220 can be provided on one or more sides of the device to further focus the microwave energy into the tissue and/or to protect non-target tissues. Shielding 1220 can be metallic foil, metal loaded polymer, metallic mesh with mesh opening of an appropriate fraction of the wavelength in use so as to block transmission of the waves therethrough, or any known microwave shielding material. Not shown in Figures 48A and 48B is an optional esophageal protection system. This system can take any of the forms previously disclosed.

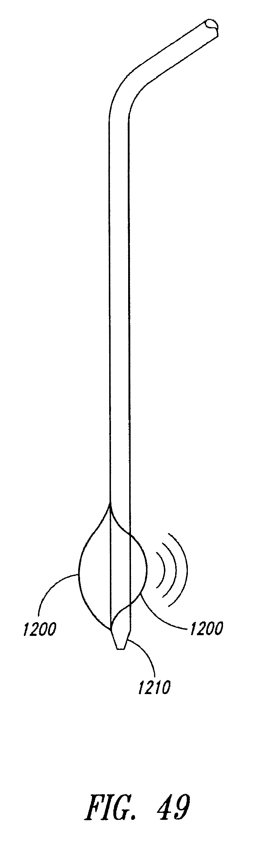

Also noted in Figure 48B is a tissue plane discontinuity between the esophagus and the trachea. If therapy is to be delivered at this level rather than down in the bronchi below the carina, it is possible that the differences in tissue properties will cause reflection, or that the air in the esophagus, or the protectant system in the esophagus, will cause reflection of the microwaves. Reflection of waves can result in cancellation, summation, or additive power of the waves, or it can result in standing waves. Cancellation would tend to negate clinical effectiveness and must be avoided in the system design. Summation or standing waves can be beneficial, and may be designed into the system to provide higher effective energy levels at the target tissue than the level of energy delivered by the system alone. Figure 49 shows emitted waves.

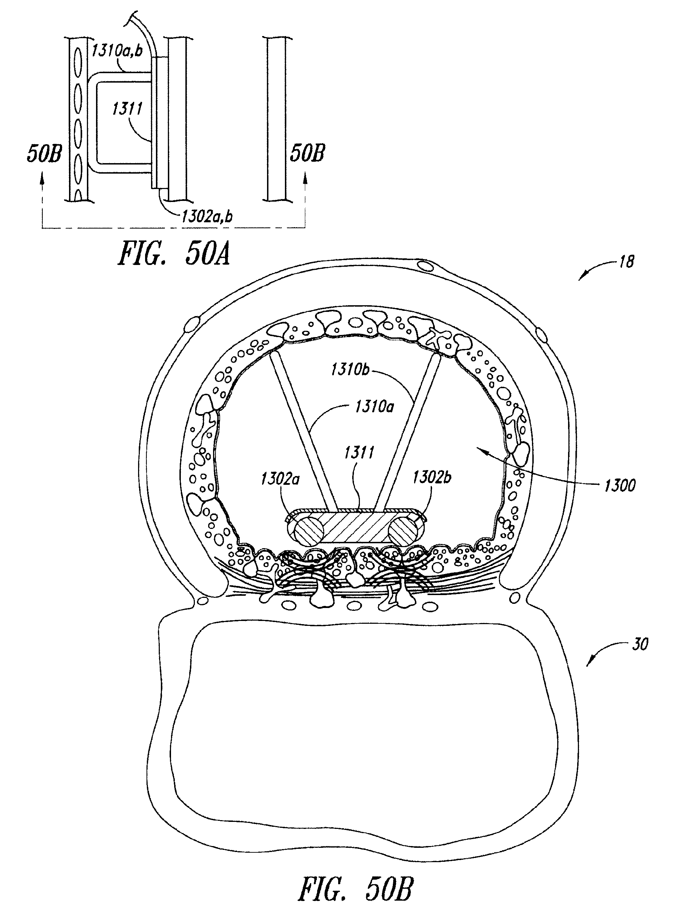

Figures 50A and 50B illustrate a further alternative embodiment of the present invention including a dual antenna system 1300 built on the same basic principles as described in connection with the embodiments disclosed above. A shield of dielectric material 1311 can be mechanically coupled to antennae 1302a, 1302b. Support structures 1310a, 1310b can help hold the antennae 1302a, 1302b proximate or against the posterior tissue of the trachea 18. The support structures 1310a, 1310b can be elongate arms, ribs, inflatable members, or the like. The antennae 1302a, 1302b can cooperate to form standing waves in a desired configuration. Optionally, a protective device can be used to protect tissue of the esophagus 30 or any other bridging tissue proximate or adjacent to the trachea 18 and/or the esophagus 30. Note that while two antennae 1302a, 1302b are shown in this embodiment, any number of antennae can be included without departing from the teachings of the present invention. The antennae may be bound edge-to-edge down the longitudinal axis of the catheters, or they may be separated by an appropriate dielectric material. The antennae 1302a, 1302b can be fired simultaneously, in sequence, alternating or in various other patterns to modify or optimize the SAR distribution to the desired tissue.

Figures 51A and 51B illustrates yet another embodiment of the microwave therapy system wherein an esophageal device 1340 is included to modify or optimize the microwave SAR pattern in the target tissues. The esophageal device 1340 shown here is a reflector 1342. The reflector 1342 includes a balloon filled with inflation media chosen for specific dielectric properties that alter the SAR pattern in the tissue therebetween. This alteration of the SAR pattern acts to reflect microwave energy back toward the delivering device in order to sum the wave energies or to create a standing wave within the tissue. It could alternatively be used to provide negation of oncoming waves, or it could be used to absorb microwave energy in order to draw the energy deeper into the tissue and then negate it at the device. The balloon 1342 can be connected to the media source. The media source can be the media delivery system 246 discussed in connection with Figure 10.

While a balloon is shown in the embodiment of Figures 51A and 51B, persons of ordinary skill in the art will recognize based on the teachings herein that other devices may be used whose materials, design, use or any combination of these factors provide an alteration to the SAR pattern created by the matched microwave antenna when used in concert with that antenna. Other types of reflectors may include, without limitation, one or more balloons, plates, or the like. Also note that although the microwave embodiment in Figure 51B is a dual antenna design, any contemplated antenna design could be substituted in this system. Although the use of the dielectric SAR altering device is described with that device in the esophagus and the microwave antenna in the trachea or bronchi, the devices could be placed in the reverse arrangement as desired.

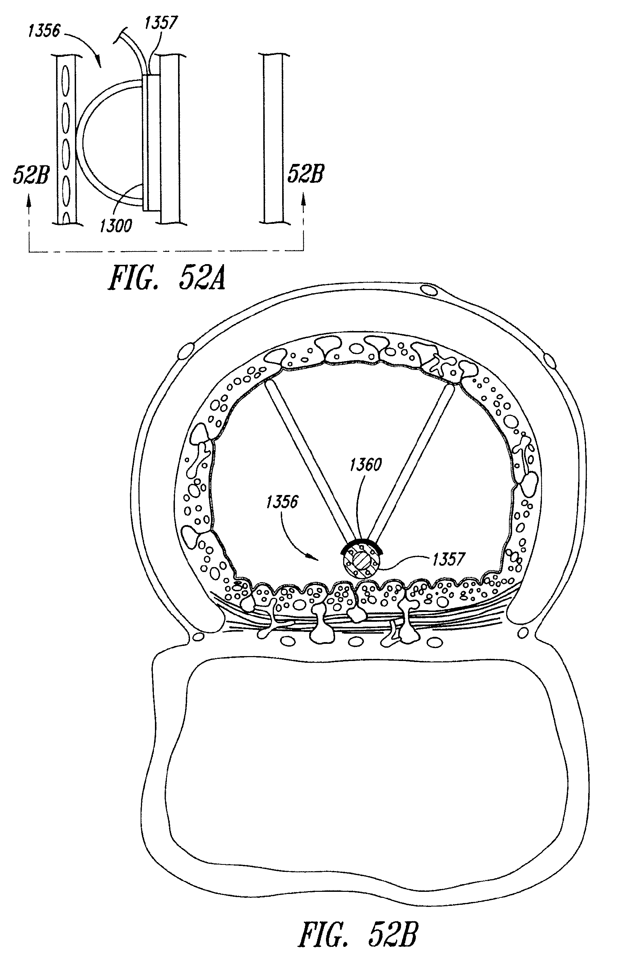

In another alternative embodiment, as shown in Figures 52A and 52B, microwave systems such as those shown in Figures 48A-50B can be outfitted with a cooling device in the form of an outer jacket 1356 through which media can be introduced or circulated. A plurality of channels can extend through a main body 1357. This media can serve as a cooling agent via temperature control or flow control of the media, or a combination of the two. The media may be chosen for dielectric properties which provide better coupling between the antenna and the tissue. The outer jacket 1356 may also include shielding 1360.

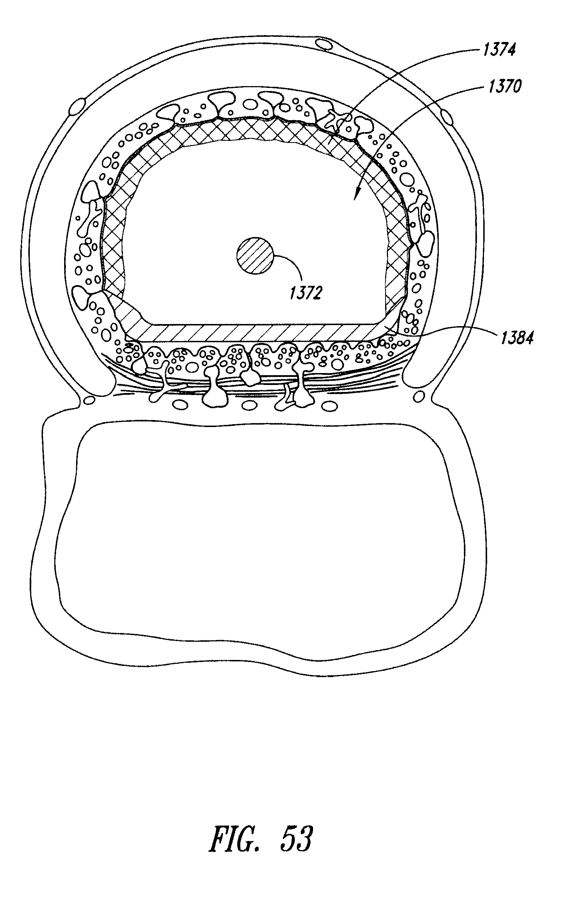

Figure 53 illustrates another alternative embodiment including cooling or coupling media in a chamber 1370 to surround an antenna 1372. In this embodiment, a cooling device includes an outer member 1374 (illustrated as a balloon wall) of the device that surrounds the antenna 1372 and couples with substantially the entire circumference of the trachea or bronchi. The outer member 1374 cools at least a portion of the non-target tissue while the microwave antenna 1372 delivers the microwave energy. Thus, the wall of the outer member 1374 is positioned between the microwave antenna 1372 and the wall of the airway. The microwave energy can pass through the outer member 1374 and penetrates the airway wall to a depth of the target tissue with an intensity sufficient to alter the tissue. Optionally, shielding 1384 may be built into the device to block transmission on a portion of the circumference to protect that portion from treatment as explained below. In other embodiments, the shielding 1384 can absorb the microwave energy. This shielding could be used to protect the esophagus, for example.

Alternatively, the embodiment of Figure 53 could be used in a method of treatment for which multiple embodiments throughout this disclosure may be used. To use the device in Figure 53 with shielding, as an example of a method of treatment according to one embodiment of the present invention, the device would be introduced to a point along the desired treatment zone of the airway. Energy is delivered to a portion of the circumference of the airway which is less than 360 degrees. The device is then advanced or withdrawn so that the next treatment zone either barely overlaps, or allows a small gap between it and the last treatment zone. Additionally, the device is rotated such that there is either a slight overlap or a slight gap circumferentially as compared to the prior treatment site. By repositioning the device both longitudinally and circumferentially, in two or more treatments the entire circumference of the airway could be treated, but not contiguously. In effect, there is a spiral treatment area created, with the proximal and distal ends of the spiral approximately matched or overlapped when compared circumferentially, but which are separated longitudinally.

This spiral or displaced treatment pattern would allow modulation or ablation of the nerves surrounding an airway, without risking the creation of a circumferential zone of treatment which could cause unwanted wall effects such as hyperproliferation of cells during healing, scarring, stenosis or the like.

Another embodiment that would provide the spiral treatment pattern desired would be a multi-slotted antennae 800 as was described in connection with Figure 47. In addition to the extra slots 811 a, 811b, 811c, and hence extra treatment zones spaced longitudinally down a catheter shaft 820, the spiral design may have partial-circumferential shielding (device not shown). Figure 47 also shows a SAR pattern. The position of the shielding would vary by position along the length of the catheter. For example, a multi-slot design providing four treatment areas longitudinally could be shielded from 12-3 o'clock in longitudinal segment 1, 3-6 o'clock in longitudinal segment 2, 6-9 o'clock in longitudinal segment 3, and 9-12 o'clock in the final longitudinal segment. Thus, it is possible with a single energy application that the entire spiral-shaped energy deposition is made.

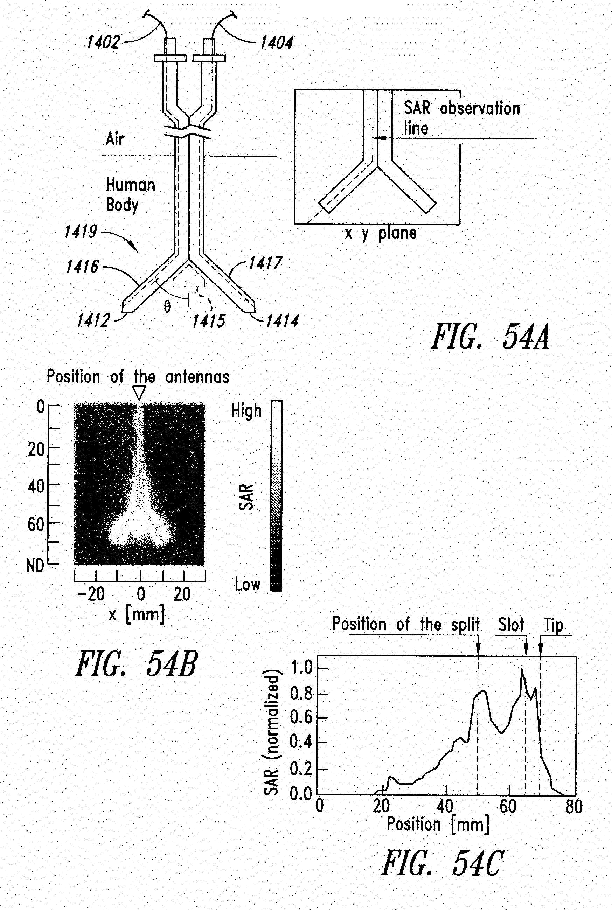

Figure 54A shows a further alternative embodiment for a microwave antenna intended to create as large an area of ablation as possible for a given insertion into the body. While the bifurcated shape of the antenna in Figures 54A and 54B are interesting for lung applications, several issues make it infeasible to use for this application as shown. Given the rigidity of coaxial cable used in antennae such as that shown in Figures 54A and 54B, it may require specific device designs to achieve delivery of such a split tip design to the lung. Pull wires 1402, 1404 attached to the tips 1412, 1414 could be added to deflect the tips 1412, 1414 actively as desired. Memory materials could be built into the shafts of the split segments to bias them outward, and an outer sheath provided to hold them together for delivery. Given the stiffness of some coaxial wire, a wedge-shaped element 1415 (illustrated in dashed line) can be added between legs 1416, 1417 of the split tip 1419, which when retracted via pull wires 1402, 1404 or the like, the legs 1416, 1417 are forced outward and apart.

Additionally, the actual SAR pattern of the antenna shown is not applicable in the pulmonary indication. Note the "tail" of the SAR pattern which extends downward between the legs 1416, 1417 of the device shown in Figure 54B. This energy deposition would occur in non-target tissues if used in the lung as designed-most probably, the heart.

Significant redesign of the system shown can be performed for pulmonary applications. One embodiment which would provide both the deployment of the legs 1416, 1417 of the split antenna device as well as creating a more desirable SAR pattern would be to provide a sliding wedge element 1415to separate the legs 1416, 1417, but the material of which is a dielectric material selected to modify the SAR pattern to more closely follow the legs 1416, 1417 of the antennae, without the unwanted "tail" energy directed towards the heart.

High intensity ultrasound (HIFU) is another energy modality that can be employed to provide pulmonary nerve modulation. In HIFU, ultrasound transducers are shaped, or in some cases multiple transducers are electronically beam-formed to a focal point. At the focal point, relatively low intensity ultrasound departs the ultrasound transducer(s) and converges at the focal point designed into the transducer to create a zone of heating and tissue ablation.

A jacketed esophageal HIFU device appears in "US2007/0027445 Method and Apparatus for Noninvasively Treating Patent Foramen Ovale Using High Intensity Focused Ultrasound" by the present inventors, which disclosure is incorporated herein by reference in its entirety. This device is a transesophageal HIFU device coupled to the target tissue with a cooling jacket or balloon surrounding the HIFU elements. This device was initially designed to treat atrial fibrillation by targeting the posterior wall of the heart from the esophagus. However, the same or similar device could be adapted for use in the currently disclosed methods for pulmonary treatment.

HIFU devices are to be used to fire energy into structures which are either tissue or fluid. While reflections of ultrasound may occur at transitions between different tissue types, all of the structures are essentially acoustic conductors. Air, however, will not conduct ultrasound. So in the unique case of pulmonary neuromodulation, HIFU fired from either the airway or esophagus will encounter an air barrier just beyond the target tissue, and become attenuated, or reflect to form a standing wave within the target tissues.

In order to maximize the desired effects, a device similar to the one shown in Figures 54A and 54B may be employed wherein the microwave device would be replaced with a HIFU transducer. For HIFU, the dielectric properties of the fluid in the balloon 1342 would be replaced by specific acoustic properties, to either enhance the absorption or reflection of the applied acoustic power.

Different types of modifications can be made to treat tissue with different types of energy. Energy can be used to damage target regions. As used herein, the term "energy" is broadly construed to include, without limitation, thermal energy, cryogenic energy (*e.g.,* cooling energy), electrical energy, acoustic energy (*e.g.,* ultrasonic energy), HIFU energy, RF energy, pulsed high voltage energy, mechanical energy, ionizing radiation, optical energy (*e.g.,* light energy), microwave energy, and combinations thereof, as well as other types of energy suitable for treating tissue. In some embodiments, the catheter system, devices, or apparatus disclosed herein delivers energy and one or more substances (*e.g.,* radioactive seeds, radioactive materials, etc.), treatment agents, and the like. For example, the assembly 208 of Figures 5 and 6 can include one or more ports through which a treatment agent is delivered. Exemplary non-limiting treatment agents include, without limitation, one or more antibiotics, antiinflammatory agents, pharmaceutically active substances, bronchoconstrictors, bronchodilators (*e.g.,* beta-adrenergic agonists, anticholinergics, etc.), nerve blocking drugs, photoreactive agents, or combinations thereof. For example, long acting or short acting nerve blocking drugs (*e.g.,* anticholinergics) can be delivered to the nerve tissue to temporarily or permanently attenuate signal transmission. Substances can also be delivered to chemically damage the nerve tissue. The electrodes, antenna, or other energy emitting components can be replaced with other types of components based on the desired type of energy to be used for treatment.

The various embodiments described above can be combined to provide further embodiments. These and other changes can be made to the embodiments in light of the above-detailed description. The embodiments, features, systems, devices, materials, methods and techniques described herein may, in some embodiments, be similar to any one or more of the embodiments, features, systems, devices, materials, methods and techniques described in U.S. Provisional Patent Application No. 61/321,346 filed April 6, 2010; U.S. Application No. 12/463,304 filed on May 8, 2009; U.S. Application No. 12/913,702 filed on October 27, 2010; PCT Application No. PCT/US2010/056424 filed November 11, 2010; U.S. Application No. 12/944,666 filed November 11, 2010; and PCT Patent Application No. PCT/US2010/56425 filed November 11, 2010. Each of these applications is incorporated herein by reference in its entirety. In addition, the embodiments, features, systems, devices, materials, methods and techniques described herein may, in certain embodiments, be applied to or used in connection with any one or more of the embodiments, features, systems, devices, materials, methods and techniques disclosed in the above-mentioned U.S. Application No. 12/463,304 filed on May 8, 2009; U.S. Application No. 12/913,702 filed on October 27, 2010; PCT Application No. PCT/US2010/056424 filed November 11, 2010; U.S. Application No. 12/944,666 filed November 11, 2010; and PCT Patent Application No. PCT/US2010/56425 filed November 11, 2010. For example, the apparatuses of disclosed in U.S. Application No. 12/463,304 may incorporate the electrodes or other features, such as the protection devices, disclosed herein. All of the U.S. patents, U.S. patent application publications, U.S. patent application, foreign patents, foreign patent application and non-patent publications referred to in this specification and/or listed in the Application Data Sheetare incorporated herein by reference, in their entirety. Aspects of the embodiments can be modified, if necessary to employ concepts of the various patents, application and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

Further preferred embodiments of the invention are as follows:
1. A system for pulmonary treatment, comprising: a pulmonary treatment device having an energy delivery element positionable through at least a portion of a trachea into an airway and configured to deliver energy to a wall of the airway to alter nerve tissue located in or proximate to the wall of the airway; and
   a protection device having a protection member positionable in an esophagus while the pulmonary treatment device is positioned in the airway, the protection member being configured to absorb heat from a wall of the esophagus to inhibit damage to esophageal tissue.
2. The system of embodiment 1 wherein the pulmonary treatment device is configured to deliver a sufficient amount of energy to the wall of the airway to heat and damage the nerve tissue, wherein the protection device is configured to absorb a sufficient amount of heat from the wall of the esophagus to inhibit damage to esophageal tissue while the nerve tissue is damaged.
3. The system of embodiment 1, further comprising a media delivery system fluidically coupled to the pulmonary treatment device and the protection device, the media delivery system being configured to deliver cooling media through the pulmonary treatment device to cool the energy delivery element and configured to deliver cooling media through the protection device to cool the protection member.
4. The system of embodiment 1 wherein the airway treatment device comprises a first elongate member configured for insertion through the airway, and the at least one energy delivery element is disposed on the first elongate member in a position corresponding to the anatomical location of at least one nerve in or proximate to the airway wall when said first elongate member is positioned therein.
5. The system of embodiment 4 wherein the protection device comprises a second elongate member configured for insertion in the esophagus, the protection member being disposed on the second elongate member in a position generally aligned with the position of the at least one energy delivery element when the airway treatment device is positioned in the airway and the second elongate member is positioned in the esophagus.
6. The system of embodiment 1, further comprising cooling means associated with said at least one energy delivery element to limit tissue damage adjacent select denervation sites.
7. The system of embodiment 1 wherein said protection member comprises an expandable member configured for insertion into the esophagus.
8. The system of embodiment 7 wherein said expandable member comprises an inflatable balloon configured to circulate a cooling medium therein.
9. The system of embodiment 7 wherein said expandable member comprises a balloon configured to occlude the esophagus and said cooling means further comprises means for circulating a cooling fluid within the occluded esophagus.
10. The system of embodiment 1 wherein said airway treatment device comprises an inflatable balloon.
11. The system of embodiment 10 wherein said inflatable balloon is configured for circulation of cooling fluid therein.
12. The system of embodiment 1 wherein the active electrode is balloonlessly expandable from a contracted configuration to an expanded configuration.
13. The apparatus of embodiment 12 wherein the active electrode is configured to fit between adjacent cartilage rings of the airway in the expanded configuration.
14. The system of embodiment 1 wherein said airway treatment device comprises a helical or ring-shaped member that includes the energy delivery element.
15. The system of embodiment 1 wherein said pulmonary treatment device comprises an energy delivery device configured to be positioned in the airway to locate the energy delivery element into an intercartilaginous region.
16. The system of embodiment 1 wherein said at least one energy delivery element comprises an RF electrode.
17. The system of embodiment 16 wherein said energy delivery element further comprises a return electrode, said electrodes being configured for bipolar energy delivery.
18. The system of embodiment 1 wherein said at least one energy delivery element comprises a microwave antenna.
19. The system of embodiment 1 wherein said protection device comprises at least one electrode configured to be operatively coupled with the energy delivery element of the airway treatment device.
20. A method for pulmonary treatment, comprising: positioning at least one energy delivery element through at least a portion of the trachea into an airway adjacent a treatment site to be treated;
   delivering energy from said at least one element to a portion of the circumference of the airway at said treatment site; and
   cooling tissues of an esophagus to prevent damage of the tissues of the esophagus while the energy is delivered.
21. The method of embodiment 20 wherein said cooling comprises delivering a cooling medium into the esophagus.
22. The method of embodiment 20 wherein said portion of the circumference of the trachea of the subject is less than 360 degrees around the trachea.
23. The method of embodiment 20, further comprising:
   repositioning the at least one energy delivery element in close proximity to the previous position;
   and
   delivering energy in an adjacent treatment site wherein said adjacent site barely overlaps or allows a small gap with the previous treatment site.
24. The method of embodiment 20, further comprising rotating the at least one energy delivery element to provide a slight overlap or a slight gap circumferentially with respect to the previous treatment site.
25. The method of embodiment 20 wherein said delivering comprises delivering RF energy.
26. The method of embodiment 20 wherein said delivering further comprises bipolar delivery of RF energy.
27. The method of embodiment 20 wherein said delivering comprises delivering microwave energy.
28. The method of embodiment 20, further comprising positioning at least one esophageal energy delivery element in the esophagus and delivering or receiving energy to or from said esophageally positioned element.
29. The method of embodiment 20 wherein the energy delivery element comprises a first electrode, the first electrode being positioned within a first space between a first pair of adjacent cartilage rings of the airway.
30. The method of embodiment 29, further comprising placing a second electrode in a second space between a second pair of adjacent cartilage rings of the airway.
31. The method of embodiment 30, further comprising delivering energy between the first and second electrodes to alter target tissue in a wall of the airway longitudinally displaced from the first and second spaces.
32. A pulmonary treatment apparatus comprising:
   an elongate member insertable through at least a portion of a trachea into an airway; and
   a microwave antenna coupled to the elongate member and positionable in the airway at a treatment location proximate nerve tissue in a wall thereof, the microwave antenna being configured to deliver microwave energy so as to alter the nerve tissue in a manner which disrupts transmission of nerve signals therein while non-target tissue disposed between the microwave antenna and the nerve tissue is not permanently injured.
33. The pulmonary treatment apparatus of embodiment 32, further comprising a cooling device coupled to the elongate member configured to cool at least a portion of the non-target tissue while the microwave antenna delivers the microwave energy.
34. The pulmonary treatment apparatus of embodiment 32 wherein the cooling device comprises a jacket surrounding the microwave antenna.
35. The pulmonary treatment apparatus of embodiment 32 wherein the cooling device comprises an inflatable balloon having a chamber, the microwave antenna being disposed in the chamber.
36. The pulmonary treatment apparatus of embodiment 32 wherein at least a portion of the cooling device is disposed between the antenna and the wall of the airway when the antenna is positioned at the treatment location.
37. The pulmonary treatment apparatus of embodiment 32 wherein the microwave antenna is configured to heat the nerve tissue to a lethal nerve temperature while the non-target tissue is maintained at a temperature below a lethal tissue temperature.
38. The pulmonary treatment apparatus of embodiment 32 wherein the microwave antenna is configured to alter the nerve tissue while non-target tissue disposed beyond the nerve tissue is not permanently injured.
39. The pulmonary treatment apparatus of embodiment 32 wherein the microwave antenna operates at a frequency selected so that the microwave energy penetrates the airway wall to a depth of the nerve tissue with an intensity sufficient to alter the nerve tissue while having insufficient intensity in non-target tissue beyond the nerve tissue to cause permanent injury.
40. The pulmonary treatment apparatus of embodiment 32, further comprising at least one supporting element movable from a contracted position to an expanded position and configured to engage the wall of the airway to position the microwave antenna.
41. The pulmonary treatment apparatus of embodiment 32 wherein the antenna comprises a plurality of longitudinally spaced slots.
42. The pulmonary treatment apparatus of embodiment 32 wherein the microwave antenna comprises a dual antenna system configured to produce a standing wave at a location corresponding to the nerve tissue when the microwave antenna is at the treatment location.
43. The pulmonary treatment apparatus of embodiment 32, further comprising a shield positioned and configured to block or attenuate the microwave energy emitted in a selected direction from the microwave antenna.
44. The pulmonary treatment apparatus of embodiment 32, further comprising a reflector positionable at a location spaced apart from the microwave antenna and configured to reflect the microwave energy delivered therefrom.
45. The pulmonary treatment apparatus of embodiment 44 wherein the reflector is positionable in an esophagus.
46. The pulmonary treatment apparatus of embodiment 45 wherein the reflector comprises an inflatable member connected to a source of inflation fluid.
47. The pulmonary treatment apparatus of embodiment 32, further comprising a protection member positionable in an esophagus.
48. The pulmonary treatment apparatus of embodiment 47 wherein the protection member comprises an expandable member configured to engage esophageal tissue and absorb heat therefrom.
49. The pulmonary treatment apparatus of embodiment 47 wherein the protection member is configured to absorb microwave energy delivered from the microwave antenna.
50. The pulmonary treatment apparatus of embodiment 32 wherein the microwave antenna is positionable through a bronchoscope to the treatment location.
51. A method of pulmonary treatment comprising:
   positioning an elongate member through at least a portion of the trachea into an airway, the elongate member having a treatment element and an sensor coupled thereto;
   sensing a first tissue characteristic using the sensor with the treatment element at a first airway location;
   comparing the first tissue characteristic to a reference value to evaluate the location of the treatment element in the airway; and
   activating the treatment element to treat the airway.
52. The method of embodiment 51 wherein the sensor comprises at least one impedance sensor configured to sense tissue impedance.
53. The method of embodiment 51 wherein the treatment element is activated at a second airway location between two adjacent cartilage rings of the airway.
54. The method of embodiment 53 wherein the first airway location is at least partially surrounded by one of the cartilage rings, further comprising re-positioning the treatment element to the second airway location before activating the treatment element.
55. The method of embodiment 51 , further comprising estimating the location of a non-target structure relative to the treatment element based on the first impedance.
56. The method of embodiment 55, further comprising, after estimating the location of the non-target structure, repositioning the treatment element before actuation thereof so as to avoid injury to the non-target structures.
57. The method of embodiment 51 wherein the non-target structure comprises the esophagus.
58. The method of embodiment 51 , further comprising detecting previously treated tissue based on the first impedance.
59. The method of embodiment 58, further comprising, after detecting the previously treated tissue, repositioning the treatment element before actuation thereof to avoid re-treatment of the previously treated tissue.
60. The method of embodiment 51 wherein activating the treatment element comprises delivering energy therefrom to tissue in a wall of the airway.
61. The method of embodiment 60 wherein the energy alters nerve tissue in the wall of the airway to disrupt transmission of nerve signals therein.
62. The method of embodiment 61, further comprising protecting non-target tissue between the treatment element and the nerve tissue from permanent injury.
63. An apparatus for pulmonary treatment comprising: an elongate member insertable through a trachea into an airway;
   an active electrode coupled to the elongate member and configured to deliver energy to target tissue in a wall of the airway.
   a return electrode positionable in the airway or the esophagus and configured to receive the energy from the target tissue; and
   a protection member configured to cool non-target tissue proximate to the target tissue.
64. The apparatus of embodiment 63 wherein the return electrode is coupled to the elongate member so as to be positionable in the airway adjacent to the active electrode.
65. The apparatus of embodiment 63 wherein the active electrode is configured to engage a posterior aspect of the airway and the return electrode is configured to engage an anterior aspect of the airway.
66. The apparatus of embodiment 63 wherein the active electrode comprises a plurality of conductive bands movable from a contracted configuration to an expanded configuration.
67. The apparatus of embodiment 66 wherein the return electrode comprises at least one additional conductive band coupled to the conductive bands of the active electrode and electrically isolated therefrom, the return electrode being movable with the active electrode from a contracted configuration to an expanded configuration.
68. The apparatus of embodiment 63 wherein the active electrode is disposed on the exterior of an expandable balloon coupled to the elongate member.
69. The apparatus of embodiment 68 wherein the balloon is connected to source of coolant, the protection member comprising a cooled portion of the balloon.
70. The apparatus of embodiment 63 wherein the active electrode is expandable from a contracted configuration to an expanded configuration without the use of a balloon.
71. The apparatus of embodiment 70 wherein the active electrode is configured to fit between a first pair of adjacent cartilage rings of the airway in the expanded configuration.
72. The apparatus of embodiment 70 wherein the return electrode is configured to fit between a second pair of adjacent cartilage rings of the airway while the active electrode is positioned between the first pair of adjacent cartilage rings.
73. The apparatus of embodiment 63 wherein the active electrode is carried by an actuable device movable between a delivery configuration and a deployed configuration, the actuable device is coupled to the elongate member.
74. The apparatus of embodiment 73 wherein the actuable device defines a plurality of spaced apart rings configured to preferentially seat in one or more intercartilaginous spaces.
75. The apparatus of embodiment 74 wherein the actuable device has a helical or ring-shape in the deployed configuration.
76. The apparatus of embodiment 63 wherein the active electrode comprises a cooling channel fluidly coupled to a source of coolant.
77. The apparatus of embodiment 63 wherein the return electrode is coupled to an elongate shaft positionable in the esophagus.
78. The apparatus of embodiment 63 wherein the protection member is positionable in the esophagus while the elongate member and active electrode are positioned in the airway.
79. The apparatus of embodiment 78 wherein the protection member comprises an expandable member configured to engage esophageal tissue.
80. The apparatus of embodiment 79 wherein the return electrode is coupled to the expandable member.
81. A method of pulmonary treatment comprising:
   inserting an elongate member through at least a portion of a trachea such that an energy delivery element coupled to the elongate member is positioned at a treatment site in an airway;
   delivering energy at a first power level from an active portion of the energy delivery element to create a first lesion covering a first portion of a
   circumference of the airway;
   moving the energy delivery element; and
   delivering energy at a second power level from the active portion of the energy delivery element to create a second lesion covering a second portion of the circumference of the airway displaced from the first portion;
   wherein the first power level is substantially greater than the second power level.
82. The method of embodiment 81 wherein the second portion is circumferentially or axially displaced from the first portion relative to lumen of the airway.
83. The method of embodiment 81 wherein the first portion of the circumference is on an anterior aspect of the airway.
84. The method of embodiment 81 wherein the second portion is on a posterior aspect of the airway.
85. The method of embodiment 81 wherein the second power level is about 50% to about 80% of the first power level.
86. The method of embodiment 81 wherein the second power level is selected to avoid permanent injury to non-target tissue proximate to the treatment site.
87. The method of embodiment 86 wherein the first power level would permanently injure the non-target tissues if delivered to the second portion of the circumference.
88. The method of embodiment 87 wherein the non-target tissue comprises tissue of the esophagus.
89. The method of embodiment 81 wherein the airway comprises the left main bronchus.
90. The method of embodiment 81 wherein the first and second lesions are created at a depth in the airway wall without permanently injuring non-target tissue of the airway wall between the energy delivery element and the first and second lesions.
91. The method of embodiment 90, further comprising cooling tissue of the airway wall during the creation of the first and second lesions.
92. The method of embodiment 81 wherein at least one of the first and second lesions are created so as to alter nerve tissue in or proximate to the airway wall to disrupt signal transmission along the airway.
93. The method of embodiment 81 wherein moving the energy delivery element includes rotating the energy delivery element about a longitudinal axis of the airway.
94. A method of pulmonary treatment comprising:
   delivering a first amount of energy from an energy delivery device to a first portion of a wall of an airway; and
   delivering a second amount of energy from the energy delivery device to a second portion of the wall of the airway, the first portion of the wall and the second portion of the wall are spaced apart from one another or partially overlap one another, and the second amount of energy is different from the first amount of energy.
95. The method of embodiment 94, further comprising moving an elongate body and the energy delivery device coupled to the elongated body relative to the wall of the airway after delivering the first amount of energy and prior to delivering the second amount of energy.
96. The method of embodiment 94 wherein the first portion of the airway is located at an anterior region of the airway, and the first amount of energy is greater than the second amount of energy.
97. The method of embodiment 94 wherein delivering the first amount of energy comprises delivering energy at a first power level from an active portion of the energy delivery device, and delivering the second amount of energy comprises delivering energy at a second power level from the active portion of the energy delivery device.
98. The method of embodiment 94, further comprising ablating substantially all of the nerve trunks travelling along the wall of the airway using energy delivered from the energy delivery device.
99. A method of pulmonary treatment comprising:
   positioning an energy delivery element in an airway of a subject;
   non-inflatably moving the energy delivery element into engagement with a wall of the airway;
   delivering energy from the energy delivery element to the wall of the airway to alter target nerve tissue therein or proximate thereto; and
   introducing a cooling medium into the airway into direct contact with the wall to absorb heat from the wall while delivering the energy.
100. The method of embodiment 99 wherein the energy delivery element comprises a first electrode, the first electrode being positioned within a first space between a first pair of adjacent cartilage rings of the airway.
101. The method of embodiment 100, further comprising placing a second electrode in a second space between a second pair of adjacent cartilage rings of the airway.
102. The method of embodiment 100, further comprising delivering energy between the first and second electrodes to alter target tissue in a wall of the airway longitudinally displaced from the first and second spaces.
103. The method of embodiment 99, further comprising positioning a protection device in the esophagus to absorb heat from esophageal tissue while delivering the energy.
104. The method of embodiment 103, further comprising receiving energy with or delivering energy from a second electrode coupled to the protection device.
105. The method of embodiment 99 wherein a surface layer of tissue of the wall is protected from permanent injury while a lesion of permanently injured tissue is created at a depth below the surface layer.
106. The method of embodiment 105 wherein the surface layer is at least about 2 mm in thickness.
107. The method of embodiment 105 wherein the lesion contains the nerve tissue.
108. The method of embodiment 105 wherein the nerve tissue is altered sufficiently to reduce airway constriction in the subject.
109. The method of embodiment 99 wherein the cooling medium is a gas.
110. The method of embodiment 99 wherein the energy delivery element is coupled to an elongate member, and the cooling medium is introduced into the airway through a channel in the elongate member.
111. The method of embodiment 110 wherein the cooling medium flows through a channel in the energy delivery element to absorb heat therefrom.

## Claims

1. A pulmonary treatment apparatus comprising:
an elongate member insertable through at least a portion of a trachea into a first airway;
a microwave antenna coupled to the elongate member and positionable in the first airway at a treatment location proximate nerve tissue of a nerve trunk in or around an airway wall thereof; and
a cooling device having an outer member surrounding the microwave antenna and configured to contact the airway wall, the cooling device including a cooling chamber configured to contain coolant so as to cool the outer member,
wherein the microwave antenna is configured to deliver microwave energy through the outer member and the airway wall to a depth of the nerve tissue so as to alter the nerve tissue in a manner which reduces airway resistance in a second airway of higher generation than the first airway, and
wherein the cooling device is configured to absorb sufficient heat from non-target tissue disposed between the outer member and the nerve tissue during delivery of the microwave energy such that the non-target tissue is not permanently injured.

2. The pulmonary treatment apparatus of claim 1, wherein the cooling device comprises a jacket surrounding the microwave antenna.

3. The pulmonary treatment apparatus of claim 1 or 2, wherein the cooling device comprises an inflatable balloon having a chamber, the microwave antenna being disposed in the chamber.

4. The pulmonary treatment apparatus of any of the preceding claims, wherein at least a portion of the cooling device is disposed between the antenna and the wall of the airway when the antenna is positioned at the treatment location.

5. The pulmonary treatment apparatus of any of the preceding claims, wherein the microwave antenna operates at a frequency selected so that the microwave energy penetrates the airway wall to a depth of the nerve tissue with an intensity sufficient to alter the nerve tissue while having insufficient intensity in non-target tissue beyond the nerve tissue to cause permanent injury.

6. The pulmonary treatment apparatus of any of the preceding claims, further comprising at least one supporting element movable from a contracted position to an expanded position and configured to engage the wall of the airway to position the microwave antenna.

7. The pulmonary treatment apparatus of any of the preceding claims, wherein the antenna comprises a plurality of longitudinally spaced slots.

8. The pulmonary treatment apparatus of any of the preceding claims, wherein the microwave antenna comprises a dual antenna system configured to produce a standing wave at a location corresponding to the nerve tissue when the microwave antenna is at the treatment location.

9. The pulmonary treatment apparatus of any of the preceding claims, further comprising a shield positioned and configured to block or attenuate the microwave energy emitted in a selected direction from the microwave antenna.

10. The pulmonary treatment apparatus of any of the preceding claims, further comprising a reflector positionable at a location spaced apart from the microwave antenna and configured to reflect the microwave energy delivered therefrom.

11. The pulmonary treatment apparatus of claim 10, wherein the reflector is positionable in an esophagus and preferably comprises an inflatable member connected to a source of inflation fluid.

12. The pulmonary treatment apparatus of any of the preceding claims, further comprising a protection member positionable in an esophagus.

13. The pulmonary treatment apparatus of claim 12, wherein the protection member comprises an expandable member configured to engage esophageal tissue and absorb heat therefrom.

14. The pulmonary treatment apparatus of claim 12, wherein the protection member is configured to absorb microwave energy delivered from the microwave antenna.

15. The pulmonary treatment apparatus of any of the preceding claims, wherein the microwave antenna is positionable through a bronchoscope to the treatment location.
